# EUROPEAN PATENT SPECIFICATION

(11) **EP 2 928 502 B1**
(45) Date of publication and mention of the grant of the patent: **23.01.2019**
(21) Application number: 13802328.8
(22) Date of filing: 04.12.2013
(51) Int. Cl.: A61K 47/64, A61K 51/08, A61K 51/10, A61P 35/00

(54) **CONJUGATES OF PROTEINS AND MULTIVALENT CELL-PENETRATING PEPTIDES AND THEIR USES**
KONJUGATE VON PROTEINEN UND MULTIVALENTE ZELLENPENETRIERENDE PEPTIDE UND DEREN VERWENDUNGEN
CONJUGUÉS DE PROTÉINES ET DE PEPTIDES DE PÉNÉTRATION CELLULAIRE MULTIVALENTS ET LEURS UTILISATIONS

(30) Priority: 05.12.2012 US 201261733619 P
(43) Date of publication of application: 14.10.2015
(73) Proprietor: Ruprecht-Karls-Universität Heidelberg, 69117 Heidelberg (DE)
(72) Inventor: STRIEKER, Matthias, 38179 Schwülper (DE); MIER, Walter, 64625 Bensheim (DE); HABERKORN, Uwe, Schwetzingen 67823 (DE)
(74) Representative: Grahn, Sibylla Maria
(86) International application number: PCT/EP2013/075494
(87) International publication number: WO 2014/086835

(56) References cited:
- WO-A1-95/33766
- WO-A1-2006/056227
- WO-A2-03/103718
- WO-A2-2010/129033
- ANDREA L.J. MARSCHALL ET AL: "Targeting antibodies to the cytoplasm", MABS, vol. 3, no. 1, 1 January 2011 (2011-01-01) , pages 3-16, XP055100755, ISSN: 1942-0862, DOI: 10.4161/mabs.3.1.14110
- COLIN V. BONDUELLE ET AL: "Dendritic Guanidines as Efficient Analogues of Cell Penetrating Peptides", PHARMACEUTICALS, vol. 3, no. 3, 1 January 2010 (2010-01-01) , pages 636-666, XP055040573, ISSN: 1424-8247, DOI: 10.3390/ph3030636

## Description

The present invention relates to conjugates comprising a protein and multivalent cell-penetrating peptide(s), each multivalent cell-penetrating peptide comprising at least two cell-penetrating peptides, wherein the multivalent cell-penetrating peptide(s) is/are covalently attached to the protein. The present invention furthermore relates to a method of generating the conjugates and to their medical uses, in particular their use in the diagnosis, prevention and/or treatment of diseases.

### BACKGROUND OF THE INVENTION

The application of novel biological therapeutics based on molecular medicine knowledge is often complicated by the large size of these compounds. Most biological therapeutics with a pronounced specificity, such as antibodies, possess a high molecular weight, but which results in unfavourable pharmacokinetic qualities setting hurdles for their clinical applications. The successful clinical application of many high molecular weight drugs is hampered by their inability to efficiently bind to their target cell surfaces and/or traverse the cellular membrane (Sarko et al., 2010).

For example, antibodies and immunoglobulin-based agents are widely used in therapies for an increasing number of human malignancies (Waldmann 2003), especially cancer (Oldham & Dillman, 2008). Besides immunotherapeutic regimens based on antibody-dependent cellular cytotoxicity (ADCC) and/or complement-dependent cytotoxicity (CDC), immunoconjugates charged with toxins or radionuclides have been developed to enhance the antitumor potency (Carter 2001). The latter mode of treatment is known as radioimmunotherapy (RAIT), a concept which was proposed in the early 1950s. Now, almost 60 years later, there are only two radiolabeled antibodies, ⁹⁰Y-ibritumomab tiuxetan (Zevalin®, Spectrum Pharmaceuticals) and ¹³¹I-tositumab (Bexxar®, GlaxoSmithKline), approved for clinical use (Witzig et al., 2002; Kaminski et al., 2001). Application of these two is indicated for patients with relapsed or refractory, low grade, follicular or transformed, non-Hodgkin's lymphoma (NHL). NHLs are good targets for RAIT, because they often are highly sensitive to radiation. Solid tumors are usually slow in growth and thus successful treatment remains a challenge. Especially, as full size antibodies have poor pharmacokinetic properties, i.e. slow binding kinetics and poor clearance, which results in collateral radiation-based damage (Song & Sgouros, 2011; Pouget et al., 2011), their usage in radioimmunodetection (RAID) and RAIT of cancer is limited. A plethora of antibody-based fragments and pretargeting approaches have been developed.

Cell-penetrating peptides are a relatively new class of short peptide sequences that cross the cytoplasmic membrane efficiently. Notably, when coupled to a cargo payload, they facilitate cellular uptake of the cargo. They have a broad range of possible applications in drug delivery and molecular biology (Fonseca at al., 2009; Howl et al., 2007; Kersemans et al., 2008). Antibody modifications with single CPPs have been reported in literature but mainly for molecular imaging (Hu et al., 2007; Cornelissen et al., 2007; Hu et al. 2006) and not for tumor therapy or detection. The only example for a successful application of CPPs in radioimmunoscintigraphy, is the modification of a divalent single-chain fragment of the antitumor-associated glycoprotein 72 monoclonal antibody CC49. When co-administered with a CPP, the tumor uptake and tumor-to-normal tissue ratio of the fragment increased significantly in tumor xenografts at 24h (Jain et al., 2005). In contrast to single chain fragments, full-size monoclonal, tumor-targeting antibodies are readily available and clinically tested. Therefore, means and methods to improve their tumor-retention and pharmacokinetic properties are desired.

Small branched synthetic peptide conjugates were developed as vehicles for the delivery of diagnostic probes and cytotoxic agents into the cytoplasm and the nucleus (Sheldon et al., 1995; WO 95/33766 A1), which are particularly suitable as transfection agents (Singh et al., 1999).

WO 03/103718 A2 discloses multimeric peptide transporters with a poly-lysine core and cell-penetrating peptides (CPPs) as "transporter units", which further contain effectors, such as antibodies.

Furthermore, means and methods to improve further clinically relevant proteins, such as coagulation factors, that improve their pharmacokinetic properties are desired.

There is a need in the art for providing means and methods for improving the pharmacokinetics and/or internalization of biologically or clinically relevant and/or therapeutic proteins, in particular to improve their use for the diagnosis and treatment of diseases.

### SUMMARY OF THE INVENTION

According to the present invention the object is solved as claimed in the claims.

According to the present invention this object is solved by providing a conjugate comprising
a protein, and
one or several multivalent cell-penetrating peptide(s) (multivalent CPP(s)) each multivalent CPP comprising at least two cell-penetrating peptides (CPPs),
wherein the multivalent CPP(s) is/are covalently attached to the protein,
wherein each of the multivalent CPP(s) is a dendrimer of cell-penetrating peptides (cell-penetrating peptide dendrimer, dCPP) comprising a dendrimer core and at least two cell-penetrating peptides (CPPs), which are coupled to the dendrimer core,
said dendrimer core being a peptidyl dendrimer core comprising lysines as branching points and cysteine(s) as the anchoring group(s), and comprises or consists of 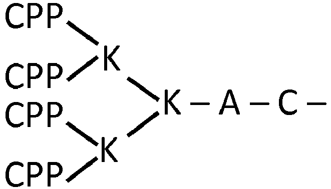 or 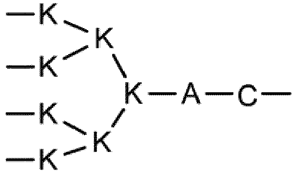 wherein K is Lys, A is Ala and C is Cys,
wherein the cell-penetrating peptide(s) comprise the amino acid sequence of
penetratin (SEQ ID NO. 1),
TAT (47-60) (human immunodeficiency virus-derived trans-activator of transcription, SEQ ID NO. 2),
PreS2-TLM (hepatitis B virus-preS2-domain-derived translocation motif, SEQ ID NO. 3),
R9 (SEQ ID NO. 4),
MTS (membrane translocation signal, SEQ ID NO. 5),
SynB1 (synthetic porcine protegrin 1-derived CPP, SEQ ID NO. 6),
pVEC (vascular endothelial cadherin-derived CPP, SEQ ID NO. 7),
NLS (nuclear localization signal, SEQ ID NO. 8),
or combinations thereof.

According to the present invention this object is solved by a method for generating a conjugate according to the invention, comprising the steps of
(a) providing multivalent cell-penetrating peptide(s) comprising anchoring group(s),
   wherein each of the multivalent CPP(s) is a dendrimer of cell-penetrating peptides (cell-penetrating peptide dendrimer, dCPP) comprising a dendrimer core and at least two cell-penetrating peptides (CPPs), which are coupled to the dendrimer core, as defined in the present invention,
(b) generating a chemically activated protein, such as a chemically activated antibody, by using a linker, or providing a protein comprising coupling site(s),
(c) coupling the multivalent CPP(s) of step (a) to the protein of step(b),
(d) obtaining the conjugate,
(e) purifying the conjugate,
wherein in step (a) cell-penetrating peptide dendrimer(s) (dCPPs) comprising one or several anchoring group(s) are provided,
and/or wherein the coupling site(s) of the protein are the sides chains of cysteine(s), glutamine(s) and/or lysine(s) and/or unnatural amino acids,
wherein the linker is a bifunctional (cross) linker, such as succinimidyl-4-(N-maleimidomethyl)cyclohexane-1-carboxylate (SMCC),
and/or comprising using in step (b) excess of the linker, preferably to generate a chemically activated protein, such as an antibody, having one or several maleimide molecule(s) on the surface,
and/or comprising using in step (c) excess of the cell-penetrating peptide dendrimer(s). According to the present invention this object is solved by providing the conjugate according to the invention for use in medicine.

According to the present invention this object is solved by providing the conjugate according to the invention for use in the diagnosis, prevention and/or treatment of diseases.

According to the present invention this object is solved by using the conjugate according to the invention as an *in vitro* diagnostic agent.

### DESCRIPTION OF THE PREFERRED EMBODIMENTS OF THE INVENTION

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "5 to 40 amino acids" should be interpreted to include not only the explicitly recited values of 5 to 40, but also include individual values and sub-ranges within the indicated range. Thus, included in this numerical range are individual values such as 5, 6, 7, 8, 9, 10, 11, 12, 13, 14, 15, 16, 17, 18, 19, 20,... 38, 39, 40 and sub-ranges such as from 5 to 25, from 5 to 20, from 10 to 25, from 10 to 20 and from 15 to 25, etc. As an illustration, a numerical range of "at least 2 branching points, preferably 2 to 10" should be interpreted to include not only the explicitly recited values of 2 to 10, but also include individual values and sub-ranges within the indicated range. Thus, included in this numerical range are individual values such as 2, 3, 4, 5, 6, 7, 8, 9, 10 and sub-ranges such as from 2 to 8, from 2 to 7, from 3 to 8, 4 etc. This same principle applies to ranges reciting only one numerical value. Furthermore, such an interpretation should apply regardless of the breadth of the range or the characteristics being described.

### Conjugates of a protein with multivalent cell-penetrating peptide(s)

As described above, the present invention provides conjugates of a protein and multivalent CPP(s).

A conjugate of the invention comprises:
a *protein,* and
one or several *multivalent cell-penetrating peptide(s)* (multivalent CPP(s)) each multivalent CPP comprising at least two cell-penetrating peptides (CPPs),
wherein the multivalent CPP(s) is/are covalently attached to the protein.

According to the invention, each of the multivalent CPP(s) is *a dendrimer of cell-penetrating peptides* (cell-penetrating peptide dendrimer, dCPP) comprising a dendrimer core and at least two cell-penetrating peptides (CPPs), which are coupled to the dendrimer core.

Said dendrimer core is a peptidyl dendrimer core comprising lysines as branching points and cysteine(s) as the anchoring group(s), and comprises or consists of 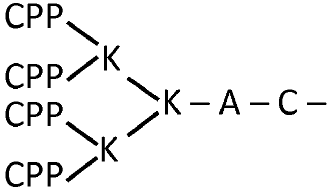 or 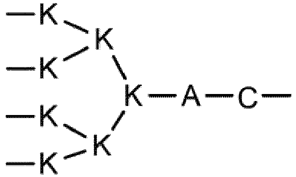 wherein K is Lys, A is Ala and C is Cys.

According to the invention, the cell-penetrating peptide(s) comprise the amino acid sequence of
penetratin (SEQ ID NO. 1),
TAT (47-60) (human immunodeficiency virus-derived trans-activator of transcription, SEQ ID NO. 2),
PreS2-TLM (hepatitis B virus-preS2-domain-derived translocation motif, SEQ ID NO. 3),
R9 (SEQ ID NO. 4),
MTS (membrane translocation signal, SEQ ID NO. 5),
SynB1 (synthetic porcine protegrin 1-derived CPP, SEQ ID NO. 6),
pVEC (vascular endothelial cadherin-derived CPP, SEQ ID NO. 7),
NLS (nuclear localization signal, SEQ ID NO. 8),
or combinations thereof.

Preferably, each *multivalent cell-penetrating peptide* (multivalent CPP) comprises 2 to 200 CPPs, preferably 2 to 50, more preferably 2 to 20 or 4 to 20 CPPs.

In one embodiment, each multivalent CPP consists of a *dendrimer of cell-penetrating peptides.*

Each multivalent CPP is a dendrimer of cell-penetrating peptides (cell-penetrating peptide dendrimer, dCPP) comprising a dendrimer core or scaffold and at least two cell-penetrating peptides (CPPs), which are coupled to the dendrimer core.

Dendrimers are known in the art.

"Dendrimers" or "dendrons" as used herein refer to repetitively branched (macro)molecules comprising a core, which are preferably wedge-like branched (macro)molecules. A dendrimer as used herein contains a single chemically addressable group called the focal point in the art and is called an "anchoring group" herein. A dendrimer as used herein can be not symmetric around the core.

According to the invention, the dendrimer core or scaffold comprises
- anchoring group(s) for coupling to the protein or linker, namely cysteines,
- branching point(s), namely lysines.

Preferably, the dendrimer core or scaffold comprises spacer between the anchoring group(s) and the branching point(s).

The anchoring group of focal point serves for coupling to the protein or linker.

Preferably, the dendrimer core or scaffold comprises one anchoring group.

In some embodiments, the dendrimer core or scaffold can comprise more than one anchoring groups, such as 1 to 25 or 1 to 4.

The branching point(s) are for branching the dendrimer core and/or for coupling to the CPPs.

According to the invention, the dendrimer core or scaffold is a peptide dendrimer core or peptidyl dendrimer core or peptidic dendrimer core. A peptide/peptidic/peptidyl dendrimer core is a dendrimer core comprising amino acids which comprise the anchoring group(s) and which form the branching points.

Peptide dendrimer cores or scaffolds are known in the art.

Peptide dendrimer core or scaffolds can comprise natural amino acids, amino acid derivatives, L-and/or D-amino acids, modified amino acids, such as β-amino acid derivatives, α,α-disubstituted amino acid derivatives, N-substituted α-amino acid derivatives, aliphatic or cyclic amines, amino- and carboxy- substituted cycloalkyl derivatives, amino- and carboxy-substituted aromatic derivatives, γ-amino acid derivatives, aliphatic α-amino acid derivatives, diamines and polyamines.

For example, a dendrimer core or scaffold can be a polylysine.

According to the invention, the dendrimer core or scaffold comprises lysines as branching points and cysteine(s) as the anchoring group(s) (-SH group).

For example, a dendrimer core or scaffold can comprise lysine(s) as the anchoring group(s).

Further anchoring group(s) are known in the art, see e.g. Sarko et al., 2012.

Dendrimer cores or scaffolds are known in the art, see for example the review of Khandare et al. 2012.

Further dendrimer cores or scaffolds are described herein that comprise or consist of a polyamidoamine (PAMAM), poly(propylene imine), polyaryl ether, polyester, polyamide, polyglycerol, triazine based, poly(glycerol-succinic acid) core.

Preferably, the dCPP(s) have a size ranging from about 2 to about 100 kDa, preferably from about 2 to about 50 kDa, more preferably about 4 to 20 kDa.

In one embodiment, each multivalent CPP consists of *multiple copies of CPPs,* such as a linear sequence or combination of cell-penetrating peptides.

For example, the multiple copies of CPPs are linked to a linear polymer or linear scaffold.

Examples for suitable linear polymers or scaffolds are polyoxazoline copolymer (see Farkas et al., 2010) or methacrylamide polymers, such as pHPMAm (poly[N-2-hydroxypropyl]methacrylamide) (see Jay et al., 2009).

Also the linear polymer or scaffold comprises one or several anchoring groups for coupling to the protein or linker.

"Cell-penetrating peptides" (CPPs) refer to short peptides of 5 to 40 amino acids in length that facilitate cellular uptake of various cargoes, from nanosized particles to small chemical molecules and macromolecules, such as nucleic acids, peptides, proteins, drugs, liposomes etc.

The "cargo" is associated with the peptides either through chemical linkage via covalent bonds or through non-covalent interactions. The attachment of one or multiple CPPs to the cargo facilitates the delivery of the cargo into cells.

CPPs typically have an amino acid composition that
(1) either contains a high relative abundance of positively charged amino acids, such as lysine or arginine,
   or
(2) have sequences that contain an alternating pattern of polar/charged amino acids and non-polar, hydrophobic amino acids.

These two types of structures are referred to as
(1) polycationic or cationic
   or
(2) amphipathic.

CPPs are of different sizes, amino acid sequences, and charges but all CPPs have one distinct characteristic, which is the ability to translocate the plasma membrane and facilitate the delivery of various molecular cargoes to the cytoplasm or an organelle. There has been no real consensus as to the mechanism of CPP translocation, but the theories of CPP translocation can be classified into three main entry mechanisms: direct penetration in the membrane, endocytosis-mediated entry, and translocation through the formation of a transitory structure.

Preferably, the *cell-penetrating peptide,* each comprising the amino acid sequence selected from the group of SEQ ID Nos. 1 to 8, comprises an amino acid sequence having 5 to 50 amino acids, preferably up to 30 amino acids, such as 5 to 30 amino acids, more preferably 5 to 25 or 8 to 25 amino acids.

Thereby, each of a CPP of a dendrimer of CPPs or each of a CPP of multiple copies of CPPs comprises such an amino acid sequence.

Preferably, the CPPs comprise natural amino acids, amino acid derivatives, D-amino acids, modified amino acids, β-amino acid derivatives, α,α-disubstituted amino acid derivatives, N-substituted α-amino acid derivatives, aliphatic or cyclic amines, amino- and carboxy-substituted cycloalkyl derivatives, amino- and carboxy- substituted aromatic derivatives, γ-amino acid derivatives, aliphatic α-amino acid derivatives, diamines and polyamines. Further modified amino acids are known to the skilled artisan.

The term "natural amino acid residue" or "natural amino acid", as used herein, denotes any of the 22 "standard" amino acids that are naturally incorporated into peptides. Of these twenty-two, twenty are directly encoded by the universal genetic code. The remaining two, selenocysteine and pyrrolysine are incorporated into proteins by unique synthetic mechanisms. Typically, the amino acid residues of a peptide according to the invention are present as L-isomers. In some embodiments, one or more, eventually all amino acid residues of a peptide according to the invention are present as D-isomers. The term "modified amino acid residue", as used herein, denotes non-standard amino acids such as modified amino acids. Examples of modifications include inter alia phosphorylation, glycosylation, acylation (e.g., acetylation, myristoylation, palmitoylation), alkylation, carboxylation, hydroxylation, glycation, biotinylation, ubiquitinylation, changes of the chemical nature (e.g., [beta]-elimination deimidation, deamidation,), and structural changes (e.g., the forming of disulfide bridges). The amino acid sequences of the peptides as defined herein are written, according to the general convention, from the amino (N)-terminus to the carboxyl (C)-terminus. However, the corresponding "reverse" peptides are also within the present invention. The term "reverse peptide", as used herein, denotes peptides having the same sequence as their "regular" counterparts but in reverse orientation, that is, from the C-terminus to the N-terminus.

Preferably, a CPP, comprising the amino acid sequence selected from the group of SEQ ID Nos. 1 to 8,
(i) is a (carrier) peptide capable of being internalized into a cell;
   and/or
(ii) comprises in its amino acid sequence at least 25% positively charged amino acid residues;
   and/or
(iii) is internalized into a cell with an efficacy being at least 50% of the internalization efficacy of the TAT peptide having the amino acid sequence of SEQ ID NO: 2.

In one embodiment (ii), the CPPs have in their respective primary amino acid sequences (that is, over their entire length) at least 25%, preferably at least 30% positively charged amino acid residues. The term "positively charged amino acids" (herein also referred to as "basic amino acids"), as used herein, denotes the entirety of lysine (K), histidine (H), and arginine (R) residue present in a particular peptide. In specific embodiments, a peptide used in the present invention comprises in its primary amino acid sequence 25%, 26%, 27%, 28%, 29%, 30%, 31 %, 32%, 33%, 34% or 35% positively charged amino acid residues. In other embodiments, the peptides used herein comprise in their respective primary amino acid sequences at least 35%, at least 40%, at least 45%, at least 50%, at least 55%, or at least 60% positively charged amino acid residues.

### (i) Preferably, a CPP is a (carrier) peptide capable of being internalized into a cell.

The term "capable of being internalized into a cell", as used herein, refers to the ability of the peptides to pass cellular membranes (including inter alia the outer "limiting" cell membrane (also commonly referred to as "plasma membrane"), endosomal membranes, and membranes of the endoplasmatic reticulum) and/or to direct the passage of a given agent or cargo through these cellular membranes. Such passage through cellular membranes is herein also referred to as "cell penetration". Accordingly, peptides having said ability to pass through cellular membranes are herein referred to as "cell-penetrating peptides". In the context of the present invention, any possible mechanism of internalization is envisaged including both energy-dependent (i.e. active) transport mechanisms (e.g., endocytosis) and energy-independent (i.e. passive) transport mechanism (e.g., diffusion). As used herein, the term "internalization" is to be understood as involving the localization of at least a part of the peptides that passed through the plasma cellular membrane into the cytoplasma (in contrast to localization in different cellular compartments such as vesicles, endosomes or in the nucleus). In specific embodiments, a given transport mechanism that is employed ensures that at least 0.01 %, at least 0.05%, at least 0.1 %, at least 0.5%, at least 1 %, at least 2%, at least 5% or at lest 10% of the peptides or compositions internalized localize into the cytoplasm.

### (iii) Preferably, a CPP is internalized into a cell with an efficacy being at least 50% of the internalization efficacy of the TAT peptide having the amino acid sequence of SEQ ID NO: 2.

Hereby, the internalization efficacy of a single CPP is referred to and not the internalization efficacy of a multivalent CPP.

According to the invention, the internalization efficacy of the multivalent CPP is preferably higher compared to the internalization efficacy of a single CPP. This allows the use of CPPs which show a rather low internalization efficacy compared to TAT and when used as single copy.

The peptides used in the present invention are internalized into a cell with an efficacy being at least 50%, such as 60% of the internalization efficacy of the TAT peptide (preferably having the amino acid sequence of SEQ ID NO. 2) (see Vives; E. et al. 1997). In other words, the functional activity of the peptides is characterized in comparison to a reference peptide (TAT represents the "gold standard" with regard to cell- penetrating peptides). In specific embodiments, the peptides used herein are internalized with an efficacy being 80%, 85%, 90% or 95% of the internalization efficacy of the TAT peptide. In specific preferred embodiments, the peptides used herein are internalized with at least the same efficacy (i.e. 100%) as the TAT peptide. Particularly preferably, the peptides used herein are internalized with a higher efficacy (i.e. more than 100% or at least 101 %) as the TAT peptide, e.g., with 105%, 1 10%, 1 15%, 120%, 125%, 130%, 140%, 150%, 160%, 170%, 180%, 190%, or 200% of the internalization efficacy of the TAT peptide.

The term "internalization efficacy", as used herein, is to be understood in a broad sense. The term does not only refer to the extent to which a peptide used in the invention passes through the plasma membrane of cells (i.e. the internalization behavior per se) but also to the efficiency by which the peptide directs the passage of a given agent or cargo through the cell plasma membrane (i.e. its transfection capability; herein also referred to as "transfectivity"). Numerous methods of determining the internalization behavior and/or transfection capability of a given peptide are established in the art, for example, by attaching a detectable label (e.g. a fluorescent dye) to the peptide (and/or to the cargo to be transfected) or by fusing the peptide with a reporter molecule, thus enabling detection once cellular uptake of the peptide occurred, e.g., by means of FACS analysis or via specific antibodies (see, e.g., Ausubel, et al., 2001). The skilled person is also well aware how to select the respective concentration ranges of the peptide and, if applicable, of the cargo to be employed in such methods, which may depend on the nature of the peptide, the size of the cargo, the cell type used, and the like. In further embodiments, the peptides used in the present invention do not exert significant cytotoxic and/or immunogenic effects to their respective target cells after having been internalized, that is, they do not interfere with cell viability (at least at concentrations that are sufficient to mediate cellular transfection and/or penetration). The term "not significant", as used herein, is to be understood that less than 50%, preferably less than 40% or 30%, and particularly less than 20% or 10% of the target cells are killed after internalization of a peptide of the invention. In other embodiments, the cytotoxic (and/or immunogenic) effects exerted by the peptides upon internalization into a cell are the same or less than the corresponding effects exerted by the TAT peptide having the amino acid sequence of SEQ ID NO. 2. In specific embodiments, the cytotoxic (and/or immunogenic) effects exerted by the peptides upon internalization into a cell are less than 95%, less than 90%, less than 85%, less than 80%, less than 75%, less than 70%, less than 60%, less than 50%, less than 40%, less than 30%, less than 20%, or less than 10% of the effects exerted by the TAT peptide. The skilled person is well aware of methods of determining the cytotoxicity of a given compound and/or the viability of a given target cell to which such a compound is applied (see also, e.g., Ausubel, F.M. et al. (2001), supra). Corresponding assay kits are commercially available from various suppliers.

There exists a database of experimentally validated CPPs (CPPsite, Gautam et al., 2012).

In one embodiment, the multivalent CPP of the invention comprises several of the same CPP (having an identical amino acid sequence) or comprises different CPPs, i.e. CPPs with different amino acid sequences, such as 2, 3, 4 or more different CPPs.

In the embodiment, where the multivalent CPP comprises different CPPs, the CPP(s) comprise or consist of the amino acid sequence selected from the group of SEQ ID NOs. 1 to 8 or from combinations of SEQ ID NOs. 1 to 8 and/or from any subset of amino acid sequences with SEQ ID NOs. 1 to 8.

According to the invention, the cell-penetrating peptide(s) comprise or consist of the amino acid sequence of
penetratin (SEQ ID NO. 1),
TAT (47-60) (human immunodeficiency virus-derived trans-activator of transcription, SEQ ID NO. 2),
PreS2-TLM (hepatitis B virus-preS2-domain-derived translocation motif, SEQ ID NO. 3),
R9 (SEQ ID NO. 4),
MTS (membrane translocation signal, SEQ ID NO. 5),
SynB1 (synthetic porcine protegrin 1-derived CPP, SEQ ID NO. 6),
pVEC (vascular endothelial cadherin-derived CPP, SEQ ID NO. 7),
NLS (nuclear localization signal, SEQ ID NO. 8),
or combinations thereof.

For example, the cell-penetrating peptide(s) comprise the amino acid sequence of penetratin (SEQ ID NO. 1) and/or R9 (SEQ ID NO. 4).

The present disclosure also describes cell-penetrating peptide(s) that comprise or consist of the amino acid sequence of SEQ ID NOs. 9 to 77 or combinations thereof.

| Name | Sequence | SEQ ID NO. |
|---|---|---|
| TAT peptide (49-57) | RKKRRQRRR | 9 |
| TAT (48-60) | GRKKRRQRRRPPQ | 10 |
| calcitonin-derived | LGTYTQDFNKFHTFPQTAIGVGAP | 11 |
| CPP | | |
| NLS | VQRKRQKLMP | 12 |
| NLS | SKKKKTKV | 13 |
| NLS | GRKRKKRT | 14 |
| polybasic CPP | RRRERRAEK | 15 |
| polybasic CPP | KCPSRRPKR | 16 |
| N-terminal repetitive | (VRLPPP)n | 17 |
| domain of maize | (VHLPPP)n | 18 |
| gamma-zein | (VKLPPP)n | 19 |
| Peptide from gp41 fusion sequence | AVGAIGALFLGFLGAAG | 20 |
| SSHR | VTVLALGALAGVGVG | 21 |
| pVEC | IAARIKLRSRQHIKLRHL | 22 |
| Vpr | DTWPGVEALIRILQQLLFIHFRIGCQH | 23 |
| VP22 | DAATATRGRSAASRPTERPRAPARSASRPRRPVD | 24 |
| gp41 fusion sequence | GALFLGWLGAAGSTMGA | 25 |
| Caiman crocodylus Ig(v) light chain | MGLGLHLLVLAAALQGA | 26 |
| CPP from pestivirus env glycoprotein | RQGAARVTSWLGRQLRIAGKRLEGRSK | 27 |
| CPP from the prion protein | MANLGYWLLALFVTMWTDVGLCKKRPKP | 28 |
| Yeast PRP6 (129-144) | TRRNKRNRIQEQLNRK | 29 |
| Phi21 N (12-29) | TAKTRYKARRAELIAERR | 30 |
| Delta N (1-22) | MDAQTRRRERRAEKQAQWKAAN | 31 |
| FHV coat (35-49) | RRRRNRTRRNRRRVR | 32 |
| BMV Gag (7-25) | KMTRAQRRAAARRNRWTAR | 33 |
| HTLV-II Rex (4-16) | TRRQRTRRARRNR | 34 |
| HIV-1 Rev (9-20) | RQARRNRRRRWR | 35 |
| RSG-1.2 | DRRRRGSRPSGAERRRRRAAAA | 36 |
| Lambda-N (48-62) | QTRRRERRAEKQAQW | 37 |
| Bipartite, Nucleoplasmin (155-170), NLS | KRPAAIKKAGQAKKKK | 38 |
| Herpesvirus, 8 k8 protein (124-135) | TRRSKRRSHRKF | 39 |
| Buforin-II (20-36) | RAGLQFPVGRVHRLLRK | 40 |
| Magainin | TRSSRAGLQFPVGRVHRLLRK | 41 |
| PDX-1-PTD | RHIKIWFQNRRMKWKK | 42 |
| crotamine | YKQCHKKGGHCFPKEKICLPPSSDFGKMDCRWRWKCCKKGSG | 43 |
| pIsI | RVIRVWFQNKRCKDKK | 44 |
| Fushi-tarazu (254-313) | | 45 |
| Engrailed (454-513) | | 46 |
| transportan | GWTLNSAGYLLGKINLKALAALAKKIL | 47 |
| Polyarginine CPP (R8) | RRRRRRRR | 48 |
| Poly-D-arginine | rrrrrrrrr | 49 |
| KLAL peptide/ model amphiphatic peptide (MAP) | KLALKLALKALKAALKLA | 50 |
| KALA model amphiphatic peptide | WEAKLAKALAKALAKHLAKALAKALKACEA | 51 |
| modeled TAT peptide | YARAAARQARA | 52 |
| b-sheet-forming peptide | DPKGDPPKGVTVTVTVTVTGKGDPKPD | 53 |
| retro-inverso forms of established CPPs | KKWKMRRNQFWVRVQR | 54 |
| W/R penetratin | RRWRRWWWRRWWRRWRR | 55 |
| MPG | GALFLGFLGAAGSTMGAWSQPKSKRKVC | 56 |
| Pep-1 | KETWWETWWTEWSQPKKKRKV | 57 |
| Signal-sequence peptides (I) | GALFLGWLGAAGSTMGAWSQPKKKRKV | 58 |
| PTD-5 | RRQRRTSKLMKR | 59 |
| RSV-A9 | RRIPNRRPRR | 60 |
| CTP-512 | YGRRARRRRRR | 61 |
| U2AF | SQMTRQARRLYV | 62 |
| 105Y | SIPPEVKFNKPFVYLI | 63 |
| Antennapedia Leader Peptide | KKWKMRRNQFWVKVQRG | 64 |
| Anti-BetaGamma | AAVALLPAVLLALLAVTDQLGEDFFAVDLEAFLQEFGLLPEKE | 65 |
| Lipid Membrane Translocating | KKAAAVLLPVLLAAP | 66 |
| Mastoparan | INLKALAALAKKIL | 67 |
| MEK1 Nterm | MPKKKPTPIQLNP | 68 |
| MPS | AAVALLPAVLLALLAK | 69 |
| RV-MAT | MNLLRKIVKNRRDEDTQKSSPASAPLDDG | 70 |
| Transdermal peptide | ACSSSPSKHCG | 71 |
| SynB3 | RRLSYSRRRF | 72 |
| PTD-4 | PIRRRKKLRRLK | 73 |
| SBP | MGLGLHLLVLAAALQGAWSQPKKKRKV | 74 |
| Pep-2 | KETWFETWFTEWSQPKKKRKV | 75 |
| Polyarginines | (R)n | 76 |
| | wherein n is at least 3, preferably 3 to 50, more preferably 3 to 15 | |
| Polylysines | (K)n | 77 |
| | wherein n is at least 3, preferably 3 to 50, more preferably 3 to 15 | |

The present disclosure also describes cell-penetrating peptide(s) that comprise or consist of the amino acid sequence of SEQ ID NOs. 78 to 760 or combinations thereof.

For the amino acid sequences of SEQ ID NOs. 1 to 760, see Table 1.

In an embodiment, the CPP does not consist of the amino acid sequence TPPKKKRKVEDP or of the amino acid sequence TPPKKKRKVEDP in combination with the amino acid sequence KKKKK ((Lys)₅). For example, the CPP does not consist of the amino acid sequence TPPKKKRKVEDPKKKKK **or** KKKKKTPPKKKRKVEDP.

In an embodiment, the multivalent CPP does not consist of the amino acid sequence (TPPKKKRKVEDPKKKKK)ₙ or (TPPKKKRKVEDP)ₙ
with n ≥ 2, such as 2 to 8, such as 8.

Preferably, the *protein* is a biological or clinically active or therapeutic protein.

Such biological or clinically active or therapeutic proteins are known in the art. See, for example, http://www.genomicglossaries.com/content/Protein categories.asp.

Preferred examples are:
an antibody or an antibody fragment,
a coagulation factor or cofactor,
a receptor or receptor ligand(s),
insulin or insulin analogues,
interferon(s), such as IFN-α2a, IFN-β1a,
enzyme(s), such as arginine deiminase methioninase superoxide dismutase uricase cytokine(s), such as granulocyte colony stimulating factor (G-CSF),
erythropoietin(s),
interleukin(s), such as interleukin-6, PEG-interleukin-2,
hormone(s), such as calcitonin, human growth hormone (HGF),
a vaccine,
or combinations thereof.

The antibody can be a full-size antibody, such as a monoclonal antibody, or a recombinant antibody, or engineered antibodies/antibody fragments, such as a single chain variable fragment (scFv), the fragment antigen-binding (Fab) or the Fab-Dimer (F(ab')₂ fragment.

In a preferred embodiment, the antibody of the conjugate of the invention is a "full-size" antibody, such as a monoclonal antibody.

Depending on the protein and/or type of multivalent CPP used in the conjugate of the invention, there are different possibilities for covalently attaching the multivalent CPP to the protein.

For example, the multivalent CPP(s) can be coupled to cysteine, glutamine or lysine residue(s) of the protein.

For example, the multivalent CPP(s) can be coupled to a chemically activated protein, wherein the protein can be chemically activated by using a suitable linker.

For example, the multivalent CPP(s) can be coupled to a protein comprising a functional group of an unnatural amino acid, which has been incorporated into the protein either by chemically modification or by recombinant expression using an expanded genetic code (see e.g. Xie & Schultz, 2006).

In one embodiment, the conjugates of the invention furthermore comprise *linker* connecting the protein and the multivalent CPP(s).

Preferably, the *linker* is a bifunctional (cross)linker covalently coupling the protein with the multivalent cell-penetrating peptide(s).

For example, the linker is a bifunctional (cross)linker, such as succinimidyl-4-(N-maleimidomethyl)cyclohexane-1-carboxylate (SMCC).

Further linker are known in the art, see e.g. the review Sarko et al., 2012.

In a preferred embodiment, the conjugate of the invention comprises
an antibody,
one or several cell-penetrating peptide dendrimer(s) (dCPP) as defined herein,
one or several linker, preferably bifunctional (cross)linker(s), each covalently coupling the antibody with a dCPP.

For example, the conjugate of the invention comprises
an antibody,
one cell-penetrating peptide dendrimer (dCPP),
a linker, preferably one bifunctional (cross)linker.

Preferably, the conjugates of the invention comprise *further component(s)*, such as
- labels
   such as radioisotope(s), fluorescent dye(s), quantum dots, contrast agent(s)
- drugs or prodrugs,
- further biologically active component(s),
or combinations thereof.

Depending on the further component(s) and/or the protein, such as the specificity of the antibody, the conjugates of the invention can be used for the diagnosis, prevention and/or treatment of diseases.

For example, a conjugate comprising a cell-specific antibody and a radioisotope is suitable for radioimaging, radioimmunodetection but also for radioimmunotherapy.

For example, a conjugate comprising a cell-specific antibody and drug(s) is suitable for cell-specific drug delivery.

For example, a conjugate comprising a tumor-specific antibody is suitable for the diagnosis, prevention and/or treatment of that tumor / cancer.

### Method of generating the multivalent CPP-protein conjugates

As described above, the present invention provides a method for generating the conjugates of a protein and a multivalent cell-penetrating peptide of the invention.

Depending on the protein and/or type of multivalent CPP used in the conjugate of the invention, there are different possibilities for covalently attaching the multivalent CPP to the protein.

For example, the multivalent CPP(s) can be coupled to coupling site(s) comprised in the protein.

The coupling site(s) can be the side chains of cysteine(s), glutamine and/or lysine(s) and/or unnatural amino acids.

The coupling site(s) can be the side chains of cysteine(s), glutamine or lysine(s), which are within the amino acid sequence of the protein, such as naturally occurring and/or added.

For coupling to glutamine, see e.g. Jeger at al., 2010, describing the site-specific modification of proteins at glutamine residues using transglutaminase.

The coupling site(s) can be unnatural amino acids, preferably incorporated into the amino acid sequence of the protein by recombinant expression of the protein by using an expanded genetic code, as described in Xie & Schultz, 2006.

For example, the multivalent CPP(s) can be coupled to a chemically activated protein, wherein the protein can be chemically activated by using a suitable linker.

The method of the invention comprises the steps of
(a) providing multivalent cell-penetrating peptide(s) (multivalent CPP(s)) comprising one or several anchoring group(s),
   wherein each of the multivalent CPP(s) is a dendrimer of cell-penetrating peptides (cell-penetrating peptide dendrimer, dCPP) comprising a dendrimer core and at least two cell-penetrating peptides (CPPs), which are coupled to the dendrimer core, as defined herein,
(b) generating a chemically activated protein, such as a chemically activated antibody, by using a linker, or providing a protein comprising coupling site(s),
(c) coupling the multivalent CPP(s) of step (a) to the protein of step(b),
(d) obtaining the conjugate,
(e) purifying the conjugate.

In one embodiment, the method of the invention comprises the steps of
(a) providing cell-penetrating peptide dendrimer(s) (dCPPs) comprising a dendrimer core and at least two cell-penetrating peptides (CPPs), which are coupled to the dendrimer core, and comprising one or several anchoring group(s),
(b) generating a chemically activated protein by using a linker, or providing a protein comprising coupling site(s),
(c) coupling the cell-penetrating peptide dendrimer(s) of step (a) to the protein of step (b), namely the chemically activated protein or the protein comprising coupling site(s),
(d) obtaining the conjugate,
(e) purifying the conjugate.

For example, the anchoring group(s) of the dCPP(s) provided in step (a) can be sulfhydryl group(s) (such as comprised in a cysteine) or amino group(s) (such as comprised in lysine(s).

For example, in step (b) a chemically activated protein, such as a chemically activated antibody, can be provided, such as a maleimide-activated protein/antibody.

The linker is a bifunctional (cross)linker, such as succinimidyl-4-(N-maleimidomethyl)cyclohexane-1-carboxylate (SMCC).

Further linker are known in the art, see e.g. the review Sarko et al., 2012.

The method preferably comprises using in step (b) excess of the linker, preferably to generate a protein (such as an antibody) having one or several maleimide molecules on the surface.

For example, in step (b) the coupling site(s) of the protein can be
- the side chains of cysteine(s), glutamine(s) or lysine(s), which are within the amino acid sequence of the protein, such as naturally occurring and/or added,
   and/or
- unnatural amino acids, preferably incorporated into the amino acid sequence of the protein by recombinant expression of the protein by using an expanded genetic code, as described in Xie & Schultz, 2006.

The method preferably comprises using in step (c) excess of the cell-penetrating peptide dendrimer(s).

### Uses of the conjugates of a protein and multivalent CPP(s)

As described above, the present invention provides the conjugates of the invention for use in medicine.

As described above, the present invention provides the conjugates of the invention for use in the diagnosis, prevention and/or treatment of diseases.

Preferably, the diagnosis comprises radioimmunodetection, radioimmunoscintigraphy, radioimmunotomography, radioimmunotomography.

Preferably, the prevention and/or treatment of a disease comprises immunotherapy and radioimmunotherapy.

The disease can be cancer, a coagulation disorder, a cardiovascular disease, an immune disease, an infectious disease, a neuronal disease, an inflammatory disease, a heritable disease or a rheumatic disease.

Preferably, the diagnosis, prevention and/or treatment comprises cell-specific targeting.

More preferably, the cell-specific targeting of diseased cells, tissues and organs; cell-specific labelling of diseased cells, tissues and organs; and/or cell-specific drug delivery to diseased cells, tissues and organs, wherein the diseased cells, tissues and organs are preferably tumor related.

As described above, the present invention provides the use of the conjugates of the invention as an *in vitro* diagnostic or an *in vitro* diagnostic agent.

The conjugates of the present invention can also be used *in vitro,* such as for cell-specific targeting.

### Methods of diagnosis and therapy

As described above, a method for the diagnosis, prevention and/or treatment of a disease is described.

The method of the invention can comprise the step of administering a conjugate of the invention to a patient.

The diagnosis can comprise radioimmunodetection, radioimmunoscintigraphy, radioimmunotomography.

The prevention and/or treatment of a disease can comprise immunotherapy and radioimmunotherapy.

The disease can be cancer, a coagulation disorder, a cardiovascular disease, an immune disease, an infectious disease, a neuronal disease, an inflammatory disease, a heritable disease or a rheumatic disease.

The diagnosis, prevention and/or treatment can comprise cell-specific targeting, such as the cell-specific targeting of diseased cells, tissues and organs; cell-specific labelling of diseased cells, tissues and organs; and/or cell-specific drug delivery to diseased cells, tissues and organs, wherein the diseased cells, tissues and organs are tumor related.

### Further description of the invention and of a preferred embodiment

The inventors have developed a novel modification route employing cell-penetrating peptide dendrimers (dCPPs) for the generation of antibody conjugates, in particular conjugates with highly specific, full-size monoclonoal antibodies (mAbs) for application in immunotherapy, radioimmunotherapy and imaging.

In the present invention, the development of a general modification strategy for antibodies, such as mAbs, by employing a commercially available, heterobifunctional crosslinker and tetravalent cell-penetrating peptide dendrimers, is described. The crosslinker reacts with a random lysine side chain of the antibody and allows in a second step the attachment of the dCPP. A tetravalent CPP dendrimer was used, as the attachment of a single CPP reportedly did not significantly increase the tumor-to-blood ratio (Hu et al., 2007) and therefore will not result in improved pharmacokinetics as compared to the unmodified antibody.

In the present invention, the syntheses of eight different CPP dendrimers and their conjugation to a tumor-targeting antibody is described. In addition, the impact of the dCPP attachment to the antibody (mAb) in terms of target cell binding is lined out. Finally, the improvement of the pharmacokinetic properties of the antibody, resulting from the coupling to the dCPPs, is shown by biodistribution studies as well as small animal scintigraphic and PET imaging.

The use of radiolabeled antibodies in imaging and therapy of solid tumors is limited due to their size, resulting in poor pharmacokinetics and clearance. To overcome these problems cell-penetrating peptide dendrimers (dCPPs) were conjugated to an EGFR-specific antibody and evaluated *in vitro* and *in vivo.*

Results: In contrast to the unmodified antibody, the immunoconjugates showed up to fourfold higher EGFR-positive cell binding *in vitro* and were internalized. Organ distribution studies of the mAb-dCPP conjugates with highest target affinity showed better tumor-to-blood ratios compared to the unmodified antibody. Small animal imaging revealed faster clearance of the conjugates.

Conclusions: The conjugation of antibodies with dCPPs leads to improved target cell binding and to favorable pharmacokinetics. These results demonstrate that dCPPs serve as a powerful tool for improving antibody performance in radioimmunoscintigraphy and therapy.

### - Discussion

### Cell-penetrating peptides dendrimer syntheses and conjugation to the antibody.

The challenging syntheses of the dendrimers worked in general quite satisfactory taken into account that tetramers with a total number of up to 77 amino acid residues (dSynB1 and dpVEC) were synthesized. Two dendrimers - dpVEC and dMTS - had only moderate yields (4 µmol, 6.7%), whereas the majority of the dendrimers were obtained in good yields (≥ 15 µmol or 30%), even the dR9 with its 36 arginine residues.

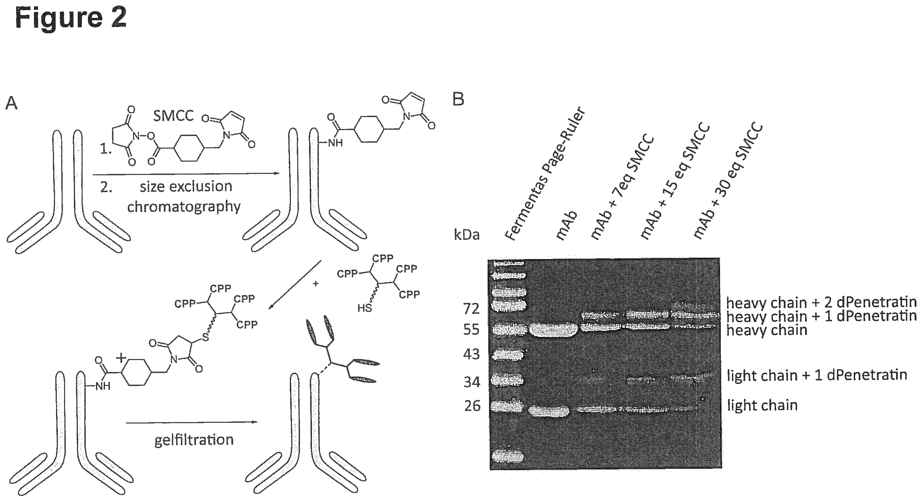

The purified dCPPs were then used in crosslinking reactions employing the heterobifunctional crosslinker SMCC. It has been previously shown that not the size of the molecule, which is attached to the antibody does impact its antigen binding capacity, but the number of molecules (Wangler et al., 2008). The fewer molecules are attached to the mAB the less likely is an inflicted damage to the antigen binding capability; i.e. a single dCPP, which is up to 10 kDa in size can be attached to an antibody without disturbing the antibody performance, whereas coupling of four significantly smaller, non-dendritic CPPs, with 2.5 kDa each, to the mAb are more likely to have a negative effect on the antigen binding capability. Therefore, in the first reaction - the attack of the activated *N*-hydroxyl succinimidyl (NHS) ester by side chain amines of the antibody - different equivalents of the crosslinker SMCC were used to generate a maleimide-activated antibody (Figure 2A). The excess of crosslinker is necessary, as most of the NHS ester will hydrolyze in the aqueous conjugation buffer. An excess between 7- and 30-fold of SMCC was used to determine optimal reaction conditions to yield in average one maleimide molecule on the surface of the antibody. This was done to ensure in the second reaction a single sulfhydryl-mediated dCPP attachment to the maleimide and hence to the antibody. The dCPP concentration was kept constant in the second reaction (15-fold excess) in order to keep the reaction conditions simple and to facilitate complete modification of all maleimide molecules present on the antibody. As demonstrated in Figure 2B, 15 equivalents of SMCC in the first reaction generated mAb-dCPP conjugates with an approximate ratio of 1:1, leaving a small portion of the antibody unmodified. Although a 30-fold excess of the crosslinker yielded almost complete modification of the mAb, it also resulted in more than one dCPP attachment. As outlined before, a single attachment of a large molecule will most likely not affect antigen binding, but multiple crosslinked molecules will. Therefore, the incomplete outcome of the modification reaction with 15-fold excess of SMCC was accepted to prevail antigen binding of the mAB-dCPP conjugates. Excess dCPPs were removed and the conjugate was purified by size exclusion chromatography.

### Cell binding and internalization studies.

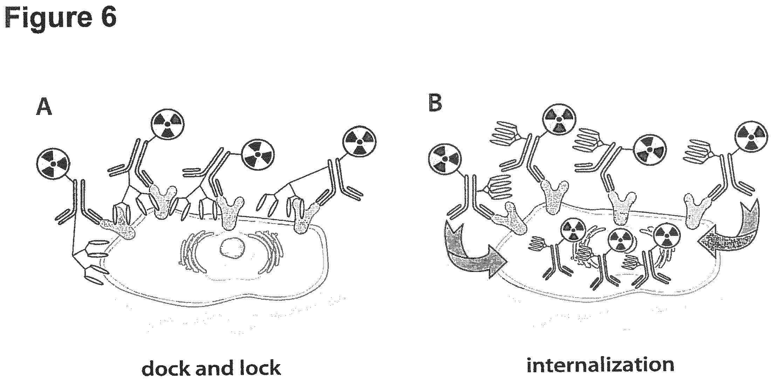

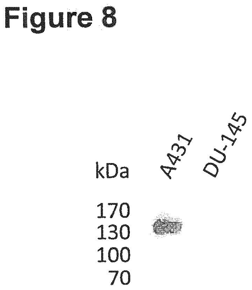

In these studies, the conjugates and the unmodified antibody were incubated with tumor cell lines *in vitro* to examine the influence of the dCPPs on cell binding and internalization. Prior to the assays it was proven that the two cell lines were definitely suitable as model cell lines for the antibody's target - EGFR - expression (A431) and the lack of the antigen (DU-145) by western blot (Figure 8). The cells were then incubated with radioactively labeled mAb and the conjugates thereof. In general a higher binding percentage of the applied dose per one million A431 cells was observed for the conjugates compared to the unmodified mAb. Notably the absolute values were at least twice the mAb binding percentage. For most of the conjugates - dTAT, dPenetratin, dMTS - these values were threefold higher and for dR9 even 4.2-fold. Another interesting finding was that the dCPP-conjugates were internalized but to very different extent. Whereas for some dCPPs high accumulation inside the cells of up to 40% of the applied dose was observed, for other dCPPs this value was barely 10%. The difference between the internalization rates might be due to tetrameric structure of dCPP. As a single CPP together with its cargo is usually capable to translocate into the cell, this uptake might be disturbed by the dendritic structure. Although CPP cell membrane transduction is still not fully understood and an ongoing area of research three mechanisms are proposed, dependent on the CPP these are: direct membrane penetration; uptake via endocytosis; and translocation by transitory structure formation (Madani et al., 2011; Trabulo et al., 2010). The latter was proposed to be the main mechanism of internalization for amphipathic CPPs, such as pVEC and SynB1 (Deshayes et al., 2006). Thus, it is likely that this transitory structure formation cannot occur or it as least disturbed in the dendritic structure, which might explain the low internalization rates for these conjugates. Nevertheless, even those amphipathic dCPPs showed high overall binding rates, which might be explained by a dock and lock mechanism, i.e. the immunoconjugate binds to the antigen present on the target cell and the dCPP locks onto the cell surface (Figure 6A) but the transitory structure formation necessary for membrane translocation is disturbed or slowed down. However, the interactions between the membrane and the dCPP facilitate strong binding interaction between the immunoconjugate and the membrane. For most of the mAb-dCPP the following binding mode can be hypothesized: first, the antibody part binds to the antigen; second, the dCPP subunit binds to the cell membrane; and third facilitates endocytosis or other modes of membrane translocation and subsequent internalization (Figure 6B). The hypothesis that antigen binding occurs prior to and might be crucial for cell penetration is supported by the fact that binding to the control cell line was low for most of the conjugates.

### Biodistribution studies.

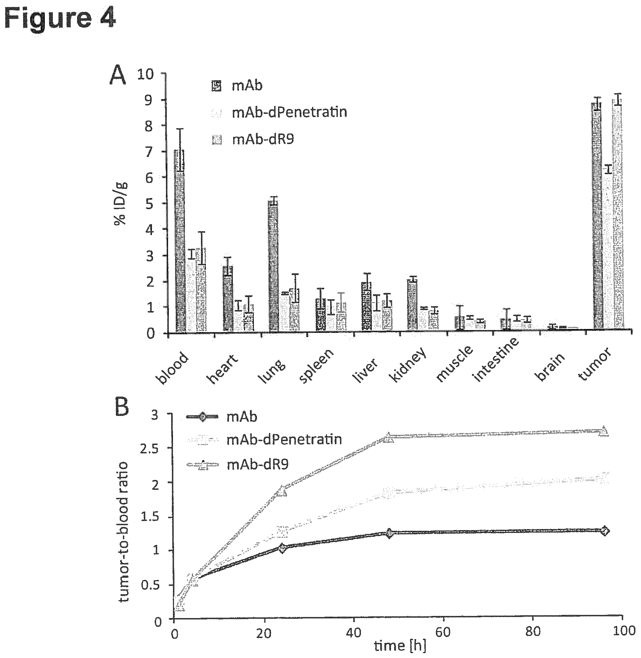

In order to keep the number of laboratory animals low, only for the two most promising mAb-dCPPs and the unmodified antibody organ distribution studies were conducted. In general it was found that the conjugates were enriched in the tumor tissue at approximately the same level as the unmodified mAb. However, all other organs contained very little radioactivity for the conjugates, whereas all well blood perfused organs - kidneys, heart, and lung - contained relatively high radioactivity amounts for the mAb (Figure 4A). The most notable difference between the immunoconjugates and matuzumab was the tumor-to-blood ratio. Within the first 4h after injection, this ratio was approximately the same for all examined radiopharmaceuticals. But already after 24h this ratio developed into different directions. For the conjugates mAb-dPenetratin and mAb-dCPP the tumor-to-blood ratio showed a 23 and 81% improvement, and 47 and 112% after 48h, respectively. Thus, it became obvious that the conjugates bound faster to the tumor lesion compared to unmodified matuzumab. Taking into account that the conjugates still contained a small amount of unmodified mAb, one could hypothesize that the residual activity found in the blood might result from the unconjugated mAb impurity. Anyway, the overall clearance from the blood circuit is definitely improved compared to the unmodified antibody.

### Imaging.

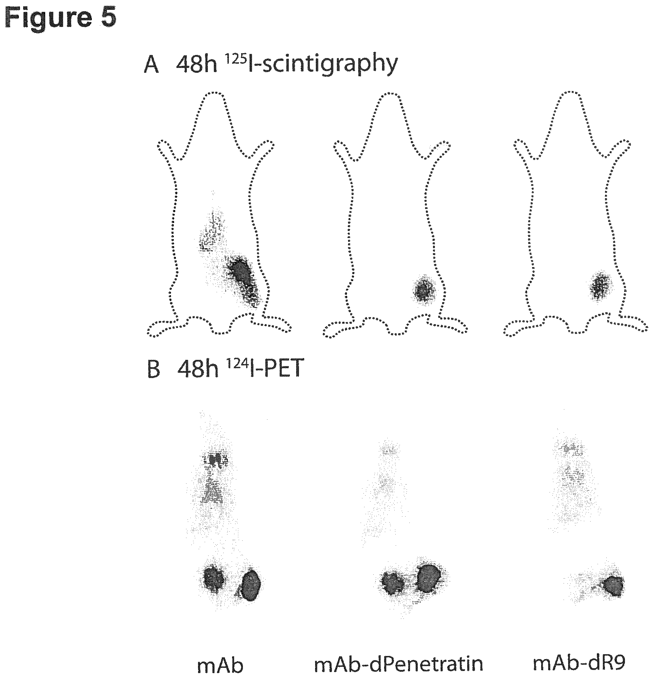

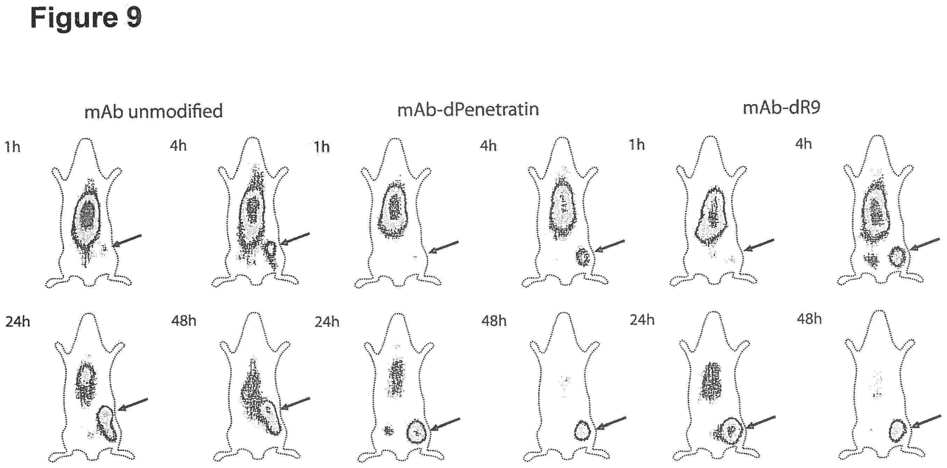

The recorded planar scintigraphic images of the ¹²⁵I-labeled mAb-conjugates are of remarkable quality. In most cases a good contrast, i.e. a good tumor-blood-ratio, for full size antibodies in radioimmunoscintigraphy is not observed until three to four days post injection (Smith-Jones et al., 2003). However, with the here described immunoconjugates 24 hours were more than sufficient to distinguish clearly between the tumor lesion stored activity and the little still circulating activity (Figure 9), which to date, has been achieved only with smaller antibody fragments co-administered with penetratin, but not with full-size mAbs (Jain et al., 2005). The observed differences between the immoconjugates and the unmodified antibody became even more obvious after 48 hours: a significant amount of the unmodified antibody was still not cleared from the blood flow (Figure 5A). This observance is in good agreement with the biodistribution studies and further proves that the conjugates bind more efficiently to the target cells. Consequently the radioimmunoconjugates a faster clearance compared to the unmodified antibody was seen.

The PET images of ¹²⁴I-matuzumab and its conjugates further validates that the unmodified antibody is longer in circulation than its dCPP-modified derivatives. In addition, bladder visualization for mAb and mAb-dPenetratin indicates renal clearance for both, the antibody and the conjugates.

### Conclusions

The conjugation of the EGFR targeting antibody matuzumab to dCPPs improved the antibody performance. This was demonstrated by *in vitro* cell binding experiments in which the immunoconjugates bound up to fourfold stronger to the target cells and for some dCPP-conjugates a large portion was internalized into the target cells. Depending on the intended therapeutic use of the conjugates the dCPP can be chosen. If internalization is desired, e.g. for radioimmunotherapy, as the radiation damage will be more efficient by trapping of the tracer inside the cells, dR9 is a good choice, where one third of the activity was taken up by the cells. For the use in normal immunotherapy a CPP dendrimer, like dpVEC or dSynB1, which increases binding of the antibody to the target cells, but only slightly mediates internalization, is a good candidate to increase antibody-dependent cell-mediated cytotoxicity. The biodistribution studies demonstrated that the conjugates, if compared with the unmodified antibody bind faster and more specific to the target cells, as very little activity was observed in other organs. In addition the radioimmunoconjugates developed favorable tumor-to-blood ratios as they are faster cleared from the blood stream. This was further validated by radioimmunoscintigraphy and PET imaging: the conjugates showed almost no background of circulating activity, whereas a significant amount was visualized for the unmodified antibody.

Slow binding and clearance are the major drawbacks of full size antibodies in radioimmunotherapy and imaging often resulting in radiation damage. This can now be improved by conjugation to cell-penetrating peptide dendrimers. Herein a valuable example is presented for how a full size antibody can be modified with large molecules without reducing the antigen binding capability.

More generally, the method described increases the specificity of tumor uptake of antibodies und thus provides the basis to reduce the side effects.

Notably, the modification strategy works without the necessity to use smaller antibody-based entities, such as single chain antibodies or antibody fragments. Such smaller antibody-based entities can, however, also be used in the conjugates of the invention in order to generate diagnostically and/or therapeutically valuable conjugates. Depending on the desired use of the conjugate(s), the skilled artisan can choose between using a specific full size antibody or its fragments or genetically engineered constructs which might show further clinically interesting features.

**Table 1 CPP sequences.**

| One letter code used. L-amino acids are in upper case, D-amino acids in lower case. Repetitions are written in parenthesis. | |
|---|---|
| Seq ID NO | Sequence |
| 1 | RQIKIWFQNRRMKWKK |
| 2 | YGRKKRRQRRRPPQ |
| 3 | PLSSIFSRIGDP |
| 4 | RRRRRRRRR |
| 5 | AAVALLPAVLLALLAP |
| 6 | RGGRLSYSRRRFSTSTGR |
| 7 | LLIILRRRIRKQAHAHSK |
| 8 | PKKKRKV |
| 9 | RKKRRQRRR |
| 10 | GRKKRRQRRRPPQ |
| 11 | LGTYTQDFNKFHTFPQTAIGVGAP |
| 12 | VQRKRQKLMP |
| 13 | SKKKKTKV |
| 14 | GRKRKKRT |
| 15 | RRRERRAEK |
| 16 | KCPSRRPKR |
| 17 | (VRLPPP)n |
| 18 | (VHLPPP)n |
| 19 | (VKLPPP)n |
| 20 | AVGAIGALFLGFLGAAG |
| 21 | VTVLALGALAGVGVG |
| 22 | IAARIKLRSRQHIKLRHL |
| 23 | DTWPGVEALIRILQQLLFIHFRIGCQH |
| 24 | DAATATRGRSAASRPTERPRAPARSASRPRRPVD |
| 25 | GALFLGWLGAAGSTMGA |
| 26 | MGLGLHLLVLAAALQGA |
| 27 | RQGAARVTSWLGRQLRIAGKRLEGRSK |
| 28 | MANLGYWLLALFVTMWTDVGLCKKRPKP |
| 29 | TRRNKRNRIQEQLNRK |
| 30 | TAKTRYKARRAELIAERR |
| 31 | MDAQTRRRERRAEKQAQWKAAN |
| 32 | RRRRNRTRRNRRRVR |
| 33 | KMTRAQRRAAARRNRWTAR |
| 34 | TRRQRTRRARRNR |
| 35 | RQARRNRRRRWR |
| 36 | DRRRRGSRPSGAERRRRRAAAA |
| 37 | QTRRRERRAEKQAQW |
| 38 | KRPAAIKKAGQAKKKK |
| 39 | TRRSKRRSHRKF |
| 40 | RAGLQFPVGRVHRLLRK |
| 41 | TRSSRAGLQFPVGRVHRLLRK |
| 42 | RHIKIWFQNRRMKWKK |
| 43 | YKQCHKKGGHCFPKEKICLPPSSDFGKMDCRWRWKCCKKGSG |
| 44 | RVIRVWFQNKRCKDKK |
| 45 | SKRTRQTYTRYQTLELEKEFHFNRYITRRRRIDIANALSLSERQIKIWFQNRRMKSKKDR |
| 46 | EKRPRTAFSSEQLARLKREFNENRYLTERRRQQLSSELGLNEAQIKIWFQNKRAKIKKST |
| 47 | GWTLNSAGYLLGKINLKALAALAKKIL |
| 48 | RRRRRRRR |
| 49 | rrrrrrrrr |
| 50 | KLALKLALKALKAALKLA |
| 51 | WEAKLAKALAKALAKHLAKALAKALKACEA |
| 52 | YARAAARQARA |
| 53 | DPKGDPPKGVTVTVTVTVTGKGDPKPD |
| 54 | KKWKMRRNQFWVRVQR |
| 55 | RRWRRWWWRRWWRRWRR |
| 56 | GALFLGFLGAAGSTMGAWSQPKSKRKVC |
| 57 | KETWWETWWTEWSQPKKKRKV |
| 58 | GALFLGWLGAAGSTMGAWSQPKKKRKV |
| 59 | RRQRRTSKLMKR |
| 60 | RRIPNRRPRR |
| 61 | YGRRARRRRRR |
| 62 | SQMTRQARRLYV |
| 63 | SIPPEVKFNKPFVYLI |
| 64 | KKWKMRRNQFWVKVQRG |
| 65 | AAVALLPAVLLALLAVTDQLGEDFFAVDLEAFLQEFGLLPEKE |
| 66 | KKAAAVLLPVLLAAP |
| 67 | INLKALAALAKKIL |
| 68 | MPKKKPTPIQLNP |
| 69 | AAVALLPAVLLALLAK |
| 70 | MNLLRKIVKNRRDEDTQKSSPASAPLDDG |
| 71 | ACSSSPSKHCG |
| 72 | RRLSYSRRRF |
| 73 | PIRRRKKLRRLK |
| 74 | MGLGLHLLVLAAALQGAWSQPKKKRKV |
| 75 | KETWFETWFTEWSQPKKKRKV |
| 76 | (R)n (n ≥ 3) |
| 77 | (K)n (n ≥ 3) |
| 78 | KIAAKSIAKIWKSILKIA |
| 79 | KALAKALAKLWKALAKAA |
| 80 | KLALKLALKWAKLALKAA |
| 81 | KLLAKAAKKWLLLALKAA |
| 82 | KLLAKAALKWLLKALKAA |
| 83 | KALKKLLAKWLAAAKALL |
| 84 | KLAAALLKKWKKLAAALL |
| 85 | KALAALLKKWAKLLAALK |
| 86 | KALAALLKKLAKLLAALK |
| 87 | KLALKLALKALKAALK |
| 88 | KLALKALKAALKLA |
| 89 | KLALKLALKALKAA |
| 90 | KLGLKLGLKGLKGGLKLG |
| 91 | KLALKLALKALQAALQLA |
| 92 | KLALQLALQALQAALQLA |
| 93 | QLALQLALQALQAALQLA |
| 94 | ELALELALEALEAALELA |
| 95 | LKTLATALTKLAKTLTTL |
| 96 | LLKTTALLKTTALLKTTA |
| 97 | LKTLTETLKELTKTLTEL |
| 98 | LLKTTELLKTTELLKTTE |
| 99 | klalklalkalkaalkla |
| 100 | KALKLKLALALLAKLKLA |
| 101 | KKWKMRRNQFWIKIQR |
| 102 | rqikiwfqnrrmkwkk |
| 103 | RQIKIWFPNRRMKWKK |
| 104 | RQPKIWFPNRRKPWKK |
| 105 | RQIKIWFQNRRMKWK |
| 106 | RQIKIWFQNRRMKW |
| 107 | RQIKIWFQNRRMK |
| 108 | RQIKIWFQNRRM |
| 109 | RQIKIWFQNRR |
| 110 | RQIKIWFQNR |
| 111 | RQIKIWFQN |
| 112 | RQIKIWFQ |
| 113 | RQIKIW |
| 114 | QIKIWFQNRRMKWKK |
| 115 | IKIWFQNRRMKWKK |
| 116 | KIWFQNRRMKWKK |
| 117 | IWFQNRRMKWKK |
| 118 | WFQNRRMKWKK |
| 119 | FQNRRMKWKK |
| 120 | QNRRMKWKK |
| 121 | NRRMKWKK |
| 122 | RRMKWKK |
| 123 | RMKWKK |
| 124 | AQIKIWFQNRRMKWKK |
| 125 | RAIKIWFQNRRMKWKK |
| 126 | RQAKIWFQNRRMKWKK |
| 127 | RQIAIWFQNRRMKWKK |
| 128 | RQIKAWFQNRRMKWKK |
| 129 | RQIKIAFQNRRMKWKK |
| 130 | RQIKIWAQNRRMKWKK |
| 131 | RQIKIWFANRRMKWKK |
| 132 | RQIKIWFQARRMKWKK |
| 133 | RQIKIWFQNARMKWKK |
| 134 | RQIKIWFQNRAMKWKK |
| 135 | RQIKIWFQNRRAKWKK |
| 136 | RQIKIWFQNRRMAWKK |
| 137 | RQIKIWFQNRRMKAKK |
| 138 | RQIKIWFQNRRMKWAK |
| 139 | RQIKIWFQNRRMKWKA |
| 140 | CRQIKIWFPNRRMKWKKC |
| 141 | RQIKIWFPNRRMKWKK |
| 142 | RQIKIFFQNRRMKFKK |
| 143 | RQIRIWFQNRRMRWRR |
| 144 | RRRRRRRW |
| 145 | GRKKRRQRRRPWQ |
| 146 | GRKKRRQRRRPWQ |
| 147 | RQIRIWFQNRRMRWRR |
| 148 | RRWRRWWRRWWRRWRR |
| 149 | RQIKIWFQNMRRKWKK |
| 150 | KMDCRWRWKCCKK |
| 151 | MDCRWRWKCCKK |
| 152 | DCRWRWKCCKK |
| 153 | CRWRWKCCKK |
| 154 | RWRWKCCKK |
| 155 | KMDCRWRWKCKK |
| 156 | KMDCRWRWKKK |
| 157 | KMDRWRWKKK |
| 158 | KDCRWRWKCCKK |
| 159 | KCRWRWKCCKK |
| 160 | KRWRWKCCKK |
| 161 | MDCRWRWKXCKK |
| 162 | DCRWRWKXCKK |
| 163 | DCRWRWKCXKK |
| 164 | CRWRWKXCKK |
| 165 | CRWRWKCXKK |
| 166 | RWRWKXCKK |
| 167 | MDCRWRWKXXKK |
| 168 | DCRWRWKXXKK |
| 169 | CRWRWKXXKK |
| 170 | RWRWKXXKK |
| 171 | CRWRWKCSKK |
| 172 | SRWRWKCCKK |
| 173 | SRWRWKCSKK |
| 174 | SRWRWKSCKK |
| 175 | CRWRWKSSKK |
| 176 | SRWRWKSSKK |
| 177 | CRFRWKCCKK |
| 178 | CRWRFKCCKK |
| 179 | CRFRFKCCKK |
| 180 | crwrwkcckk |
| 181 | KCCKWRWRCK |
| 182 | kcckwrwrck |
| 183 | CrWRWKCCKK |
| 184 | CRwRWKCCKK |
| 185 | CRWrWKCCKK |
| 186 | CRWRwKCCKK |
| 187 | CrwrwKCCKK |
| 188 | CRWRWKCGCKK |
| 189 | KCGCRWRWKCGCKK |
| 190 | CRWRWKCG |
| 191 | KMDXRWRWKCCKK |
| 192 | KMDXRWRWKXCKK |
| 193 | KMDXRWRWKXXKK |
| 194 | KMDXRWRWKCXKK |
| 195 | MDCRWRWKCXKK |
| 196 | KMDCRWRWKCSKK |
| 197 | KMDCRWRWKSCKK |
| 198 | KMDSRWRWKCCKK |
| 199 | KMDCRWRWKSSKK |
| 200 | KMDSRWRWKSSKK |
| 201 | KMDSRWRWKSCKK |
| 202 | KMDSRWRWKCSKK |
| 203 | KMDCRWRPKCCKK |
| 204 | KMDCRPRPKCCKK |
| 205 | KMDXRPRPKCCKK |
| 206 | KMDXRPRPKXCKK |
| 207 | KMDXRPRPKCXKK |
| 208 | KMDCRPRPKXCKK |
| 209 | KMDCRPRPKCXKK |
| 210 | rkkrrqrrr |
| 211 | rrrqrrkkr |
| 212 | RKKRRRESRKKRRRES |
| 213 | GRPRESGKKRKRKRLKP |
| 214 | GKRKKKGKLGKKRDP |
| 215 | GKRKKKGKLGKKRPRSR |
| 216 | RKKRRRESRRARRSPRHL |
| 217 | SRRARRSPRESGKKRKRKR |
| 218 | VKRGLKLRHVRPRVTRMDV |
| 219 | SRRARRSPRHLGSG |
| 220 | LRRERQSRLRRERQSR |
| 221 | GAYDLRRRERQSRLRRRERQSR |
| 222 | VPMLK |
| 223 | VPTLK |
| 224 | VPALR |
| 225 | VSALK |
| 226 | PMLKE |
| 227 | VPALK |
| 228 | VSLKK |
| 229 | VSGKK |
| 230 | KLPVM |
| 231 | IPMIK |
| 232 | KLGVM |
| 233 | KLPVT |
| 234 | VPMIK |
| 235 | IPALK |
| 236 | IPMLK |
| 237 | VPTLQ |
| 238 | QLPVM |
| 239 | ELPVM |
| 240 | VPTLE |
| 241 | vptlk |
| 242 | AYRIKPTFRRLKWKYKGKFW |
| 243 | HARIKPTFRRLKWKYKGKFW |
| 244 | HYRIKPTARRLKWKYKGKFW |
| 245 | HYRIKPTFRRLAWKYKGKFW |
| 246 | HYRIKPTFRRLKWKYKGKFA |
| 247 | VNADIKATTVFGGKYVSLTTP |
| 248 | GKYVSLTTPKNPTKRRITPKDV |
| 249 | TKRRITPKDVIDVRSVTTEINT |
| 250 | RSVTTEINTLFQTLTSIAEKVDP |
| 251 | AEKVDPVKLNLTLSAAAEALTGLGDK |
| 252 | GLGDKFGESIVNANTVLDDLNSRMPQSRHDIQQL |
| 253 | GDVYADAAPDLFDFLDSSVTTARTINA |
| 254 | ARTINAQQAELDSALLAAAGFGNTTADVFDRG |
| 255 | ADVFDRGGPYLQRGVADLVPTATLLDTYSP |
| 256 | LDTYSPELFCTIRNFYDADRPDRGAAA |
| 257 | TKRRITPKDVIDVRSVTTEINT |
| 258 | TKRRITPDDVIDVRSVTTEINT |
| 259 | TKRRITPKKVIDVRSVTTEINT |
| 260 | TKRRITPKDVIDVRSVTTKINT |
| 261 | TKRRITPKDVIDV |
| 262 | TKRRITPKDVIDVESVTTEINT |
| 263 | TARRITPKDVIDVRSVTTEINT |
| 264 | TKAARITPKDVIDVRSVTTEINT |
| 265 | HHHHHHTKRRITPKDVIDVRSVTTEINT |
| 266 | KLWMRWYSPTTRRYG |
| 267 | DSLKSYWYLQKFSWR |
| 268 | RTLVNEYKNTLKFSK |
| 269 | IPSRWKDQFWKRWHY |
| 270 | GYGNCRHFKQKPRRD |
| 271 | KNAWKHSSCHHRHQI |
| 272 | RVREWWYTITLKQES |
| 273 | QQHLLIAINGYPRYN |
| 274 | WKCRRQCFRVLHHWN |
| 275 | RLWMRWYSPTTRRYG |
| 276 | KLWMRWYSATTRRYG |
| 277 | KLWMRWYSPWTRRYG |
| 278 | RLWMRWYSPWTRRYG |
| 279 | RLWMRWYSPWTRRWG |
| 280 | ALWMRWYSPTTRRYG |
| 281 | RAWMRWYSPTTRRYG |
| 282 | RLAMRWYSPTTRRYG |
| 283 | RLWARWYSPTTRRYG |
| 284 | RLWMAWYSPTTRRYG |
| 285 | RLWMRAYSPTTRRYG |
| 286 | RLWMRWASPTTRRYG |
| 287 | RLWMRWYAPTTRRYG |
| 288 | RLWMRWYSPATRRYG |
| 289 | RLWMRWYSPTARRYG |
| 290 | RLWMRWYSPTTARYG |
| 291 | RLWMRWYSPTTRAYG |
| 292 | RLWMRWYSPTTRRAG |
| 293 | RLWMRWYSPTTRRYA |
| 294 | RLLMRLYSPTTRRYG |
| 295 | RLFMRFYSPTTRRYG |
| 296 | RLIMRIYSPTTRRYG |
| 297 | RLVMRVYSPTTRRYG |
| 298 | RLYMRYYSPTTRRYG |
| 299 | YGRKKKRRQRRR |
| 300 | ALIILRRRIRKQAHAHSK |
| 301 | LAIILRRRIRKQAHAHSK |
| 302 | LLAILRRRIRKQAHAHSK |
| 303 | LLIALRRRIRKQAHAHSK |
| 304 | LLIIARRRIRKQAHAHSK |
| 305 | LLIILARRIRKQAHAHSK |
| 306 | LLIILRARIRKQAHAHSK |
| 307 | LLIILRRAIRKQAHAHSK |
| 308 | LLIILRRRARKQAHAHSK |
| 309 | LLIILRRRIARKQAHAHSK |
| 310 | LLIILRRRIRAQAHAHSK |
| 311 | LLIILRRRIRKAAHAHSK |
| 312 | LLIILRRRIRKQaHAHSK |
| 313 | LLIILRRRIRKQAAAHSK |
| 314 | LLIILRRRIRKQAHaHSK |
| 315 | LLIILRRRIRKQAHAASK |
| 316 | LLIILRRRIRKQAHAHAK |
| 317 | LLIILRRRIRKQAHAHSA |
| 318 | KSHAHAQKRIRRRLIILL |
| 319 | lliilrrrirkqahahsk |
| 320 | RRIRPRP |
| 321 | RRIRPRPPRLPRPRP |
| 322 | RRIRPRPPRLPRPRPRPLPFPRPG |
| 323 | RRIRPRPPRLPRPRPRP |
| 324 | PRPPRLPRPRPRPLPFPRPG |
| 325 | PPRLPRPRPRPLPFPRPG |
| 326 | RLPRPRPRPLPFPRPG |
| 327 | PRPRPRPLPFPRPG |
| 328 | PRPRPLPFPRPG |
| 329 | PRPLPFPRPG |
| 330 | RVTSWLGRQLRIAGKRLEGRSK |
| 331 | GRQLRIAGKRLEGRSK |
| 332 | RRVTSWLGRQLRIAGKRLEGRSK |
| 333 | RVRSWLGRQLRIAGKRLEGRSK |
| 334 | GRQLRIAGKRLRGRSK |
| 335 | GRQLRIAGRRLRGRSR |
| 336 | GRQLRRAGRRLRGRSR |
| 337 | GRQLRIAGRRLRRRSR |
| 338 | GRQLRRAGRRLRRRSR |
| 339 | RQLRIAGRRLRGRSR |
| 340 | rsrgrlrrgairlqrg |
| 341 | KLIKGRTPIKFGKADCDRPPKHSQNGMGK |
| 342 | KLIKGRTPIKFGKADCDRPPKHSQNGM |
| 343 | KLIKGRTPIKFGKADCDRPPKHSQNGK |
| 344 | KGRTPIKFGKADCDRPPKHSQNGMGK |
| 345 | KLIKGRTPIKFGKADCDRPPKHSGK |
| 346 | KLIKGRTPIKFGKARCRRPPKHSGK |
| 347 | KLIKGRTPIKFGK |
| 348 | KRIPNKKPGKKTTTKPTKKPTIKTTKKDLKPQTTKPK |
| 349 | KRIPNKKPGKKTTTKPTKKPTIKTTKKDLK |
| 350 | KRIPNKKPGKKTTTKPTKKPTIKTTKK |
| 351 | KRIPNKKPGKKTTTKPTKKPTIK |
| 352 | KRIPNKKPGKKTTTKPTKK |
| 353 | KRIPNKKPGKKT |
| 354 | KRIPNKKPGKK |
| 355 | KRIPNKKPKK |
| 356 | KKPGKKTTTKPTKKPTIKTTKK |
| 357 | KKPGKKTTTKPTKK |
| 358 | KKPTIKTTKK |
| 359 | KKTTTKPTKK |
| 360 | KSICKTIPSNKPKKK |
| 361 | KTIPSNKPKKK |
| 362 | KPRSKNPPKKPK |
| 363 | DRDDRDDRDDRDDRDDR |
| 364 | ERERERERERERER |
| 365 | WRWRWRWRWRWRWR |
| 366 | DRDRDRDRDR |
| 367 | GALFLGFLGAAGSTMGAWSQPKKKRKV |
| 368 | DRRRRGSRPSGAERRRR |
| 369 | NRARRNRRRVR |
| 370 | RTRRNRRRVR |
| 371 | RNRSRHRR |
| 372 | MVRRFLVTLRIRRACGPPRVRV |
| 373 | FVTRGCPRRLVARLIRVMVPRR |
| 374 | VRRFLVTLRIRRA |
| 375 | RVRILARFLRTRV |
| 376 | RVRVFVVHIPRLT |
| 377 | VIRVHFRLPVRTV |
| 378 | MVRRFLVTLRIRRACGPPRVRVFVVHIPRLTGEWAAP |
| 379 | FRVPLRIRPCVVAPRLVMVRHTFGRIARWVAGPLETR |
| 380 | AGYLLGKINLKALAALAKKIL |
| 381 | GTKMIFVGIKKKEERADLIAYLKKA |
| 382 | KKKEERADLIAYLKKA |
| 383 | KMIFVGIKKKEERA |
| 384 | KMIFVGIKKK |
| 385 | EKGKKIFIMK |
| 386 | KGKKIFIMK |
| 387 | RRRRNRTRRNRRRVRGC |
| 388 | TRRQRTRRARRNRGC |
| 389 | KMTRAQRRAAARRNRWTARGC |
| 390 | KLTRAQRRAAARKNKRNTRGC |
| 391 | NAKTRRHERRRKLAIERGC |
| 392 | MDAQTRRRERRAEKQAQWKAANGC |
| 393 | TAKTRYKARRAELIAERRGC |
| 394 | SQMTRQARRLYBGC |
| 395 | KRRIRRERNKMAAAKSRNRRRELTDTGC |
| 396 | RIKAERKRMRNRIAASKSRKRKLERIARGC |
| 397 | KRARNTEAARRSRARKLQRMKQGC |
| 398 | KCFQWQRNMRKVRGPPVSCIKR |
| 399 | KCFQWQRNMRKVRGPPVSC |
| 400 | KCFQWQRNMRKVRGPPVSSIKR |
| 401 | KCFQWQRNMRKVR |
| 402 | FQWQRNMRKVRGPPVS |
| 403 | QWQRNMRKVRGPPVSCIKR |
| 404 | QWQRNMRKVR |
| 405 | KCFMWQEMLNKAGVPKLRCARK |
| 406 | KWFETWFTEWPKKRK |
| 407 | GLWRALWRLLRSLWRLLWRA |
| 408 | GLWWRLWWRLRSWFRLWFRA |
| 409 | DAATATRGRSAASRPTQRPRAPARSASRPRRPVE |
| 410 | GALFLGFLGAAGSTMGAWSQPKKKRKV |
| 411 | GALFLGFLGAAGSTMGAWSQPKSKRKV |
| 412 | AKVKDEPQRRSARLSAKPAPPKPEPKPKKAPAKK |
| 413 | akvkdepqrrsarlsakpappkpepkpkkapakk |
| 414 | PSSSSSSRIGDP |
| 415 | vrlpppvrlpppvrlppp |
| 416 | VELPPPVELPPPVELPPP |
| 417 | ALWMTLLKKVLKAAAKAALNAVLVGANA |
| 418 | ALWKTLLKKVLKA |
| 419 | ALWKTLLKKVLKAPKKKRKV |
| 420 | PKKKRKVALWKTLLKKVLKA |
| 421 | VKRKKKPALWKTLLKKVLKA |
| 422 | RQARRNRRRALWKTLLKKVLKA |
| 423 | RQARRNRRRC |
| 424 | EEEAAGRKRKKRT |
| 425 | EEE |
| 426 | EEEAA |
| 427 | EEEAAKKK |
| 428 | FFFAAGRKRKKRT |
| 429 | AAGRKRKKRT |
| 430 | YYYAAGRKRKKRT |
| 431 | MVTVLFRRLRIRRACGPPRVRV |
| 432 | AGYLLGKINLKALAALAKKIL |
| 433 | GKKKKRKREKL |
| 434 | ERKKRRRE |
| 435 | FKKFRKF |
| 436 | YTQDFNKFHTFPQTAIGVGAP |
| 437 | DFNKFHTFPQTAIGVGAP |
| 438 | KFHTFPQTAIGVGAP |
| 439 | TFPQTAIGVGAP |
| 440 | GYGRKKRRQRRRG |
| 441 | FLGKKFKKYFLQLLK |
| 442 | FLIFIRVICIVIAKLKANLMCKT |
| 443 | KKAAQIRSQVMTHLRVI |
| 444 | YIVLRRRRKRVNTKRS |
| 445 | RRKLSQQKEKK |
| 446 | VQAILRRNWNQYKIQ |
| 447 | KTVLLRKLLKLLVRKI |
| 448 | LLKKRKVVRLIKFLLK |
| 449 | KLPCRSNTFLNIFRRKKPG |
| 450 | KKICTRKPRFMSAWAQ |
| 451 | rggrlsysrrrfststgr |
| 452 | rrlsysrrrf |
| 453 | RGGRLAYLRRRWAVLGR |
| 454 | MANLGCWMLVLFVATWSDLGLCKKRPKP |
| 455 | MVKSKIGSWILVLFVAMWSDVGLCKKRPKP |
| 456 | LLGDFFRKSKEKIGKEFKRIVQRIKDFLRNLVPRTESC |
| 457 | GIGKFLHSAKKWGKAFVGQIMNC |
| 458 | TRSSRAGLQWPVGRVHRLLRKGGC |
| 459 | YGRKKRRQRRR |
| 460 | EKRPRTAFSSEQLARLKREFNENRYLTTERRRQQLSSELGLNEAQIKIWFQNKRAKIKKST |
| 461 | GRRRRRRRRRPPQ |
| 462 | GALFLGFLGAAGSTMGAWSQPKKKRKV |
| 463 | GALFLAFLAAALSLMGLWSQPKKKRRV |
| 464 | MLLLTRRRST |
| 465 | CGNKRTRGC |
| 466 | TSPLNIHNGQKL |
| 467 | GLRKRLRKFRNKIKEK |
| 468 | GLLEALAELLEGLRKRLRKFRNKIKEK |
| 469 | CVQWSLLRGYQPC |
| 470 | RQIKIFFQNRRMKWKK |
| 471 | ASMWERVKSIIKSSLAAASNI |
| 472 | ASMWERVKSIIKSSLAAASNI |
| 473 | DPKGDPKGVTVTVTVTVTGKGDPKPD |
| 474 | CSIPPEVKFNPFVYLI |
| 475 | csippevkfnpfvyli |
| 476 | PFVYLI |
| 477 | NKPILVFY |
| 478 | YKQCHKKGGKKGSG |
| 479 | YKQCHKKGGXKKGSG |
| 480 | GSGKKGGKKHCQKY |
| 481 | GSGKKGGKKICQKY |
| 482 | YTAIAWVKAFIRKLRK |
| 483 | IAWVKAFIRKLRKGPLG |
| 484 | LIRLWSHLIHIWFQNRRLKWKKK |
| 485 | KKKKKKGGFLGFWRGENGRKTRSAYERMCILKGK |
| 486 | RLSGMNEVLSFRWL |
| 487 | GPFHFYQFLFPPV |
| 488 | GSPWGLQHHPPRT |
| 489 | AAVALLPAVLLALLAPEILLPNNYNAYESYKYPGMFIALSK |
| 490 | AAVALLPAVLLALLAPVQRKRQKLMP |
| 491 | MGLGLHLLVLAAALQGAKKKRKV |
| 492 | WEAALAEALAEALAEHLAEALAEALEALAA |
| 493 | GLFEALLELLESLWELLLEA |
| 494 | GLFKALLKLLKSLWKLLLKA |
| 495 | GLFRALLRLLRSLWRLLLRA |
| 496 | CGAYDLRRRERQSRLRRRERQSR |
| 497 | RKKRRRESRKKRRRESC |
| 498 | CVKRGLKLRHVRPRVTRDV |
| 499 | CRQIKIWFQNRRMKWKK |
| 500 | PPKKSAQCLRYKKPE |
| 501 | DPVDTPNPTRRKPGK |
| 502 | KRVSRNKSEKKRR |
| 503 | GRRHHCRSKAKRSRHH |
| 504 | SARHHCRSKAKRSRHH |
| 505 | SRAHHCRSKAKRSRHH |
| 506 | SRRAHCRSKAKRSRHH |
| 507 | SRRHACRSKAKRSRHH |
| 508 | SRRHHARSKAKRSRHH |
| 509 | SRRHHCRAKAKRSRHH |
| 510 | SRRHHCRSAAKRSRHH |
| 511 | SRRHHCRSKAARSRHH |
| 512 | SRRHHCRSKAKASRHH |
| 513 | SRRHHCRSKAKRARHH |
| 514 | SRRHHCRSKAKRSAHH |
| 515 | RRHHCRSKAKRSR |
| 516 | GRKGKHKRKKLP |
| 517 | GKKKKKKKKK |
| 518 | GKRVAKRKLIEQNRERRR |
| 519 | GRKLKKKKNEKEDKRPRT |
| 520 | GKKTNLFSALIKKKKTA |
| 521 | GRRERNKMAAAKCRNRRR |
| 522 | GKRARNTEAARRSRARKL |
| 523 | GRRRRATAKYRTAH |
| 524 | GKRRRRATAKYRSAH |
| 525 | GRRRRKRLSHRT |
| 526 | GRRRRRERNK |
| 527 | GKHRHERGHHRDRRER |
| 528 | GKKKRKLSNRESAKRSR |
| 529 | MIIYRDLISH |
| 530 | MIIYRDLIS |
| 531 | MIIYRDLI |
| 532 | IIYRDLISH |
| 533 | MIIYRDL |
| 534 | MIIYRD |
| 535 | IYRDLISH |
| 536 | AIIYRDLIS |
| 537 | MAIYRDLIS |
| 538 | MIAYRDLIS |
| 539 | MIIARDLIS |
| 540 | MIIYADLIS |
| 541 | MIIYRALIS |
| 542 | MIIYRDAIS |
| 543 | MIIYRDLAS |
| 544 | MIIYRDLIA |
| 545 | MIIYRDLISKK |
| 546 | MIIYRDKKSH |
| 547 | MIIFRDLISH |
| 548 | MIISRDLISH |
| 549 | QIISRDLISH |
| 550 | CIISRDLISH |
| 551 | MIIYRALISHKK |
| 552 | MIIYRIAASHKK |
| 553 | MIIRRDLISE |
| 554 | MIIYRAEISH |
| 555 | MIIYARRAEE |
| 556 | MIIFRIAASHKK |
| 557 | MIIFRALISHKK |
| 558 | MIIFRAAASHKK |
| 559 | FIIFRIAASHKK |
| 560 | LIIFRIAASHKK |
| 561 | WIIFRIAASHKK |
| 562 | WIIFRAAASHKK |
| 563 | WIIFRALISHKK |
| 564 | MIIFRIAAYHKK |
| 565 | WIIFRIAAYHKK |
| 566 | MIIFRIAATHKK |
| 567 | WIIFRIAATHKK |
| 568 | MIIFKIAASHKK |
| 569 | WIIFKIAASHKK |
| 570 | MIIFAIAASHKK |
| 571 | LIIFRILISHKK |
| 572 | MIIFRILISHKK |
| 573 | LIIFRILISHRR |
| 574 | LIIFRILISHHH |
| 575 | LIIFRILISHK |
| 576 | LIIFRILISHR |
| 577 | LIIFRILISH |
| 578 | LIIFAIAASHKK |
| 579 | LIIFAILISHKK |
| 580 | RILQQLLFIHFRIGCRHSRI |
| 581 | RILQQLLFIHFRIGCRH |
| 582 | RILQQLLFIHFRIGC |
| 583 | RIFIHFRIGC |
| 584 | RIFIRIGC |
| 585 | RILQQLLFIHF |
| 586 | RIFIGC |
| 587 | FIRIGC |
| 588 | DTWAGVEAIIRILQQLLFIHFR |
| 589 | IGCRH |
| 590 | GYGRKKRRGRRRTHRLPRRRRRR |
| 591 | KRIIQRILSRNS |
| 592 | KRIHPRLTRSIR |
| 593 | PPRLRKRRQLNM |
| 594 | MHKRPTTPSRKM |
| 595 | RQRSRRRPLNIR |
| 596 | RIRMIQNLIKKT |
| 597 | SRRKRQRSNMRI |
| 598 | QRIRKSKISRTL |
| 599 | PSKRLLHNNLRR |
| 600 | HRHIRRQSLIML |
| 601 | PQNRLQIRRHSK |
| 602 | PPHNRIQRRLNM |
| 603 | SMLKRNHSTSNR |
| 604 | GSRHPSLIIPRQ |
| 605 | SPMQKTMNLPPM |
| 606 | NKRILIRIMTRP |
| 607 | HGWZIHGLLHRA |
| 608 | AVPAKKRZKSV |
| 609 | PNTRVRPDVSF |
| 610 | LTRNYEAWVPTP |
| 611 | SAETVESCLAKSH |
| 612 | YSHIATLPFTPT |
| 613 | SYIQRTPSTTLP |
| 614 | AVPAENALNNPF |
| 615 | SFHQFARATLAS |
| 616 | QSPTDFTFPNPL |
| 617 | HFAAWGGWSLVH |
| 618 | HIQLSPFSQSWR |
| 619 | LTMPSDLQPVLW |
| 620 | FQPYDHPAEVSY |
| 621 | FDPFFWKYSPRD |
| 622 | FAPWDTASFMLG |
| 623 | FTYKNFFWLPEL |
| 624 | SATGAPWKMWVR |
| 625 | SLGWMLPFSPPF |
| 626 | SHAFTWPTYLQL |
| 627 | SHNWLPLWPLRP |
| 628 | SWLPYPWHVPSS |
| 629 | SWWTPWHVHSES |
| 630 | SWAQHLSLPPVL |
| 631 | SSSIFPPWLSFF |
| 632 | LNVPPSWFLSQR |
| 633 | LDITPFLSLTLP |
| 634 | LPHPVLHMGPLR |
| 635 | VSKQPYYMWNGN |
| 636 | NYTTYKSHFQDR |
| 637 | AIPNNQLGFPFK |
| 638 | NIENSTLATPLS |
| 639 | YPYDANHTRSPT |
| 640 | DPATNPGPHFPR |
| 641 | TLPSPLALLTVH |
| 642 | HPGSPFPPEHRP |
| 643 | TSHTDAPPARSP |
| 644 | MTPSSLSTLPWP |
| 645 | VLGQSGYLMPMR |
| 646 | QPIIITSPYLPS |
| 647 | TPKTMTQTYDFS |
| 648 | NSGTMQSASRAT |
| 649 | QAASRVENYMHR |
| 650 | HQHKPPPLTNNW |
| 651 | SNPWDSLLSVST |
| 652 | KTIEAHPPYYAS |
| 653 | EPDNWSLDFPRR |
| 654 | HQHKPPPLTNNW |
| 655 | GLWRALWRLLRSLWRLLWKA |
| 656 | GLWRALWRALWRSLWKLKRKV |
| 657 | GLWRALWRALRSLWKLKRKV |
| 658 | GLWRALWRGLRSLWKLKRKV |
| 659 | GLWRALWRGLRSLWKKKRKV |
| 660 | GLWRALWRALWRSLWKLKWKV |
| 661 | GLWRALWRALWRSLWKSKRKV |
| 662 | GLWRALWRALWRSLWKKKRKV |
| 663 | GLWRALWRALWRSLWKLKRKV |
| 664 | GLWRALWRLLRSLWRLLWSQPKKKRKV |
| 665 | YARAARRAARR |
| 666 | PARAARRAARR |
| 667 | YPRAARRAARR |
| 668 | YRRAARRAARA |
| 669 | YGRAARRAARR |
| 670 | YAREARRAARR |
| 671 | YEREARRAARR |
| 672 | YKRAARRAARR |
| 673 | YARKARRAARR |
| 674 | YKRKARRAARR |
| 675 | YGRRARRAARR |
| 676 | YGRRARRRARR |
| 677 | YGRRRRRRRRR |
| 678 | YRRRRRRRRRR |
| 679 | GKINLKALAALAKKIL |
| 680 | GRKKRRQRRRPPQGRKKRRQRRRPPQGRKKRRQRRRPPQ |
| 681 | GEQIAQLIAGYIDIILKKKKSK |
| 682 | AAVALLPAVLLALLAPRKKRRQRRRPPQ |
| 683 | AAVALLPAVLLALLAPRKKRRQRRRPPQC |
| 684 | RKKRRQRRRPPQCAAVALLPAVLLALLAP |
| 685 | RRRQRRKRGGDIMGEWGNEIFGAIAGFLG |
| 686 | RRRQRRKRGGDIMGEWGNEIFGAIAGFLG |
| 687 | YGRKKRRQRRRGCYGRKKRRQRRRG |
| 688 | AAVALLPAVLLALLAPRRRRRR |
| 689 | RLWRALPRVLRRLLRP |
| 690 | AAVALLPAVLLALLAPSGASGLDKRDYV |
| 691 | LLETLLKPFQCRICMRNFSTRQARRNHRRRHRR |
| 692 | AAVACRICMRNFSTRQARRNHRRRHRR |
| 693 | RQIKIWFQNRRMKWKKDIMGEWGNEIFGAIAGFLG |
| 694 | SGRGKQGGKARAKAKTRSSRAGLQFPVGRVHRLLRKG |
| 695 | SGRGKQGGKARAKAKTRSSRAGLQFPVGRVHRLLRKGC |
| 696 | KKDGKKRKRSRKESYSVYVYKVLKQ |
| 697 | KGSKKAVTKAQKKDGKKRKRSRKESYSVYVYKVLKQ |
| 698 | KETWWETWWTEWSQPGRKKRRQRRRPPQ |
| 699 | RVIRWFQNKRCKDKK |
| 700 | LGLLLRHLRHHSNLLANI |
| 701 | KLWSAWPSLWSSLWKP |
| 702 | GLGSLLKKAGKKLKQPKSKRKV |
| 703 | FKQqQqQqQqQq |
| 704 | YRFK |
| 705 | YRFKYRFKYRLFK |
| 706 | WRFKKSKRKV |
| 707 | WRFKAAVALLPAVLLALLAP |
| 708 | WRFKWRFK |
| 709 | WRFKWRFKWRFK |
| 710 | KGSKKAVTKAQKKDGKKRKRSRKESYSVYVYKVLKQ |
| 711 | RGSRRAVTRAQRRDGRRRRRSRRESYSVYVYRVLRQ |
| 712 | RVIRWFQNKRSKDKK |
| 713 | AAVALLPAVLLALLAPRKKRRQRRRPPQ |
| 714 | CWKKK |
| 715 | CWKKKKKKKK |
| 716 | CWKKKKKKKKKKKKK |
| 717 | CWKKKKKKKKKKKKKKKKKK |
| 718 | KKKKKKKKKKKKKKKKKKK |
| 719 | kkwkmrrGaGrrrrrrrrr |
| 720 | APWHLSSQYSRT |
| 721 | AAVALLPAVLLALLAKKNNLKDCGLF |
| 722 | AAVALLPAVLLALLAKKNNLKECGLY |
| 723 | AHALCLTERQIKIWFQNRRMKWKKEN |
| 724 | AHALCPPERQIKIWFQNRRMKWKKEN |
| 725 | AYALCLTERQIKIWFANRRMKWKKEN |
| 726 | GGVCPKILKKCRRDSDCPGACICRGNGYCGSGSD |
| 727 | GGVCPKILAACRRDSDCPGACICRGNGYCGSGSD |
| 728 | GGVCPAILKKCRRDSDCPGACICRGNGYCGSGSD |
| 729 | GGVCPKILAKCRRDSDCPGACICRGNGYCGSGSD |
| 730 | GGVCPKILKACRRDSDCPGACICRGNGYCGSGSD |
| 731 | GLPVCGETCVGGTCNTPGCKCSWPVCTRN |
| 732 | GLPVCGETCVGGTCNTPGCTCSWPKCTRN |
| 733 | GRCTKSIPPICFPD |
| 734 | RQIKIWFQNRRMKWKKTYADFIASGRTGRRNAI |
| 735 | GRKKRRQRRRPPQTYADFIASGRTGRRNAI |
| 736 | AGYLLGKINLKALAALAKKIL |
| 737 | AGYLLGKINLKALAALAKKILTYADFIASGRTGRRNAI |
| 738 | RRRRRRRRRRR |
| 739 | RRRRRRRRRRRTYADFIASGRTGRRNAI |
| 740 | rrrrrrrrrk |
| 741 | rRRRRRRRr |
| 742 | rRrRrRrRr |
| 743 | KCFQWQRNMRKVRGPPVSCIKR |
| 744 | kcfqwqrnmrkvrgppvscikr |
| 745 | KLALKLALKALKAALKLAGC |
| 746 | KLULKLULKULKAULKLUGC |
| | Wherein U is Aib (α-aminoisobutyric acid) |
| 747 | GGGARKKAAKAARKKAAKAARKKAAKAARKKAAKA |
| 748 | GRKKRRQRRRPPQC |
| 749 | TRQARRNRRRRWRERQRGC |
| 750 | RRRRNRTRRNRRRVRGC |
| 751 | KMTRAQRRAAARRNRWTARGC |
| 752 | TRRQRTRRARRNRGC |
| 753 | RIKAERKRMRNRIAASKSRKRKLERIARGC |
| 754 | KRRIRRERNKMAAAKSRNRRRELTDTGC |
| 755 | WLRRIKAWLRRIKALNRQLGVAA |
| 756 | crkkrrqrrr |
| 757 | crrrrrrrrr |
| 758 | ckkkkkkkkk |
| 759 | GRKKRRQRRRPP |
| 760 | rrrrrrrr |

The following examples and drawings illustrate the present invention without, however, limiting the same thereto.

### BRIEF DESCRIPTION OF THE DRAWINGS

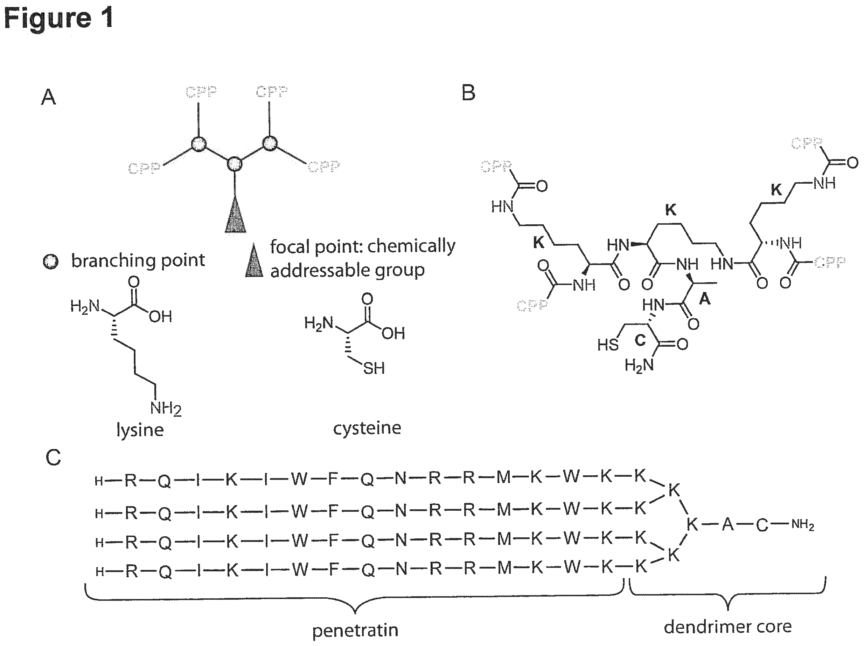

**Figure 1****:** *Overview of cell-penetrating dendrimer syntheses.*
   A) Schematic presentation of the dendrimer showing branching (lysine) and focal (cysteine) points.
   B) Chemical structure of the dendrimer core. Lysine branching points are shown in dark orange and the crosslinking-referring sulfhydryl group of cysteine in red.
   C) Schematic dCPP structure in one letter code exemplified by penetratin.
**Figure 2****:** *Conjugation of the mAb with dCPPs.*
   A) Schematic overview of antibody activation by SMCC and subsequent coupling to the dCPP. In the first step, the mAb is activated as maleimide, which is attacked by the free sulfhydryl-group of the dCPP in the second step. Excess dCPP is removed by gelfiltration.
   B) Influence of SMCC excesses in the conjugation reaction analyzed by SDS-PAGE. The image is shown in reverse grayscale to improve contrast.

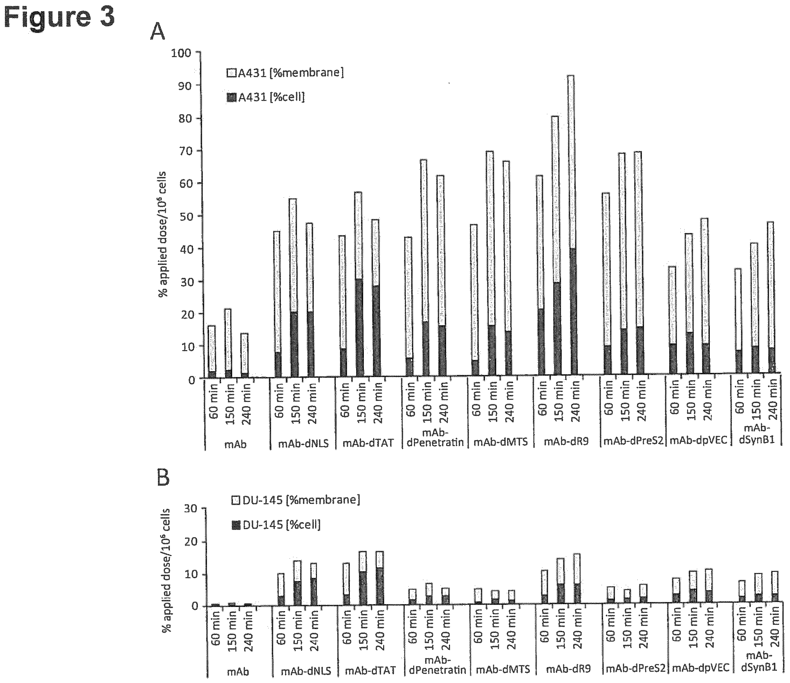

**Figure 3****:** *Binding of matuzumab and of the mAb-dCPP conjugates*
   to A) A431 cells and B) to the control cell line DU-145. The internalized portion of the activity is illustrated in the lower part of the bars in black, the membrane bound activity by the upper part shown in gray. For clarity reasons the error bars are omitted.
**Figure 4****:**
   A) Organ and tissue distribution of the ¹³¹I-labeld antibody, mAb-dPenetratin and mAb-dR9 96 hours after injection into A431 xenografts.
   B) Time-dependent development of the tumor-to-blood ratio of the naked antibody and the immunoconjugates.
**Figure 5****:**
   A) Planar scintigraphic images of A431 tumor-bearing nude mice 48 hours after injection of ¹²⁵I-labeled mAb, mAb-dPenetratin and mAb-dR9. B) As Figure 5A, except that the radioiodination was carried out with iodine-124 and that static PET images were recorded.
**Figure 6****:** *Possible mechanisms for target cell binding by the mAb-dCPPs.*
   A) For little internalized conjugates, mainly with amphipathic dCPPs, a dock and lock mechanism is hypothesized. The antibody binds to its antigen, but the internalization is disturbed, as a transitory structure necessary for uptake, is not formed by the CPP dendrimer, but the dCPP still locks onto the cell surface.
   B) Internalization mechanism: The antibody binds to the target structure on the cell surface and then the dCPP promotes cell uptake either by direct translocation or endocytosis.
**Figure 7****:** *Schematic overview of the structures of the synthesized dCPPs.*
**Figure 8****:** *Western blot analysis of EGFR-expression by A431 and DU-145 cells.* Matuzumab was used as primary antibody. Horseradish peroxidase conjugated goat anti-human IgG served as secondary antibody.
**Figure 9****:**
   Planar scintigraphic images of A431 xenografts injected with ¹²⁵I-labeled matuzumab, mAb-dPenetratin and mAb-dR9. The images were recorded at 1, 4, 24, and 48 hours p.i.

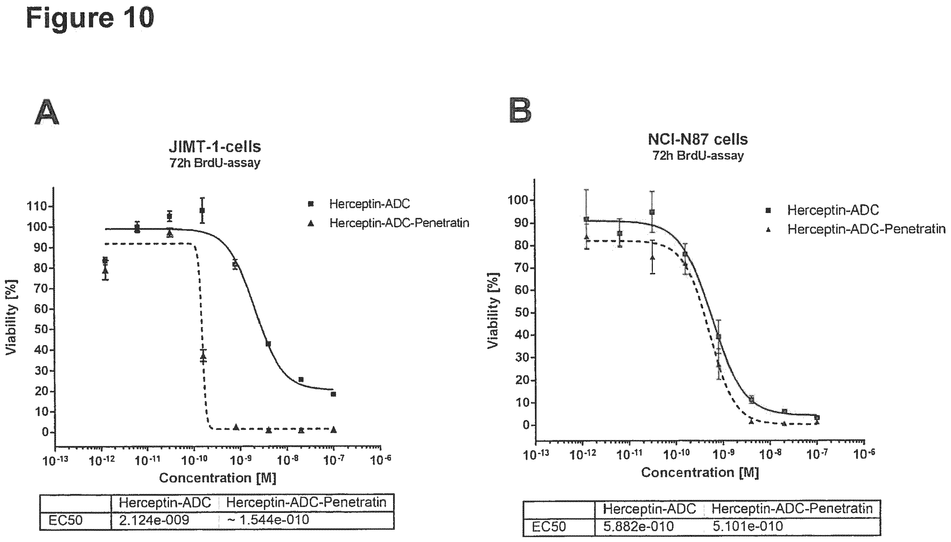

**Figure 10****:** *Cytotoxicity assays with Penetratin antibody conjugates*
   A) Cytotoxicity assay with EGFR-positive cells
   B) Cytotoxicity assay with EGFR-negative cells

   The cells were treated for 72 hours with different concentrations of a toxin-antibody-Penetratin (4-mer) conjugate (Herceptin-ADC-Penetratin) or a toxin-antibody-conjugate without Penetratin (Herceptin-ADC), respectively. Then, the cell viability was tested via BrdU incorporation. The tables show the molar EC50 concentrations.

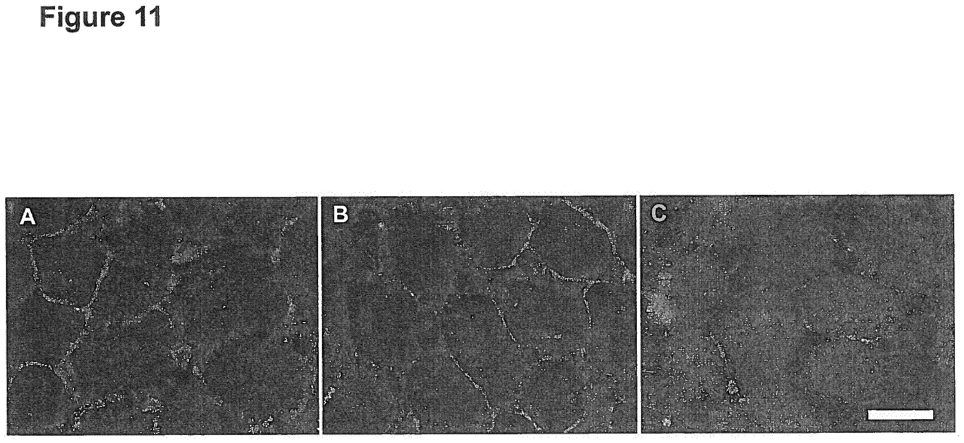

**Figure 11****:** *Cellular uptake of Penetratin antibody conjugates*
   Incubation of A431 cells (EGFR positive cells) with
   A) Native EGFR antibody
   B) EGFR antibody Pen10-conjugate (4-mer) (Pen-10 = 10-mer-Penetratin partial structure, a control peptide causing reduced internalization)
   C) EGFR antibody Penetratin-conjugate (4-mer)

   Experimental conditions: Incubation for 1 hour with a concentration of 1 µg/ml of all 3 antibodies, fixation of the cells with PFA after incubation, staining with fluorescence-labelled secundary anti-huIgG antibody (AlexaFluor-488), image acquisition with 100x objective lens, fluorescence microscope Keyence BioRevo BZ-9000
   Bar: 20 µm

### EXAMPLES

### 1. Materials and Methods

### 1.1 General.

All chemicals were purchased from Sigma-Aldrich (Schnelldorf, Germany) at the highest available purity unless otherwise stated. Fmoc-protect amino acid builing blocks were purchased from Bachem (Bubendorf, Switzerland). Anti-EGFR antibody Matuzumab (EMD72000) was provided by Merck KGaA (Darmstadt, Germany) (see also EP 0 531 472 B1, US 5,558,864, WO 2009/043490 A1). Radioactive iodine 1-125 and 1-131 isotopes were purchased from Perkin-Elmer (Rodgau, Germany) and Eckert & Ziegler (Berlin, Germany) for 1-124.

### 1.2 Synthesis of dendritic cell-penetrating peptides (dCPPs).

Branched structures of the CPPs were obtained by manual generation of a solid phase peptide synthesis (SPPS) resin, presenting four amino groups - α- and ε-amines of lysines - as branching points, one alanine residue as spacer and a cysteine as focal group for crosslinking with the antibody using 9-fluorenylmethoxycarbonyl (Fmoc)-protected L-α-amino acids. The desired sequence - K₂KAC - was yielded on resin by subsequent incubation of 1.5 g Amphisphere 40 RAM (0.4 mmol/g), swollen in dichloromethane (DCM) and washed four times using dimethyl formamide (DMF), with 4 eq of Fmoc-Cys(Trt)-OH, 4 eq Fmoc-Ala-OH, 3 eq Fmoc-Lys(Fmoc)-OH and 6 eq Fmoc-Lys(Fmoc)-OH respectively. For the coupling steps (75 min) equal amounts of *O*-(Benzotriazol-1-yl)-*N*,*N*,*N*,*N*-tetramethyluronium hexafluoro- phosphate (HBTU) and 500 µL *N*,*N*-Diisopropylethylamine (DIPEA) were added. After each coupling the resin was washed five times with DMF and Fmoc was cleaved by consecutive incubation with 10 mL (1 min) and 7 mL (10 min) of 25% (v/v) piperidine solution in DMF followed by DMF washing steps (5x).

This dried resin (150 mg, for each dCPP), containing the four-branch core molecule, was then used in a fully automated peptide synthesizer (Applied Biosystems 433 A, Carlsbad, CA, USA) with 10 eq (Fmoc)-protected amino acid building blocks. HBTU/DIPEA in NMP was used as a coupling reagent. Fmoc deprotection efficiency was monitored at 301 nm. Cleavage from solid support was performed with TFA/H₂O/triisopropylsilane (95:2.5:2.5) for 2h at ambient temperature. The peptide was precipitated with cold diethyl ether and pelletized (4000 rpm, 4 °C, 5 min), washed with diethyl ether, dried in vacuo, and subject to preparative HPLC purification (Waters, Eschborn, Germany; XBridge BEH 130 PREP; C18 column, 5 µm pore size, 150x19 mm). Fractions containing the product were identified by using HPLC-ESI-MS with Orbitrap technology (Exactive, Thermo Fisher Scientific, Waltham, MA, USA) equipped with an Agilent 1200 HPLC system and a Hypersil Gold C18 column (Thermo Fisher Scientific, Bonn, Germany; 1.9 µm, 200x2.1 mm), pooled and freeze-dried.

### 1.3 Conjugation of dCPPs with the monoclonal antibody.

Conjugation was carried out using the commercially available, hetero-bifunctional crosslinker SMCC (Thermo Fisher Scientific, Bonn, Germany). Crosslinking was carried out according to the manufacturer's protocol. In brief, 7, 15 and 30 equivalents of the crosslinker (15 mg/mL in DMF) were added to the monoclonal antibody (mAb) solution (1.5 mg/mL in PBS) and incubated at room temperature for 30 min. Excess crosslinker was removed by using pre-equilibrated (PBS, pH 7.4) NAP-10 columns (GE Healthcare, Freiburg, Germany). The maleimide-activated mAb solutions were concentrated to 1 mg/mL (concentration determination using Bradford test (Bradford, 1976)) using 100k Amicon Ultra-0.5 mL centrifugal filters (Merck Millipore, Darmstadt, Germany). Then the maleimide-activated mAb was incubated with 15 eq of the eight different dCPPs (20 mg/mL) at room temperature for 45 min. Reactions with 15 eq SMCC in the first coupling yielded the best results, i.e. predominantly a single attachment of a dCPP to the mAb. Excess dCPP and in part unmodified mAb were removed by size exclusion chromatography using a FPLC manifold equipped with a Superdex 200, 10/300 GL (GE Healthcare) column and PBS as the mobile phase. Fractions containing the antibody-dCPP conjugates were identified by UV-monitoring (λ=280 nm), pooled and concentrated to approximately 0.1 mg/mL using 30k Amicon Ultra-15 centrifugal filters (Merck Millipore).

### 1.4 Western Blot.

A431 and DU-145 cells were grown to 80% confluency. Cells were washed twice with 10 mL ice-cold PBS pH 7.4, scraped off and centrifuged (3 min, 1000 rpm). The pellet was washed with 5 mL PBS (3 min, 1000 rpm) and lysed by addition of 2 mL 1% Triton X-100 followed by centrifugation (10 min, 2700 rpm). The supernatant was loaded onto a polyacrylamide gel and SDS-PAGE was performed. Proteins were transferred from the gel to a nitrocellulose membrane using a Mini Trans-Blotter (100 V for 90 min). Non-specific binding sites were blocked by 5% non-fat milk powder in TBST buffer (1h, RT). Matuzumab (anti-EGFR-antibody; 1:1000 dilution) was used as primary antibody and incubated with the membrane overnight at 4°C. After washing in TBST, the nitrocellulose membrane was incubated with horseradish peroxidase conjugated goat polyclonal anti-human IgG antibody (Thermo Fisher Scientific, Bonn, Germany, 1:1000 dilution) in blocking buffer at room temperature for 60 min. Antibody binding was determined using an enhanced chemiluminescence detection system (Western Lightening Plus ECL, Perkin-Elmer) according to the manufacturer's protocol and exposures were recorded on hyperfilms (10 s to 3 min).

### 1.5 Radiolabeling.

A modified version of the established chloramine-T method (Hunter & Greenwood, 1962) employing [¹²⁵I]-NaI or [¹³¹I]-NaI was used to introduce the radioactive iodine at random tyrosine side-chains of the mAb-dCPP conjugate. In brief, 30 µL of the conjugates (0.1 mg/mL) was mixed with 20 µL of phosphate buffer (0.25 M, pH 7.5). A solution containing 1-30 MBq [¹²⁵I]-NaI or [¹³¹I]-NaI in 10 µM NaOH was added and the labeling reaction was started by addition of an aqueous chloramine-T solution (10 mM, 5 µL). After 30 s, the labeling reaction was quenched by adding a saturated aqueous solution of methionine (10 µL). The labeling reaction mixture was passed over a PBS equilibrated buffer exchange column (NAP-10, GE Healthcare) and 300 µL fractions were collected. 5 µL of each fraction was transferred to a new vial and analyzed for γ-radiation using a γ-counter (LB 2111, Berthold Technologies, Bad Wildbad, Germany). Fractions 4-6 usually contained the radioactively labeled immunoreagent and were pooled. For in vivo experiments, the volume was reduced to 100 µL *in vacuo,* and for cell binding experiments the solution was used as was.

### 1.6 Cell binding and uptake assays.

For binding experiments approximately 5×10⁵ cells of the EGFR-positive cell line A431 or the EGFR-negative, control cell line DU-145 cells were seeded into six-well plates and cultivated in 3 mL/well of RPMI-1640 (with 10% fetal calf serum - FCS) at 37°C in a 5% CO₂ incubator. After 24 h the medium was replaced with 1 mL fresh medium (without FCS) containing 0.8-1.2×10⁶ cpm of ¹²⁵I-labeled mAb-dCPP conjugate and incubated for 60, 150 or 240 min at 37°C. After incubation the medium was removed and cells were washed three times with 1 mL ice cold PBS in order to remove unbound radiolabeled mAb-dCPP conjugates. To determine membrane bound activity, each well was incubated with 1 mL glycine buffer (50 mM glycine-HCl, pH 2.2) for 10 min at room temperature. The cells were washed again with PBS and subsequently lysed using 0.5 mL 0.3 mM sodium hydroxide solution. Radioactivity of the membrane bound fraction (glycine wash) and the internalized fraction (sodium hydroxide lysis) was measured using a γ-counter (LB 951G, Berthold Technologies). The radioactivity was calculated as percentage applied dose per 10⁶ cells.

### 1.7 In vivo experiments.

All animal experiments were carried out in conformity with German and European animal protection laws.

### 1.8 Biodistribution studies.

Radioactivity amounts of approximately 1 MBq of ¹³¹I-labeled mAb-dCPPs in PBS were administered intravenously into female six-week old, immunodeficient, A431 tumor-bearing BALB/c nude mice (Charles River, Sulzfeld, Germany). After 1, 4, 24 and 48 h the mice (n=3; 18-22 g) were sacrificed, and selected tissue/blood samples (heart, lung, spleen, liver, kidney, muscle, intestine, brain and tumor) were removed, drained of blood, weighed, and the radioactivity determined using a γ-counter (LB 951G, Berthold Technologies). The percentage of injected dose per gram of tissue (%ID/g) was calculated.

### 1.9 Small animal imaging.

*Planar scintigraphy studies* were performed using female BALB/c nude mice (Charles River, Sulzfeld, Germany), carrying subcutaneously transplanted A431 tumors. A cell suspension of 5×10⁶ cells in 100 µL OPTI-MEM (Life Technologies, Darmstadt, Germany) was injected subcutaneously into the hind leg of the animals and the tumors were grown to a size of 1.0 cm³ in 10-12 days. Selected ¹²⁵I-labeled mAb-dCPP conjugates (7-10 MBq) were injected into the tail vein of the animals and planar scintigraphic images were recorded, while mice were anesthetized by 3-4% sevoflurane (Abbott, Wiesbaden, Germany), at defined time points using a γ-imager (Biospace Lab, Paris, France).

*Small-animal PET imaging* was carried out using female BALB/c nude mice (Charles River) with xenografted A431 tumors. A suspension of 5×10⁶ A431 cells in 100 µL OPTI-MEM (Life Technologies) was injected subcutaneously into the hind leg of the mice. Tumors were grown to a size of 0.5-0.7 cm³ in 11 days. PET scans, of anesthetized mice (3-4% sevoflurane, Abbott) in prone position, were performed using a Inveon microPET system (Siemens, Knoxville, TN, USA). ¹²⁴I-labeled mAb-CPP conjugates (10-12 MBq in 100 µL PBS) were injected intravenously, and static images were acquired after 4, 24 and 48 h. Image data reconstructions and analyses were carried out using Inveon Research Workplace software (Siemens, Knoxville, TN, USA).

### 2. Results

### 2.1 Syntheses of cell-penetrating peptides dendrimers.

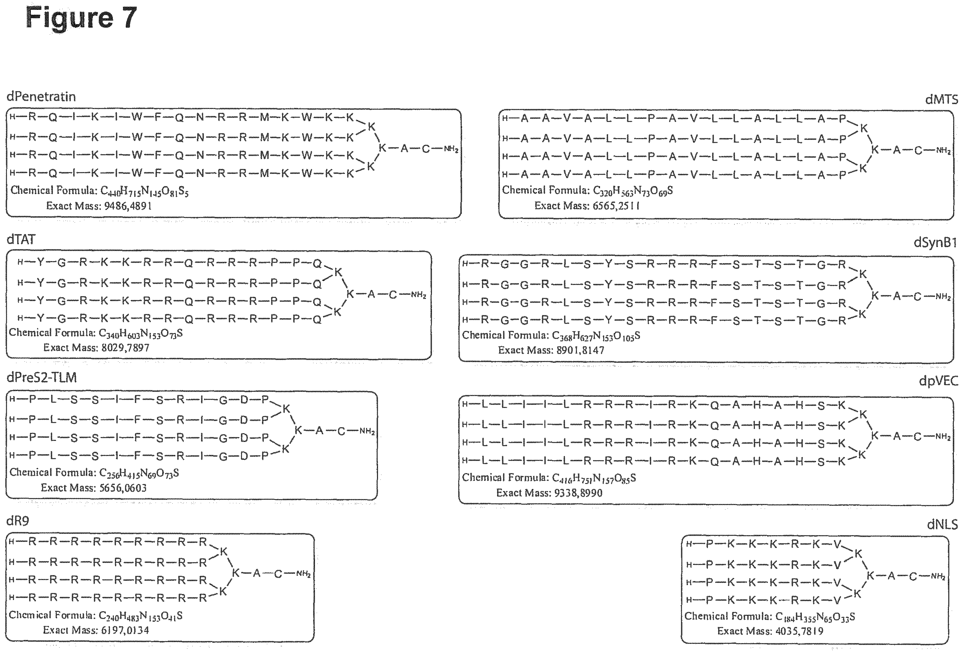

In the first step the core of the dendrimer was synthesized using Fmoc-based solid phase peptide synthesis (SPPS). A cysteine residue as orthogonal, chemically addressable sulfhydryl group for crosslinking was coupled via its carboxyl-group onto the solid support (Figure 1A), followed by an alanine residue, which served as a linker. In the next step the branching points were coupled to the resin. Due to their α- and ε-amino groups, lysine qualifies to fulfill this task (Tam, 1988). By simultaneous deprotection of both amino groups two coupling positions are generated for the next amino acid during SPPS. In the final step of the core peptide synthesis, an additional lysine residue is coupled, and a tetravalent resin was gained, i.e. dendrimers with four branches will be obtained (Figure 1B). This core peptide containing resin was used as a scaffold in SPPS for the generation of eight different tetravalent, dendritic cell-penetrating peptides (Table 2). After cleavage from solid support and side chain deprotection, the dendrimers were obtained as *C*-terminally amides for enhanced stability and their identities were proven by high-resolution mass spectrometry (Table 2). The structures of the generated dCPPs are exemplified by the dendritic tetravalent penetratin (Figure 1C). An overview on the schematic structures of the synthesized dCPPs can be found in Figure 7.

**Table 2. Synthesized dCPPs, sequences, molecular weight and references.**

| **dCPP** | **Sequence of the Monomer** | **Calc. M_{w} of dCPP [Da]** | **Observed (M+H)⁺ [Da]** | **Reference** |
|---|---|---|---|---|
| dPenetratin | RQIKIWFQNRRMKWKK | 9486 | 9487 | Dupont et al., 2011 |
| dTAT (47-60) | YGRKKRRQRRRPPQ | 8029 | 8030 | Vives et al., 1997 |
| dPreS2-TLM | PLSSIFSRIGDP | 5656 | 5657 | Oess etal., 2000 |
| dR9 | RRRRRRRRR | 6197 | 6198 | Mitchell et al., 2000 |
| dMTS | AAVALLPAVLLALLAP | 6565 | 6566 | Kersemans et al., 2008 |
| dSynB1 | RGGRLSYSRRRFSTSTGR | 8901 | 8902 | Rousselle et al., 2000 |
| dpVEC | LLIILRRRIRKQAHAHSK | 9338 | 9340 | Elmquist et al., 2001 |
| dNLS | PKKKRKV | 4035 | 4036 | Kalderon et al., 1984 |
| Abbreviations: | | | | |
| Prefix d: dendritic; | | | | |
| CPP: cell-penetrating peptide; | | | | |
| TAT: human immunodeficiency virus-derived trans-activator of transcription; | | | | |
| preS2-TLM: hepatitis B virus-preS2-domain-derived translocation motif; | | | | |
| MTS: membrane translocation signal; | | | | |
| SynB1: synthetic porcine protegrin 1-derived CPP; | | | | |
| pVEC: vascular endothelial cadherin-derived CPP; | | | | |
| NLS: nuclear localization signal. | | | | |

### 2.2 Conjugation of the dCPPs with a monoclonal antibody.

The epidermal growth factor receptor (EGFR) specific, humanized, monoclonal antibody Matuzumab (EMD72000, Merck, see also EP 0 531 472 B1, US 5,558,864, WO 2009/043490 A1) was used in conjugation experiments. Crosslinking of the individual dCPPs was carried out employing the heterobifunctional crosslinker SMCC (Figure 2A). To determine, the optimal crosslinker-to-antibody ratio the mAb was incubated with 7, 15 or 30 equivalents of SMCC. Amine groups of the mAb attacked the activated ester of SMCC, resulting in maleimide-activated antibody. Excess crosslinker was then removed by size exclusion chromatography using buffer exchange columns. Subsequently, the conjugation experiment was carried out using the maleimide-activated mAb and a 15-fold excess of the corresponding dCPP to allow efficient coupling between the mAb-maleimide and the sulfhydryl group of the dCPP. Exemplary, the reaction is shown for dendritic, tetravalent penetratin (dPenetratin, Figure 2B). 15 equivalents of SMCC in the first reaction turned out to be ideal for the desired 1:1 or less coupling of the mAb to the dCPP.

### 2.3 Western blot.

To test the binding of the immunoconjugates to EGFR-expressing cells, the expression of the target antigen was validated by western blot. Therefore, the whole cell lysates of the EGFR-positive human epidermoid carcinoma cell line A431 and the EGFR-negative human prostate carcinoma cell line DU-145 were prepared and used in western blot analysis. The binding of the EGFR-targeting antibody matuzumab was visualized by a HRP-conjugated goat anti-human IgG antibody. The western blot (Figure 8) revealed that matuzumab exclusively binds to A431, where a clear band at approximately 130 kDa is visible, which is in good agreement with the molecular weight of EGFR (132 kDa). No binding of the mAb to the control cell line DU-145 was observed.

### 2.4 Cell binding and internalization experiments.

The antibody and the conjugates were labeled with iodine-125 at a random tyrosine side chain using chloramine-T and [¹²⁵I]-NaI, as outlined in the methods section. The labeled -antibody and the eight immunoconjugates respectively were added to the cell culture medium and incubated with the EGFR-positive cell line A431 for 60, 150 or 240 min. Control experiments were carried out accordingly with the EGFR-negative cell line DU-145. To be able to distinguish between membrane bound activity and internalized activity, the cells were washed with glycine buffer (pH 2.2) first to remove membrane-bound activity. In the second step the cells were lysed and internalized activity was measured. The measurement revealed that 22 ± 0.2% of the applied dose of the radiolabeled, unmodified matuzumab bound to the EGFR-positive cells A431 (Figure 3A), whereas no binding was observed for the control cell line DU-145. In addition only a small portion of the activity was internalized into the EGFR-positive cells (3%, Figure 3A, black part of column). For the antibody, if modified with cell-penetrating peptide dendrimers, these values were in general higher and binding of the applied dose to the cells ranged from 47 ± 0.5% (mAb-dSynB1) to 92 ± 1.2% (mAb-dR9). Internalization rates were also higher, with a maximum of 38 ± 0.9% (mAb-dR9) and a minimum of 9 ± 0.7% (mAb-dSynB1). Binding of the immunoconjugates to the control cell line (Figure 3B) was low (<10%) to moderate (16 ± 1.1% for mAb-dTAT, 14 ± 0.6% for mAb-dR9 and 13 ± 0.6% for mAb-dNLS). The detailed results are visualized in Figure 3.

Furthermore, Figure 10 shows that a toxin-conjugated antibody furthermore conjugated with Penetratin (4-mer) exhibits increased cell toxicity compared to the toxin-conjugated antibody without Penetratin (Figure 10A).

In addition, Figure 11 shows fluorescence imaging results showing the cellular uptake of Penetratin antibody conjugates into A431 cells. As can be seen, EGFR antibody Penetratin-conjugate (4-mer) is internalized in A431 cells (Figure 11C), whereas native EGFR antibody is not internalized (Figure 11A) and the conjugate with Pen10 (4-mer), the control peptide, known to cause lower internalization rates, shows only reduced internalization (Figure 11B).

### 2.5 Biodistribution studies.

The two most promising immunoconjugates, based on the cell binding and internalization experiments, were chosen for biodistribution studies. These two were mAb-dPenetratin and mAb-dR9. Although mAb-R9 was more unspecific - 14 ± 0.6% binding to the control cell line - than other conjugates, it had the highest binding value of the eight different conjugates (92 ± 1.2% binding). The immunoconjugate of matuzumab and dendritic penetratin showed good binding to A431 cells (68 ± 0.9% binding) and low affinity for the control cell line (maximum 5 ± 0.2%). As control, the unmodified antibody was used. All three compounds were labeled with iodine-131 and injected into A431 tumor-bearing mice. At different time points the mice were sacrificed, dissected and organs were examined for radioactivity uptake. This is exemplified by Figure 4A, where the biodistribution after 96 hours is shown. At this time point the differences between the dCPP-modified antibody and the wildtype became most obvious. Tumor binding was high for all three compounds (8.9 ± 0.2% for mAb-dR9, 8.7 ± 0.3% for mAb and 6.2 ± 0.2% for mAb-dPenetratin), whereas binding to other organs is less than 1.7%, except for the unmodified mAb. There, high values of 2.0 ± 0.1%, 2.6 ± 0.4% and 5.1 ± 0.2% were observed for the kidneys, the heart and the lung, respectively. Most significantly, the blood contained 7.1 ± 0.8% of the injected dose for the unmodified mAb; for mAb-dPenetratin and mAb-dR9 the blood contained 3.1 ± 0.2% and 3.3 ± 0.6% respectively, resulting in favorable tumor-to-blood ratios for the conjugates. As depicted in Figure 4B, within the first four hours after administration of the antibody and the immunoconjugates, similar values for the tumor-to-blood ratios were observed. However, after 24h there was a significant difference between the conjugates and the unmodified antibody for this ratio, which was even higher after 48h. Over the full course of the 96h examination period, the tumor-to-blood ratio reached a value of 2.7 for mAb-dR9 and 2.0 for mAb-dPenetratin, whereas the unmodified antibody did not exceed 1.3.

### 2.6 Small animal imaging experiments.

In the first set of experiments, planar scintigraphic images were recorded of athymic nude mice, with A431 tumors xenografted into the upper hind limb. The antibody and the immunoconjugates mAb-dR9 and mAb-dPenetratin were labeled with iodine-125. Then the radioactive compounds were administered intravenously into individual rodents, in order to record planar images after 1, 4, 24 and 48h (Figure 9). After 48h (Figure 5A) the differences between the unmodified and the conjugated antibody became obvious. In all mice high accumulation of the different radiopharmaceuticals was observed at the upper hind limb tumor site. A significant amount of the applied dose was still in circulation for the unmodified antibody, whereas almost no background was observed for the conjugates mAB-dPenetratin and mAb-dR9.

In the second experimental setup, the antibody and its conjugates were radiolabeled with the positron emitter iodine-124 as outlined in the material and methods section. Again, these radiopharmaceuticals were administered intravenously into individual A431 nude mice xenografts and static PET images were recorded. As observed for the planar ¹²⁵I-images, the PET images showed that the conjugates had a faster clearance from the blood than the unmodified antibody. In addition, accumulation of radioactivity in the urinary tract was observed for mAb and mAb-dPenetratin. For all three examined radiopharmaceuticals a significant amount of the applied dose was found in the thyroid with the unconjugated antibody showing the highest value.

### REFERENCES

Ausubel, F.M. et al. (2001) Current Protocols in Molecular Biology, Wiley & Sons, Hoboken, NJ, USA.
Bradford MM. A rapid and sensitive method for the quantitation of microgram quantities of protein utilizing the principle of protein-dye binding. Anal Biochem. May 7 1976;72:248-254.
Carter P. Improving the efficacy of antibody-based cancer therapies. Nat Rev Cancer. Nov 2001;1(2):118-129.
Cornelissen B, Hu M, McLarty K, Costantini D, Reilly RM. Cellular penetration and nuclear importation properties of 111In-labeled and 123I-labeled HIV-1 tat peptide immunoconjugates in BT-474 human breast cancer cells. Nucl Med Biol. Jan 2007;34(1):37-46.
Deshayes S, Plenat T, Charnet P, Divita G, Molle G, Heitz F. Formation of transmembrane ionic channels of primary amphipathic cell-penetrating peptides. Consequences on the mechanism of cell penetration. Biochim Biophys Acta. Nov 2006;1758(11):1846-1851.
Dupont E, Prochiantz A, Joliot A. Penetratin story: an overview. Methods Mol Biol. 2011;683:21-29.
Elmquist A, Lindgren M, Bartfai T, Langel U. VE-cadherin-derived cell-penetrating peptide, pVEC, with carrier functions. Exp Cell Res. Oct 1 2001;269(2):237-244.
Farkas P, Korcová J, Kronek J, Bystricky S. Preparation of synthetic polyoxazoline based carrier and Vibrio cholerae O-specific polysaccharide conjugate vaccine. Eur J Med Chem. 2010 Feb;45(2):795-9.
Fonseca SB, Pereira MP, Kelley SO. Recent advances in the use of cell-penetrating peptides for medical and biological applications. Adv Drug Deliv Rev. Sep 30 2009;61(11):953-964.
Gautam A, Singh H, Tyagi A, Chaudhary K, Kumar R, Kapoor P, Raghava GP. CPPsite: a curated database of cell penetrating peptides. Database (Oxford). 2012 Mar 7;2012:bas015. Print 2012.
Howl J, Nicholl ID, Jones S. The many futures for cell-penetrating peptides: how soon is now? Biochem Soc Trans. Aug 2007;35(Pt 4):767-769.
Hu M, Chen P, Wang J, Chan C, Scollard DA, Reilly RM. Site-specific conjugation of HIV-1 tat peptides to IgG: a potential route to construct radioimmunoconjugates for targeting intracellular and nuclear epitopes in cancer. Eur J Nucl Med Mol Imaging. Mar 2006;33(3):301-310.
Hu M, Chen P, Wang J, Scollard DA, Vallis KA, Reilly RM. 123I-labeled HIV-1 tat peptide radioimmunoconjugates are imported into the nucleus of human breast cancer cells and functionally interact in vitro and in vivo with the cyclin-dependent kinase inhibitor, p21(WAF-1/Cip-1). Eur J Nucl Med Mol Imaging. Mar 2007;34(3):368-377.
Hunter WM, Greenwood FC. Preparation of iodine-131 labelled human growth hormone of high specific activity. Nature. May 5 1962;194:495-496.
Jain M, Chauhan SC, Singh AP, Venkatraman G, Colcher D, Batra SK. Penetratin improves tumor retention of single-chain antibodies: a novel step toward optimization of radioimmunotherapy of solid tumors. Cancer Res. Sep 1 2005;65(17):7840-7846.
Jay JI, Lai BE, Myszka DG, Mahalingam A, Langheinrich K, Katz DF, Kiser PF. Multivalent benzoboroxole functionalized polymers as gp120 glycan targeted microbicide entry inhibitors. Mol Pharm. 2010 Feb 1;7(1):116-29.
Jeger S, Zimmermann K, Blanc A, Grünberg J, Honer M, Hunziker P, Struthers H, Schibli R. Site-specific and stoichiometric modification of antibodies by bacterial transglutaminase. Angew Chem Int Ed Engl. 2010 Dec 17;49(51):9995-7.
Kalderon D, Roberts BL, Richardson WD, Smith AE. A short amino acid sequence able to specify nuclear location. Cell. Dec 1984;39(3 Pt 2):499-509.
Kaminski MS, Zelenetz AD, Press OW, et al. Pivotal study of iodine I 131 tositumomab for chemotherapy-refractory low-grade or transformed low-grade B-cell non-Hodgkin's lymphomas. J Clin Oncol. Oct 1 2001;19(19):3918-3928.
Kersemans V, Kersemans K, Cornelissen B. Cell penetrating peptides for in vivo molecular imaging applications. Curr Pharm Des. 2008;14(24):2415-2447.
Khandare J, Calderón M, Dagia NM, Haag R. Multifunctional dendritic polymers in nanomedicine: opportunities and challenges. Chem Soc Rev. 2012;41(7):2824-48. Review.
Madani F, Lindberg S, Langel U, Futaki S, Graslund A. Mechanisms of cellular uptake of cell-penetrating peptides. JBiophys. 2011;2011:414729.
Mammen M, Choi S-K, Whitesides GM. Polyvalent interactions in biological systems: Implications for design and use of multivalent ligands and inhibitors. Angew. Chem. Int. Ed. 1998; 37: 2754-2794. Review.
Mitchell DJ, Kim DT, Steinman L, Fathman CG, Rothbard JB. Polyarginine enters cells more efficiently than other polycationic homopolymers. JPept Res. Nov 2000;56(5):318-325.
Oess S, Hildt E. Novel cell permeable motif derived from the PreS2-domain of hepatitis-B virus surface antigens. Gene Ther. May 2000;7(9):750-758.
Oldham RK, Dillman RO. Monoclonal antibodies in cancer therapy: 25 years of progress. J Clin Oncol. Apr 10 2008;26(11):1774-1777.
Pouget JP, Navarro-Teulon I, Bardies M, et al. Clinical radioimmunotherapy--the role of radiobiology. Nat Rev Clin Oncol. Dec 2011;8(12):720-734.
Rousselle C, Clair P, Lefauconnier JM, Kaczorek M, Scherrmann JM, Temsamani J. New advances in the transport of doxorubicin through the blood-brain barrier by a peptide vector-mediated strategy. Mol Pharmacol. Apr 2000;57(4):679-686.
Sarko D, Beijer B, Garcia Boy R, Nothelfer EM, Leotta K, Eisenhut M, Altmann A, Haberkorn U, Mier W. The pharmacokinetics of cell-penetrating peptides. Mol Pharm. 2010 Dec 6;7(6):2224-31.
Sarko D, Eisenhut M, Haberkorn U, Mier W.Bifunctional chelators in the design and application of radiopharmaceuticals for oncological diseases. Curr Med Chem. 2012;19(17):2667-88. Review.
Singh D, Bisland SK, Kawamura K, Gariepy J. Peptide-based intracellular shuttle able to facilitate gene transfer in mammalian cells. Bioconj Chem 1999; 10(5):745-54.
Smith-Jones PM, Vallabhajosula S, Navarro V, Bastidas D, Goldsmith SJ, Bander NH. Radiolabeled monoclonal antibodies specific to the extracellular domain of prostate-specific membrane antigen: preclinical studies in nude mice bearing LNCaP human prostate tumor. J Nucl Med. Apr 2003;44(4):610-617.
Song H, Sgouros G. Radioimmunotherapy of solid tumors: searching for the right target. Curr Drug Deliv. Jan 2011;8(1):26-44.
Tam JP. Synthetic peptide vaccine design: synthesis and properties of a high-density multiple antigenic peptide system. Proc Natl Acad Sci USA. Aug 1988;85(15):5409-5413.
Trabulo S, Cardoso AL, Mano M, De Lima MCP. Cell-Penetrating Peptides-Mechanisms of Cellular Uptake and Generation of Delivery Systems. Pharmaceuticals. 2010;3(4):961-993.
Vives E, Brodin P, Lebleu B. A truncated HIV-1 Tat protein basic domain rapidly translocates through the plasma membrane and accumulates in the cell nucleus. J Biol Chem. Jun 20 1997;272(25):16010-16017.
Waldmann TA. Immunotherapy: past, present and future. Nat Med. Mar 2003;9(3):269-277.
Wangler C, Moldenhauer G, Eisenhut M, Haberkorn U, Mier W. Antibody-dendrimer conjugates: the number, not the size of the dendrimers, determines the immunoreactivity. Bioconjug Chem. Apr 2008;19(4):813-820.
Witzig TE, Gordon LI, Cabanillas F, et al. Randomized controlled trial of yttrium-90-labeled ibritumomab tiuxetan radioimmunotherapy versus rituximab immunotherapy for patients with relapsed or refractory low-grade, follicular, or transformed B-cell non-Hodgkin's lymphoma. J Clin Oncol. May 15 2002;20(10):2453-2463.
Xie J, Schultz PG. A chemical toolkit for proteins--an expanded genetic code. Nat Rev Mol Cell Biol. 2006 Oct;7(10):775-82.

## Claims

1. A conjugate comprising
a protein, and
one or several multivalent cell-penetrating peptide(s) (multivalent CPP(s)) each multivalent CPP comprising at least two cell-penetrating peptides (CPPs),
wherein the multivalent CPP(s) is/are covalently attached to the protein,
wherein each of the multivalent CPP(s) is a dendrimer of cell-penetrating peptides (cell-penetrating peptide dendrimer, dCPP) comprising a dendrimer core and at least two cell-penetrating peptides (CPPs), which are coupled to the dendrimer core,
said dendrimer core being a peptidyl dendrimer core comprising lysines as branching points and cysteine(s) as the anchoring group(s), and comprises or consists of 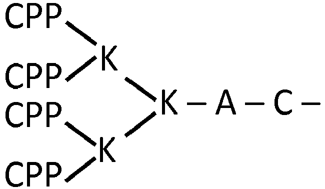 or 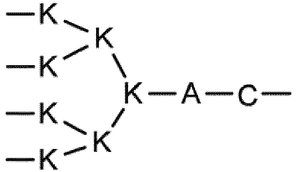 wherein K is Lys, A is Ala and C is Cys,
wherein the cell-penetrating peptide(s) comprise the amino acid sequence of penetratin (SEQ ID NO. 1),
TAT (47-60) (human immunodeficiency virus-derived trans-activator of transcription, SEQ ID NO. 2),
PreS2-TLM (hepatitis B virus-preS2-domain-derived translocation motif, SEQ ID NO. 3),
R9 (SEQ ID NO. 4),
MTS (membrane translocation signal, SEQ ID NO. 5),
SynBl (synthetic porcine protegrin 1-derived CPP, SEQ ID NO. 6),
pVEC (vascular endothelial cadherin-derived CPP, SEQ ID NO. 7),
NLS (nuclear localization signal, SEQ ID NO. 8),
or combinations thereof.

2. The conjugate of claim 1, wherein each of the multivalent CPP(s) comprises 2 to 200 CPPs, preferably 2 to 50, more preferably 2 to 20 or 4 to 20 CPPs.,

3. The conjugate of claim 1 or 2, wherein the dendrimer core comprises spacer between the anchoring group(s) and the branching point(s).

4. The conjugate of any of the preceding claims, wherein the CPP, each comprising the amino acid sequence selected from the group of of SEQ ID Nos. 1 to 8, comprises an amino acid sequence having 5 to 50 amino acids, preferably 5 to 30 amino acids, more preferably 8 to 25 amino acids,
and/or wherein the CPPs, each comprising the amino acid sequence selected from the group of of SEQ ID Nos. 1 to 8, comprise natural amino acids, amino acid derivatives, D-amino acids, modified amino acids, β-amino acid derivatives, α,α-disubstituted amino acid derivatives, N-substituted α-amino acid derivatives, aliphatic or cyclic amines, amino- and carboxy- substituted cycloalkyl derivatives, amino- and carboxy- substituted aromatic derivatives, γ-amino acid derivatives, aliphatic α-amino acid derivatives, diamines and polyamines.

5. The conjugate of any of the preceding claims, wherein a CPP, comprising the amino acid sequence selected from the group of SEQ ID Nos. 1 to 8,
is a (carrier) peptide capable of being internalized into a cell, and/or
comprises in its amino acid sequence at least 25% positively charged amino acid residues, and/or
is internalized into a cell with an efficacy being at least 50% of the internalization efficacy of the TAT peptide having the amino acid sequence of SEQ ID NO. 2.

6. The conjugate of any of the preceding claims, wherein the cell-penetrating peptide(s) comprise the amino acid sequence of penetratin (SEQ ID NO. 1) and/or R9 (SEQ ID NO. 4).

7. The conjugate of any of the preceding claims, wherein the cell-penetrating peptide(s) consist of the amino acid sequence selected from the group of SEQ ID NOs. 1 to 8.

8. The conjugate of any of the preceding claims, wherein the multivalent CPP comprises different CPPs, such as 2, 3 or more different CPPs,
and/or wherein the protein is a biological or clinically active or therapeutic protein, such as an antibody or an antibody fragment, a coagulation factor or cofactor, a receptor or receptor ligand(s), insulin or insulin analogues, interferon(s), enzyme(s), cytokine(s), erythropoietin(s), interleukin(s), hormone(s), a vaccine or combinations thereof,
and/or furthermore comprising linker connecting the protein and the multivalent CPP(s), wherein the linker is preferably a bifunctional (cross)linker covalently coupling the protein with the multivalent cell-penetrating peptide(s).

9. The conjugate of any of the preceding claims, comprising
an antibody,
one or several cell-penetrating peptide dendrimer(s) (dCPP) as defined in claims 1 to 8, one or several linker, preferably bifunctional (cross)linker(s), each covalently coupling the antibody with a dCPP,
and/or further comprising
labels, such as radioisotope(s), fluorescent dye(s), quantum dots, contrast agent(s) drugs or prodrugs,
further biologically active component(s),
or combinations thereof.

10. A method for generating a conjugate according to any of claims 1 to 9, comprising the steps of
(a) providing multivalent cell-penetrating peptide(s) (multivalent CPP(s)) comprising anchoring group(s), wherein each of the multivalent CPP(s) is a dendrimer of cell-penetrating peptides (cell-penetrating peptide dendrimer, dCPP) comprising a dendrimer core and at least two cell-penetrating peptides (CPPs), which are coupled to the dendrimer core, as defined in any of claims 1 to 8,
(b) generating a chemically activated protein, such as a chemically activated antibody, by using a linker, or providing a protein comprising coupling site(s),
(c) coupling the multivalent CPP(s) of step (a) to the protein of step(b),
(d) obtaining the conjugate,
(e) purifying the conjugate,
wherein in step (a) cell-penetrating peptide dendrimer(s) (dCPPs) comprising one or several anchoring group(s) are provided,
and/or wherein the coupling site(s) of the protein are the sides chains of cysteine(s), glutamine(s) and/or lysine(s) and/or unnatural amino acids,
wherein the linker is a bifunctional (cross) linker, such as succinimidyl-4-(N-maleimidomethyl)cyclohexane-1-carboxylate (SMCC),
and/or comprising using in step (b) excess of the linker, preferably to generate a chemically activated protein, such as an antibody, having one or several maleimide molecule(s) on the surface,
and/or comprising using in step (c) excess of the cell-penetrating peptide dendrimer(s).

11. The conjugate of any of claims 1 to 9 for use in medicine.

12. The conjugate of any of claims 1 to 9 for use in the diagnosis, prevention and/or treatment of diseases.

13. The conjugate for use according to claim 12, wherein the diagnosis comprises radioimmunodetection, radioimmunoscintigraphy, radioimmunotomography,
and/or wherein the prevention and/or treatment of a disease comprises immunotherapy and radioimmunotherapy.

14. The conjugate for use according to claim 12 or 13, wherein the disease is cancer, a coagulation disorder, a cardiovascular disease, an immune disease, an infectious disease, a neuronal disease, an inflammatory disease, a heritable disease or a rheumatic disease.

15. The conjugate for use according to any of claims 12 to 14, wherein the diagnosis, prevention and/or treatment comprises cell-specific targeting,
preferably comprising the cell-specific targeting of diseased cells, tissues and organs; cell-specific labelling of diseased cells, tissues and organs; and/or cell-specific drug delivery to diseased cells, tissues and organs, wherein the diseased cells, tissues and organs are preferably tumor related.

16. Use of a conjugate of any of claims 1 to 9 as an *in vitro* diagnostic agent.

## Patentansprüche

1. Konjugat umfassend
ein Protein, und
ein oder mehrere multivalente/s zell-penetrierende/s Peptid/e (multivalente/s CPP(s)) wobei jedes multivalente CPP mindestens zwei zell-penetrierende Peptide (CPPs) umfasst,
wobei das/die multivalente/n CPP(s) kovalent mit dem Protein verbunden sind,
wobei jedes der multivalenten CPP(s) ein Dendrimer von zell-penetrierenden Peptiden (zellpenetrierendes Peptiddendrimer, dCPP) ist, umfassend einen Dendrimerkern und mindestens zwei zell-penetrierende Peptide (CPPs), welche mit dem Dendrimerkern verbunden sind, wobei der Dendrimerkern ein Peptidyl-Dendrimerkern ist, der Lysine als Verzweigungspunkte und Cystein/e als die Verankerungsgruppe/n umfasst, und umfasst oder besteht aus 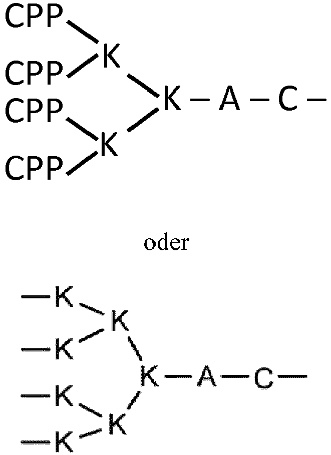 wobei K Lys ist, A Ala ist and C Cys ist,
wobei das/die zell-penetrierende/n Peptid/e die Aminosäuresequenz von
Penetratin (SEQ ID NO. 1),
TAT (47-60) (humaner Immundefizienz-Virus abgeleiteter Transaktivator der Transkription, SEQ ID NO. 2),
PreS2-TLM (Hepatitis B Virus-preS2-Domänen- abgeleitetes Translokationsmotiv, SEQ ID NO. 3),
R9 (SEQ ID NO. 4),
MTS (Membran-Translokationssignal, SEQ ID NO. 5),
SynBl (synthetisches Schweine-Protegrin 1- abgeleitetes CPP, SEQ ID NO. 6),
pVEC (vaskulär endotheliales Cadherin-abgeleitetes CPP, SEQ ID NO. 7),
NLS (Kernlokalisationssignal, SEQ ID NO. 8),
oder Kombinationen davon umfassen.

2. Das Konjugat gemäß Anspruch 1, wobei jedes der multivalenten CPP(s) 2 bis 200 CPPs umfasst, bevorzugt 2 bis 50, weiter bevorzugt 2 bis 20 oder 4 bis 20 CPPs.

3. Das Konjugat gemäß Anspruch 1 oder 2, wobei der Dendrimerkern Spacer zwischen der/den Verankerungsgruppe/n und dem/den Verzweigungspunkte/n umfasst.

4. Das Konjugat gemäß einem der vorhergehenden Ansprüche, wobei das CPP, jedes umfassend die Aminosäuresequenz ausgewählt aus der Gruppe von SEQ ID Nos. 1 bis 8, eine Aminosäuresequenz umfasst, die 5 bis 50 Aminosäuren aufweist, bevorzugt 5 bis 30 Aminosäuren, weiter bevorzugt 8 bis 25 Aminosäuren,
und/oder wobei die CPPs, jedes umfassend die Aminosäuresequenz ausgewählt aus der Gruppe von SEQ ID Nos. 1 bis 8, natürliche Aminosäuren, Aminosäurederivative, D-Aminosäuren, modifizierte Aminosäuren, β- Aminosäurederivate, α,α-disubstituierte Aminosäurederivate, N-substituierte α- Aminosäurederivate, aliphatische oder zyklische Amine, Amino- und Carboxy- substituierte Cycloalkylderivate, Amino- und Carboxysubstituierte aromatische Derivate, γ- Aminosäurederivate, aliphatische α-Aminosäurederivate, Diamine und Polyamine umfassen.

5. Das Konjugat gemäß einem der vorhergehenden Ansprüche, wobei ein CPP, umfassend die Aminosäuresequenz ausgewählt aus der Gruppe von SEQ ID Nos. 1 bis 8,
ein (Carrier-) Peptid ist, das in der Lage ist, in eine Zell internalisiert zu werden, und/oder
in seiner Aminosäuresequenz mindestens 25% positiv geladene Aminosäurereste umfasst, und/oder
in eine Zelle mit einer Effizienz internalisiert wird, die mindestens 50 % der Internalisationseffizienz des TAT-Peptids beträgt, das die Aminosäuresequenz von SEQ ID NO. 2 hat.

6. Das Konjugat gemäß einem der vorhergehenden Ansprüche, wobei das/die zell-penetrierende/n Peptid/e die Aminosäuresequenz von Penetratin (SEQ ID NO. 1) und/oder R9 (SEQ ID NO. 4) umfassen.

7. Das Konjugat gemäß einem der vorhergehenden Ansprüche, wobei das/die zell-penetrierende/n Peptid/e aus der Aminosäuresequenz ausgewählt aus der Gruppe von SEQ ID NOs. 1 bis 8 besteht/ bestehen.

8. Das Konjugat gemäß einem der vorhergehenden Ansprüche, wobei das multivalente CPP verschiedene CPPs umfasst, wie beispielsweise 2, 3 oder mehr verschiedene CPPs,
und/oder wobei das Protein ein biologisch oder klinisch aktives oder therapeutisches Protein ist, wie beispielsweise ein Antikörper oder ein Antikörperfragment, ein Koagulationsfaktor oder Kofaktor, ein Rezeptor oder Rezeptorligand(en), Insulin oder Insulinanaloga, Interferon(e), Enzym(e), Zytokin(e), Erythropoietin(e), Interleukin(e), Hormon(e), ein Vakzin oder Kombinationen davon,
und/oder weiter umfassend Linker, der/die das Protein und das/die multivalent/en CPP(s) verbinden, wobei der Linker bevorzugt ein bi-funktionaler (Cross-)Linker ist, der das Protein kovalent mit dem/den multivalenten zell-penetrierenden Peptid/en verknüpft.

9. Das Konjugat gemäß einem der vorhergehenden Ansprüche, umfassend
einen Antikörper,
ein oder mehrere zell-penetrierende Peptiddendrimer/e (dCPP), wie definiert in den Ansprüchen 1 bis 8,
einen oder mehrere Linker, bevorzugt bi-funktionale (Cross-)Linker, jeder den Antikörper mit einem dCPP kovalent verknüpfend,
und/oder weiter umfassend
Markierungen,
wie beispielsweise Radioisotop/e, Fluoreszenzfarbstoff/e, Quantum dots, Kontrastmittel
Wirkstoffe oder Prodrugs,
weitere biologische aktive Verbindung/en,
oder Kombinationen davon.

10. Ein Verfahren zur Generation eines Konjugats gemäß einem der Ansprüche 1 bis 9, umfassend die Schritte von
(a) zur Verfügung stellen von multivalenten zell-penetrierende/n Peptid/en (multivalent CPP(s)) umfassend Verankerungsgruppe/n, wobei jedes der multivalenten CPP(s) ein Dendrimer von zell-penetrierenden Peptiden (zell-penetrierendes Peptiddendrimer, dCPP) ist, umfassend einen Dendrimerkern und mindestens zwei zell-penetrierende Peptide (CPPs), die mit dem Dendrimerkern verbunden sind, wie definiert in einem der Ansprüche 1 bis 8,
(b) Generieren eines chemisch aktivierten Proteins, wie beispielsweise eines chemisch aktivierten Antikörpers, durch Verwendung eines Linkers, oder zur Verfügung stellen eines Proteins umfassend Kupplungsstelle(n),
(c) Kuppeln des/der multivalenten CPP(s) von Schritt (a) mit dem Protein von Schritt (b),
(d) Erhalten des Konjugats,
(e) Reinigen des Konjugats,
wobei in Schritt (a) zell-penetrierende Peptiddendrimer/e (dCPPs) mit einer oder mehreren Verankerungsgruppen zur Verfügung gestellt werden,
und/oder wobei die Kupplungsstelle(n) des Proteins die Seitenketten von Cystein(en), Glutamin(en) und/oder Lysin(en) und/oder unnatürlichen Aminosäuren sind,
wobei der Linker ein bi-funktionaler (Cross-) Linker ist, wie beispielsweise Succinimidyl-4-(N-Maleimidomethyl)cyclohexane-1 -carboxylate (SMCC),
und/oder umfassend in Schritt (b) das Verwenden von Überschuss des Linkers, bevorzugt um ein chemisch aktiviertes Protein, wie einen Antikörper, zu generieren, das ein oder mehrere Maleimid-Molekül(e) an der Oberfläche hat,
und/oder umfassend in Schritt (c) das Verwenden von Überschuss des/der zell-penetrierenden Peptiddendrimers/ e.

11. Das Konjugat gemäß einem der Ansprüche 1 bis 9 zur Verwendung in der Medizin.

12. Das Konjugat gemäß einem der Ansprüche 1 bis 9 zur Verwendung in der Diagnose, Prävention und/oder Behandlung von Erkrankungen.

13. Das Konjugat zur Verwendung gemäß Anspruch 12, wobei die Diagnose Radioimmundetektion, Radioimmunszintigrafie, Radioimmuntomografie umfasst,
und/oder wobei die Prävention und/oder Behandlung einer Erkrankung Immuntherapie und Radioimmuntherapie umfasst.

14. Das Konjugat zur Verwendung gemäß Anspruch 12 oder 13, wobei die Erkankung Krebs, eine Blutgerinnungserkrankung, eine kardiovaskuläre Erkankung, eine Immunerkrankung, eine Infektionskrankheit, eine neuronale Erkankung, eine Entzündungs-Erkankung, eine erbliche Krankheit oder eine rheumatische Erkankung ist.

15. Das Konjugat zur Verwendung gemäß einem der Ansprüche 12 bis 14, wobei die Diagnose, Prävention und/oder Behandlung zell-spezifisches Targeting umfasst,
bevorzugt umfassend das zell-spezifische Targeting von erkrankten Zellen, Geweben und Organen; zell-spezifisches Markieren von erkrankten Zellen, Geweben und Organen; und/oder zell-spezifische Wirkstoffzufuhr zu erkrankten Zellen, Geweben und Organen, wobei die erkrankten Zellen, Gewebe und Organe bevorzugt tumorbezogen sind.

16. Verwendung eines Konjugats von einem der Ansprüche 1 bis 9 als ein *in vitro* diagnostisches Mittel.

## Revendications

1. Conjugué comprenant
une protéine, et
un ou plusieurs peptide(s) de pénétration cellulaire multivalent (s) (CPP multivalent (s)), chaque CPP multivalent comprenant au moins deux peptides de pénétration cellulaire (CPP), dans lequel le (s) CPP multivalent(s) est/sont fixés de façon covalente à la protéine,
dans lequel chacun du (des) CPP multivalent(s) est un dendrimère de peptides de pénétration cellulaire (dendrimère de peptides de pénétration cellulaire, dCPP) comprenant un noyau dendrimère et au moins deux peptides de pénétration cellulaire (CPP), qui sont couplés au noyau dendrimère,
ledit noyau dendrimère étant un noyau dendrimère de peptidyle comprenant des lysines en tant que points de ramification et une (des) cystéine(s) en tant que groupe(s) d'ancrage, et comprend ou est constitué de 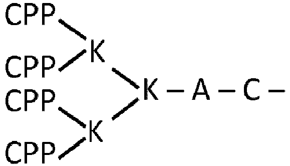 ou 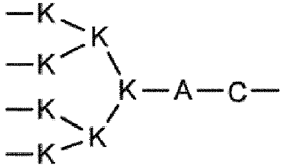
dans lequel K est Lys, A est Ala et C est Cys,
dans lequel le (s) peptide(s) de pénétration cellulaire comprennent la séquence d'acides aminés de la pénétratine (SEQ ID NO : 1),
TAT (47-60) (trans-activateur de transcription dérivé du virus de l'immunodéficience humaine, SEQ ID NO : 2),
PreS2-TLM (motif de translocation dérivé du domaine preS2 du virus de l'hépatite B, SEQ ID NO : 3),
R9 (SEQ ID NO : 4),
MTS (signal de translocation membranaire, SEQ ID NO : 5),
SynB1 (CPP dérivé de la protégrine-1 porcine synthétique, SEQ ID NO : 6),
pVEC (CPP dérivé de la cadhérine endothéliale vasculaire, SEQ ID NO : 7),
NLS (signal de localisation nucléaire, SEQ ID NO : 8),
ou des combinaisons de ceux-ci.

2. Conjugué selon la revendication 1, dans lequel chacun du (des) CPP(s) multivalent(s) comprend de 2 à 200 CPP, de préférence de 2 à 50, de manière davantage préférée de 2 à 20 ou de 4 à 20 CPP.

3. Conjugué selon la revendication 1 ou 2, dans lequel le noyau dendrimère comprend un espaceur entre le (s) groupe(s) d'ancrage et le(s) point(s) de ramification.

4. Conjugué selon l'une quelconque des revendications précédentes, dans lequel le CPP, chacun comprenant la séquence d'acides aminés sélectionnée dans le groupe constitué de SEQ ID NO : 1 à 8, comprend une séquence d'acides aminés ayant de 5 à 50 acides aminés, de préférence de 5 à 30 acides aminés, de manière davantage préférée de 8 à 25 acides aminés,
et/ou dans lequel les CPP, chacun comprenant la séquence d'acides aminés sélectionnée dans le groupe de SEQ ID NO : 1 à 8, comprennent des acides aminés naturels, des dérivés d'acides aminés, des acides D-aminés, des acides aminés modifiés, des dérivés d'acides β-aminés, des dérivés d'acides aminés α,α-disubstitués, des dérivés d'acides α-aminés N-substitués, des amines aliphatiques ou cycliques, des dérivés cycloalkyle à substitution amino et carboxy, des dérivés aromatiques à substitution amino et carboxy, des dérivés d'acides γ-aminés, des dérivés d'acides α-aminés aliphatiques, des diamines et des polyamines.

5. Conjugué selon l'une quelconque des revendications précédentes, dans lequel un CPP, comprenant la séquence d'acides aminés sélectionnée dans le groupe de SEQ ID NO : 1 à 8,
est un peptide (vecteur) capable d'être internalisé dans une cellule, et/ou
comprend dans sa séquence d'acides aminés au moins 25 % de résidus d'acides aminés à charge positive, et/ou
est internalisé dans une cellule avec une efficacité d'au moins 50 % de l'efficacité d'internalisation du peptide TAT ayant la séquence d'acides aminés de SEQ ID NO : 2.

6. Conjugué selon l'une quelconque des revendications précédentes, dans lequel le(s) peptide(s) de pénétration cellulaire comprennent la séquence d'acides aminés de la pénétratine (SEQ ID NO : 1) et/ou R9 (SEQ ID NO : 4).

7. Conjugué selon l'une quelconque des revendications précédentes, dans lequel le(s) peptide(s) de pénétration cellulaire sont constitués de la séquence d'acides aminés sélectionnée dans le groupe de SEQ ID NO : 1 à 8.

8. Conjugué selon l'une quelconque des revendications précédentes, dans lequel le CPP multivalent comprend des CPP différents par exemple 2, 3 ou plus CPP différents,
et/ou dans lequel la protéine est une protéine biologique ou cliniquement active ou thérapeutique, par exemple un anticorps ou un fragment d'anticorps, un facteur de coagulation ou cofacteur, un récepteur ou un (des) ligand(s) de récepteur, l'insuline ou des analogues de l'insuline, un (des) interféron(s), une (des) enzyme(s), une (des) cytokine(s), une (des) érythropoïétine(s), une (des) interleukine(s), une (des) hormone(s), un vaccin ou des combinaisons de ceux-ci,
et/ou comprenant en outre un lieur raccordant la protéine et le (s) CPP multivalent(s) dans lequel le lieur est de préférence un lieur (agent de réticulation) bifonctionnel couplant de façon covalente la protéine au(x) peptide(s) de pénétration cellulaire multivalent (s) .

9. Conjugué selon l'une quelconque des revendications précédentes, comprenant
un anticorps,
un ou plusieurs dendrimère(s) de peptides de pénétration cellulaire (dCPP) selon les revendications 1 à 8, un ou plusieurs lieurs, de préférence un (des) lieur(s) (agent(s) de réticulation) bifonctionnels, chacun couplant de façon covalente l'anticorps à un dCPP,
et/ou comprenant en outre
des marqueurs, tels qu'un (des) radio-isotope(s), colorant(s) fluorescent(s), point(s) quantique(s), agent(s) de contraste,
des médicaments ou promédicaments,
un autre (des autres) composant(s) biologiquement actif(s),
ou des combinaisons de ceux-ci.

10. Procédé de génération d'un conjugué selon l'une quelconque des revendications 1 à 9, comprenant les étapes de
(a) fourniture d'un (de) peptide(s) de pénétration cellulaire multivalent(s) (CPP multivalent(s)) comprenant un (des) groupe(s) d'ancrage, dans lequel chacun du (des) CPP multivalent(s) est un dendrimère de peptides de pénétration cellulaire (dendrimère de peptides de pénétration cellulaire, dCPP) comprenant un noyau dendrimère et au moins deux peptides de pénétration cellulaire (CPP) qui sont couplés au noyau dendrimère, selon l'une quelconque des revendications 1 à 8,
(b) la génération d'une protéine chimiquement active, telle qu'un anticorps chimiquement actif, en utilisant un lieur, ou en fournissant une protéine comprenant un (des) site(s) de couplage,
(c) le couplage du (des) CPP multivalent(s) de l'étape (a) à la protéine de l'étape (b),
(d) l'obtention du conjugué,
(e) la purification du conjugué,
dans lequel à l'étape (a) un (des) dendrimère(s) de peptides de pénétration cellulaire (dCPP) comprenant un ou plusieurs groupe(s) d'ancrage sont fournis,
et/ou dans lequel le (s) site (s) de couplage de la protéine sont les chaînes latérales d'une (de) cystéine(s), glutamine(s) et/ou lysine(s) et /ou d'acides aminés non naturels,
dans lequel le lieur est un lieur (agent de réticulation) bifonctionnel, par exemple le succinimidyl-4-(N-maléimidométhyl)cyclohexane-1-carboxylate (SMCC),
et/ou comprenant l'utilisation à l'étape (b) d'un excès du lieur, de préférence pour générer une protéine chimiquement active, par exemple un anticorps, ayant une ou plusieurs molécule(s) de maléimide sur la surface,
et/ou comprenant l'utilisation à l'étape (c) du (des) dendrimère(s) de peptides de pénétration cellulaire.

11. Conjugué selon l'une quelconque des revendications 1 à 9 pour son utilisation en médecine.

12. Conjugué selon l'une quelconque des revendications 1 à 9 pour son utilisation dans le diagnostic, la prévention et/ou le traitement de maladies.

13. Conjugué pour son utilisation selon la revendication 12, dans lequel le diagnostic comprend la radioimmunodétection, la radioimmunoscintigraphie, la radioimmunotomographie,
et/ou dans lequel la prévention et/ou le traitement d'une maladie comprennent l'immunothérapie et la radioimmunothérapie.

14. Conjugué pour son utilisation selon la revendication 12 ou 13, dans lequel la maladie est un cancer, un trouble de la coagulation, une maladie cardiovasculaire, une maladie immunitaire, une maladie infectieuse, une maladie neuronale, une maladie inflammatoire, une maladie héréditaire ou une maladie rhumatismale.

15. Conjugué pour son utilisation selon l'une quelconque des revendications 12 à 14, dans lequel le diagnostic, la prévention et/ou le traitement comprennent le ciblage spécifique des cellules,
de préférence comprenant le ciblage spécifique des cellules de cellules, tissus et organes malades ; le marquage spécifique des cellules de cellules, tissus et organes malades ; et/ou l'administration de médicament spécifique des cellules à des cellules, tissus et organes malades, dans lequel les cellules, tissus et organes malades sont de préférence liés à la tumeur.

16. Utilisation d'un conjugué selon l'une quelconque des revendications 1 à 9 en tant qu'agent de diagnostic *in vitro.*


### SEQUENCE LISTING

<110> Ruprecht-Karls-Universität Heidelberg
<120> Conjugates of proteins and multivalent cell-penetrating peptides
<130> U30478WO
<150> US 61/733,619
   <151> 2012-12-05
<160> 760
<170> PatentIn version 3.5
<210> 1
   <211> 16
   <212> PRT
   <213> Artificial Sequence
<220>
   <223> Penetratin
<400> 1
   Arg Gln Ile Lys Ile Trp Phe Gln Asn Arg Arg Met Lys Trp Lys Lys
   1               5                   10                  15
<210> 2
   <211> 14
   <212> PRT
   <213> Artificial Sequence
<220>
   <223> CPP
<400> 2
   Tyr Gly Arg Lys Lys Arg Arg Gln Arg Arg Arg Pro Pro Gln
   1               5                   10
<210> 3
   <211> 12
   <212> PRT
   <213> Artificial Sequence
<220>
   <223> CPP
<400> 3
   Pro Leu Ser Ser Ile Phe Ser Arg Ile Gly Asp Pro
   1 5 10
<210> 4
   <211> 9
   <212> PRT
   <213> Artificial Sequence
<220>
   <223> Polyarginine CPP (R9)
<400> 4
   Arg Arg Arg Arg Arg Arg Arg Arg Arg
   1 5
<210> 5
   <211> 16
   <212> PRT
   <213> Artificial Sequence
<220>
   <223> MTS membran translocation signal
<400> 5
   Ala Ala Val Ala Leu Leu Pro Ala Val Leu Leu Ala Leu Leu Ala Pro
   1 5 10 15
<210> 6
   <211> 18
   <212> PRT
   <213> Artificial Sequence
<220>
   <223> synthetic porcine protegrin 1-derived CPP
<400> 6
   Arg Gly Gly Arg Leu Ser Tyr Ser Arg Arg Arg Phe Ser Thr Ser Thr
   1 5 10 15
   Gly Arg
<210> 7
   <211> 18
   <212> PRT
   <213> Artificial Sequence
<220>
   <223> vascular endothelial cadherin-derived CPP
<400> 7
   Leu Leu Ile Ile Leu Arg Arg Arg Ile Arg Lys Gln Ala His Ala His
   1 5 10 15
   Ser Lys
<210> 8
   <211> 7
   <212> PRT
   <213> Artificial Sequence
<220>
   <223> NLS nuclear localization signal
<400> 8
   Pro Lys Lys Lys Arg Lys Val
   1 5
<210> 9
   <211> 9
   <212> PRT
   <213> Artificial Sequence
<220>
   <223> CPP
<400> 9
   Arg Lys Lys Arg Arg Gln Arg Arg Arg
   1 5
<210> 10
   <211> 13
   <212> PRT
   <213> Artificial Sequence
<220>
   <223> CPP
<400> 10
   Gly Arg Lys Lys Arg Arg Gln Arg Arg Arg Pro Pro Gln
   1 5 10
<210> 11
   <211> 24
   <212> PRT
   <213> Artificial Sequence
<220>
   <223> calcitonin-derived CPP
<400> 11
   Leu Gly Thr Tyr Thr Gln Asp Phe Asn Lys Phe His Thr Phe Pro Gln
   1               5                   10                  15
   Thr Ala Ile Gly Val Gly Ala Pro
               20
<210> 12
   <211> 10
   <212> PRT
   <213> Artificial Sequence
<220>
   <223> NLS
<400> 12
   Val Gln Arg Lys Arg Gln Lys Leu Met Pro
   1               5                   10
<210> 13
   <211> 8
   <212> PRT
   <213> Artificial Sequence
<220>
   <223> NLS
<400> 13
   Ser Lys Lys Lys Lys Thr Lys Val
   1               5
<210> 14
   <211> 8
   <212> PRT
   <213> Artificial Sequence
<220>
   <223> NLS
<400> 14
   Gly Arg Lys Arg Lys Lys Arg Thr
   1               5
<210> 15
   <211> 9
   <212> PRT
   <213> Artificial Sequence
<220>
   <223> polybasic CPP
<400> 15
   Arg Arg Arg Glu Arg Arg Ala Glu Lys
   1               5
<210> 16
   <211> 9
   <212> PRT
   <213> Artificial Sequence
<220>
   <223> polybasic CPP
<400> 16
   Lys Cys Pro Ser Arg Arg Pro Lys Arg
   1               5
<210> 17
   <211> 6
   <212> PRT
   <213> Artificial Sequence
<220>
   <223> N-terminal repetitive domain of maize gamma-zein
<400> 17
   Val Arg Leu Pro Pro Pro
   1               5
<210> 18
   <211> 6
   <212> PRT
   <213> Artificial Sequence
<220>
   <223> N-terminal repetitive domain of maize gamma-zein
<400> 18
   Val His Leu Pro Pro Pro
   1               5
<210> 19
   <211> 6
   <212> PRT
   <213> Artificial Sequence
<220>
   <223> N-terminal repetitive domain of maize gamma-zein
<400> 19
   Val Lys Leu Pro Pro Pro
   1               5
<210> 20
   <211> 17
   <212> PRT
   <213> Artificial Sequence
<220>
   <223> Peptide from gp41 fusion sequence
<400> 20
   Ala Val Gly Ala Ile Gly Ala Leu Phe Leu Gly Phe Leu Gly Ala Ala
   1               5                   10                  15
   Gly
<210> 21
   <211> 15
   <212> PRT
   <213> Artificial Sequence
<220>
   <223> SSHR
<400> 21
   Val Thr Val Leu Ala Leu Gly Ala Leu Ala Gly Val Gly Val Gly
   1               5                   10                  15
<210> 22
   <211> 18
   <212> PRT
   <213> Artificial Sequence
<220>
   <223> pVEC
<400> 22
   Ile Ala Ala Arg Ile Lys Leu Arg Ser Arg Gln His Ile Lys Leu Arg
   1               5                   10                  15
   His Leu
<210> 23
   <211> 27
   <212> PRT
   <213> Artificial Sequence
<220>
   <223> Vpr
<400> 23
   Asp Thr Trp Pro Gly Val Glu Ala Leu Ile Arg Ile Leu Gln Gln Leu
   1               5                   10                  15
   Leu Phe Ile His Phe Arg Ile Gly Cys Gln His
               20                  25
<210> 24
   <211> 34
   <212> PRT
   <213> Artificial Sequence
<220>
   <223> VP22
<400> 24
   Asp Ala Ala Thr Ala Thr Arg Gly Arg Ser Ala Ala Ser Arg Pro Thr
   1               5                   10                  15
   Glu Arg Pro Arg Ala Pro Ala Arg Ser Ala Ser Arg Pro Arg Arg Pro
               20                  25                  30
   Val Asp
<210> 25
   <211> 17
   <212> PRT
   <213> Artificial Sequence
<220>
   <223> gp41 fusion sequence
<400> 25
   Gly Ala Leu Phe Leu Gly Trp Leu Gly Ala Ala Gly Ser Thr Met Gly
   1               5                   10                  15
   Ala
<210> 26
   <211> 17
   <212> PRT
   <213> Artificial Sequence
<220>
   <223> Caiman crocodylus Ig(v) light chain
<400> 26
   Met Gly Leu Gly Leu His Leu Leu Val Leu Ala Ala Ala Leu Gln Gly
   1               5                   10                  15
   Ala
<210> 27
   <211> 27
   <212> PRT
   <213> Artificial Sequence
<220>
   <223> CPP from pestivirus env glycoprotein
<400> 27
   Arg Gln Gly Ala Ala Arg Val Thr Ser Trp Leu Gly Arg Gln Leu Arg
   1               5                   10                  15
   Ile Ala Gly Lys Arg Leu Glu Gly Arg Ser Lys
               20                  25
<210> 28
   <211> 28
   <212> PRT
   <213> Artificial Sequence
<220>
   <223> CPP from the prion protein
<400> 28
   Met Ala Asn Leu Gly Tyr Trp Leu Leu Ala Leu Phe Val Thr Met Trp
   1               5                   10                  15
   Thr Asp Val Gly Leu Cys Lys Lys Arg Pro Lys Pro
               20                  25
<210> 29
   <211> 16
   <212> PRT
   <213> Artificial Sequence
<220>
   <223> Yeast PRP6 (129-144)
<400> 29
   Thr Arg Arg Asn Lys Arg Asn Arg Ile Gln Glu Gln Leu Asn Arg Lys
   1               5                   10                  15
<210> 30
   <211> 18
   <212> PRT
   <213> Artificial Sequence
<220>
   <223> Phi21 N (12-29)
<400> 30
   Thr Ala Lys Thr Arg Tyr Lys Ala Arg Arg Ala Glu Leu Ile Ala Glu
   1               5                   10                  15
   Arg Arg
<210> 31
   <211> 22
   <212> PRT
   <213> Artificial Sequence
<220>
   <223> Delta N (1-22)
<400> 31
   Met Asp Ala Gln Thr Arg Arg Arg Glu Arg Arg Ala Glu Lys Gln Ala
   1               5                   10                  15
   Gln Trp Lys Ala Ala Asn
               20
<210> 32
   <211> 15
   <212> PRT
   <213> Artificial Sequence
<220>
   <223> FHV coat (35-49)
<400> 32
   Arg Arg Arg Arg Asn Arg Thr Arg Arg Asn Arg Arg Arg Val Arg
   1               5                   10                  15
<210> 33
   <211> 19
   <212> PRT
   <213> Artificial Sequence
<220>
   <223> BMV Gag (7-25)
<400> 33
   Lys Met Thr Arg Ala Gln Arg Arg Ala Ala Ala Arg Arg Asn Arg Trp
   1               5                   10                  15
   Thr Ala Arg
<210> 34
   <211> 13
   <212> PRT
   <213> Artificial Sequence
<220>
   <223> HTLV-II Rex (4-16)
<400> 34
   Thr Arg Arg Gln Arg Thr Arg Arg Ala Arg Arg Asn Arg
   1               5                   10
<210> 35
   <211> 12
   <212> PRT
   <213> Artificial Sequence
<220>
   <223> HIV-1 Rev (9-20)
<400> 35
   Arg Gln Ala Arg Arg Asn Arg Arg Arg Arg Trp Arg
   1               5                   10
<210> 36
   <211> 22
   <212> PRT
   <213> Artificial Sequence
<220>
   <223> RSG-1.2
<400> 36
   Asp Arg Arg Arg Arg Gly Ser Arg Pro Ser Gly Ala Glu Arg Arg Arg
   1               5                   10                  15
   Arg Arg Ala Ala Ala Ala
               20
<210> 37
   <211> 15
   <212> PRT
   <213> Artificial Sequence
<220>
   <223> Lambda-N (48-62)
<400> 37
   Gln Thr Arg Arg Arg Glu Arg Arg Ala Glu Lys Gln Ala Gln Trp
   1               5                   10                  15
<210> 38
   <211> 16
   <212> PRT
   <213> Artificial Sequence
<220>
   <223> Bipartite, Nucleoplasmin (155-170), NLS
<400> 38
   Lys Arg Pro Ala Ala Ile Lys Lys Ala Gly Gln Ala Lys Lys Lys Lys
   1               5                   10                  15
<210> 39
   <211> 12
   <212> PRT
   <213> Artificial Sequence
<220>
   <223> Herpesvirus, 8 k8 protein (124-135)
<400> 39
   Thr Arg Arg Ser Lys Arg Arg Ser His Arg Lys Phe
   1               5                   10
<210> 40
   <211> 17
   <212> PRT
   <213> Artificial Sequence
<220>
   <223> Buforin-II (20-36)
<400> 40
   Arg Ala Gly Leu Gln Phe Pro Val Gly Arg Val His Arg Leu Leu Arg
   1               5                   10                  15
   Lys
<210> 41
   <211> 21
   <212> PRT
   <213> Artificial Sequence
<220>
   <223> Magainin
<400> 41
   Thr Arg Ser Ser Arg Ala Gly Leu Gln Phe Pro Val Gly Arg Val His
   1               5                   10                  15
   Arg Leu Leu Arg Lys
               20
<210> 42
   <211> 16
   <212> PRT
   <213> Artificial Sequence
<220>
   <223> PDX-1-PTD
<400> 42
   Arg His Ile Lys Ile Trp Phe Gln Asn Arg Arg Met Lys Trp Lys Lys
   1               5                   10                  15
<210> 43
   <211> 42
   <212> PRT
   <213> Artificial Sequence
<220>
   <223> crotamine
<400> 43
   Tyr Lys Gln Cys His Lys Lys Gly Gly His Cys Phe Pro Lys Glu Lys
   1               5                   10                  15
   Ile Cys Leu Pro Pro Ser Ser Asp Phe Gly Lys Met Asp Cys Arg Trp
               20                  25                  30
   Arg Trp Lys Cys Cys Lys Lys Gly Ser Gly
           35                  40
<210> 44
   <211> 16
   <212> PRT
   <213> Artificial Sequence
<220>
   <223> pIsI
<400> 44
   Arg Val Ile Arg Val Trp Phe Gln Asn Lys Arg Cys Lys Asp Lys Lys
   1               5                   10                  15
<210> 45
   <211> 60
   <212> PRT
   <213> Artificial Sequence
<220>
   <223> Fushi-tarazu (254-313)
<400> 45
   Ser Lys Arg Thr Arg Gln Thr Tyr Thr Arg Tyr Gln Thr Leu Glu Leu
   1               5                   10                  15
   Glu Lys Glu Phe His Phe Asn Arg Tyr Ile Thr Arg Arg Arg Arg Ile
               20                  25                  30
   Asp Ile Ala Asn Ala Leu Ser Leu Ser Glu Arg Gln Ile Lys Ile Trp
           35                  40                  45
   Phe Gln Asn Arg Arg Met Lys Ser Lys Lys Asp Arg
       50                  55                  60
<210> 46
   <211> 60
   <212> PRT
   <213> Artificial Sequence
<220>
   <223> Engrailed (454-513)
<400> 46
   Glu Lys Arg Pro Arg Thr Ala Phe Ser Ser Glu Gln Leu Ala Arg Leu
   1               5                   10                  15
   Lys Arg Glu Phe Asn Glu Asn Arg Tyr Leu Thr Glu Arg Arg Arg Gln
               20                  25                  30
   Gln Leu Ser Ser Glu Leu Gly Leu Asn Glu Ala Gln Ile Lys Ile Trp
           35                  40                  45
   Phe Gln Asn Lys Arg Ala Lys Ile Lys Lys Ser Thr
       50                  55                  60
<210> 47
   <211> 27
   <212> PRT
   <213> Artificial Sequence
<220>
   <223> transportan
<400> 47
   Gly Trp Thr Leu Asn Ser Ala Gly Tyr Leu Leu Gly Lys Ile Asn Leu
   1               5                   10                  15
   Lys Ala Leu Ala Ala Leu Ala Lys Lys Ile Leu
               20                  25
<210> 48
   <211> 8
   <212> PRT
   <213> Artificial Sequence
<220>
   <223> Polyarginine CPP (R8)
<400> 48
   Arg Arg Arg Arg Arg Arg Arg Arg
   1               5
<210> 49
   <211> 9
   <212> PRT
   <213> Artificial Sequence
<220>
   <223> Poly-D-arginine
<400> 49
   Arg Arg Arg Arg Arg Arg Arg Arg Arg
   1               5
<210> 50
   <211> 18
   <212> PRT
   <213> Artificial Sequence
<220>
   <223> KLAL peptide/model amphiphatic peptide (MAP)
<400> 50
   Lys Leu Ala Leu Lys Leu Ala Leu Lys Ala Leu Lys Ala Ala Leu Lys
   1               5                   10                  15
   Leu Ala
<210> 51
   <211> 30
   <212> PRT
   <213> Artificial Sequence
<220>
   <223> KALA model amphiphatic peptide
<400> 51
   Trp Glu Ala Lys Leu Ala Lys Ala Leu Ala Lys Ala Leu Ala Lys His
   1               5                   10                  15
   Leu Ala Lys Ala Leu Ala Lys Ala Leu Lys Ala Cys Glu Ala
               20                  25                  30
<210> 52
   <211> 11
   <212> PRT
   <213> Artificial Sequence
<220>
   <223> modeled TAT peptide
<400> 52
   Tyr Ala Arg Ala Ala Ala Arg Gln Ala Arg Ala
   1               5                   10
<210> 53
   <211> 27
   <212> PRT
   <213> Artificial Sequence
<220>
   <223> b-sheet-forming peptide
<400> 53
   Asp Pro Lys Gly Asp Pro Pro Lys Gly Val Thr Val Thr Val Thr Val
   1               5                   10                  15
   Thr Val Thr Gly Lys Gly Asp Pro Lys Pro Asp
               20                  25
<210> 54
   <211> 16
   <212> PRT
   <213> Artificial Sequence
<220>
   <223> retro-inverso forms of established CPPs
<400> 54
   Lys Lys Trp Lys Met Arg Arg Asn Gln Phe Trp Val Arg Val Gln Arg
   1               5                   10                  15
<210> 55
   <211> 17
   <212> PRT
   <213> Artificial Sequence
<220>
   <223> W/R penetratin
<400> 55
   Arg Arg Trp Arg Arg Trp Trp Trp Arg Arg Trp Trp Arg Arg Trp Arg
   1               5                   10                  15
   Arg
<210> 56
   <211> 28
   <212> PRT
   <213> Artificial Sequence
<220>
   <223> MPG
<400> 56
   Gly Ala Leu Phe Leu Gly Phe Leu Gly Ala Ala Gly Ser Thr Met Gly
   1               5                   10                  15
   Ala Trp Ser Gln Pro Lys Ser Lys Arg Lys Val Cys
               20                  25
<210> 57
   <211> 21
   <212> PRT
   <213> Artificial Sequence
<220>
   <223> Pep-1
<400> 57
   Lys Glu Thr Trp Trp Glu Thr Trp Trp Thr Glu Trp Ser Gln Pro Lys
   1               5                   10                  15
   Lys Lys Arg Lys Val
               20
<210> 58
   <211> 27
   <212> PRT
   <213> Artificial Sequence
<220>
   <223> Signal-sequence peptides (I)
<400> 58
   Gly Ala Leu Phe Leu Gly Trp Leu Gly Ala Ala Gly Ser Thr Met Gly
   1               5                   10                  15
   Ala Trp Ser Gln Pro Lys Lys Lys Arg Lys Val
               20                  25
<210> 59
   <211> 12
   <212> PRT
   <213> Artificial Sequence
<220>
   <223> PTD-5
<400> 59
   Arg Arg Gln Arg Arg Thr Ser Lys Leu Met Lys Arg
   1               5                   10
<210> 60
   <211> 10
   <212> PRT
   <213> Artificial Sequence
<220>
   <223> RSV-A9
<400> 60
   Arg Arg Ile Pro Asn Arg Arg Pro Arg Arg
   1               5                   10
<210> 61
   <211> 11
   <212> PRT
   <213> Artificial Sequence
<220>
   <223> CTP-512
<400> 61
   Tyr Gly Arg Arg Ala Arg Arg Arg Arg Arg Arg
   1               5                   10
<210> 62
   <211> 12
   <212> PRT
   <213> Artificial Sequence
<220>
   <223> U2AF
<400> 62
   Ser Gln Met Thr Arg Gln Ala Arg Arg Leu Tyr Val
   1               5                   10
<210> 63
   <211> 16
   <212> PRT
   <213> Artificial Sequence
<220>
   <223> 105Y
<400> 63
   Ser Ile Pro Pro Glu Val Lys Phe Asn Lys Pro Phe Val Tyr Leu Ile
   1               5                   10                  15
<210> 64
   <211> 17
   <212> PRT
   <213> Artificial Sequence
<220>
   <223> Antennapedia Leader Peptide
<400> 64
   Lys Lys Trp Lys Met Arg Arg Asn Gln Phe Trp Val Lys Val Gln Arg
   1               5                   10                  15
   Gly
<210> 65
   <211> 43
   <212> PRT
   <213> Artificial Sequence
<220>
   <223> Anti-BetaGamma
<400> 65
   Ala Ala Val Ala Leu Leu Pro Ala Val Leu Leu Ala Leu Leu Ala Val
   1               5                   10                  15
   Thr Asp Gln Leu Gly Glu Asp Phe Phe Ala Val Asp Leu Glu Ala Phe
               20                  25                  30
   Leu Gln Glu Phe Gly Leu Leu Pro Glu Lys Glu
           35                  40
<210> 66
   <211> 15
   <212> PRT
   <213> Artificial Sequence
<220>
   <223> Lipid Membrane Translocating
<400> 66
   Lys Lys Ala Ala Ala Val Leu Leu Pro Val Leu Leu Ala Ala Pro
   1               5                   10                  15
<210> 67
   <211> 14
   <212> PRT
   <213> Artificial Sequence
<220>
   <223> Mastoparan
<400> 67
   Ile Asn Leu Lys Ala Leu Ala Ala Leu Ala Lys Lys Ile Leu
   1               5                   10
<210> 68
   <211> 13
   <212> PRT
   <213> Artificial Sequence
<220>
   <223> MEK1 Nterm
<400> 68
   Met Pro Lys Lys Lys Pro Thr Pro Ile Gln Leu Asn Pro
   1               5                   10
<210> 69
   <211> 16
   <212> PRT
   <213> Artificial Sequence
<220>
   <223> MPS
<400> 69
   Ala Ala Val Ala Leu Leu Pro Ala Val Leu Leu Ala Leu Leu Ala Lys
   1               5                   10                  15
<210> 70
   <211> 29
   <212> PRT
   <213> Artificial Sequence
<220>
   <223> RV-MAT
<400> 70
   Met Asn Leu Leu Arg Lys Ile Val Lys Asn Arg Arg Asp Glu Asp Thr
   1               5                   10                  15
   Gln Lys Ser Ser Pro Ala Ser Ala Pro Leu Asp Asp Gly
               20                  25
<210> 71
   <211> 11
   <212> PRT
   <213> Artificial Sequence
<220>
   <223> Transdermal peptide
<400> 71
   Ala Cys Ser Ser Ser Pro Ser Lys His Cys Gly
   1               5                   10
<210> 72
   <211> 10
   <212> PRT
   <213> Artificial Sequence
<220>
   <223> SynB3
<400> 72
   Arg Arg Leu Ser Tyr Ser Arg Arg Arg Phe
   1               5                   10
<210> 73
   <211> 12
   <212> PRT
   <213> Artificial Sequence
<220>
   <223> PTD-4
<400> 73
   Pro Ile Arg Arg Arg Lys Lys Leu Arg Arg Leu Lys
   1 5 10
<210> 74
   <211> 27
   <212> PRT
   <213> Artificial Sequence
<220>
   <223> SBP
<400> 74
   Met Gly Leu Gly Leu His Leu Leu Val Leu Ala Ala Ala Leu Gln Gly
   1 5 10 15
   Ala Trp Ser Gln Pro Lys Lys Lys Arg Lys Val
   20 25
<210> 75
   <211> 21
   <212> PRT
   <213> Artificial Sequence
<220>
   <223> Pep-2
<400> 75
   Lys Glu Thr Trp Phe Glu Thr Trp Phe Thr Glu Trp Ser Gln Pro Lys
   1 5 10 15
   Lys Lys Arg Lys Val
   20
<210> 76
   <211> 4
   <212> PRT
   <213> Artificial Sequence
<220>
   <223> Polyarginines (R)n wherein n is at least 3, preferably 3 to 50, more preferably 3 to 15
<400> 76
   Arg Arg Arg Arg
   1
<210> 77
   <211> 4
   <212> PRT
   <213> Artificial Sequence
<220>
   <223> Polylysines (K)n wherein n is at least 3, preferably 3 to 50, more preferably 3 to 15
<400> 77
   Lys Lys Lys Lys
   1
<210> 78
   <211> 18
   <212> PRT
   <213> Artificial Sequence
<220>
   <223> CPP
<400> 78
   Lys Ile Ala Ala Lys Ser Ile Ala Lys Ile Trp Lys Ser Ile Leu Lys
   1               5                   10                  15
   Ile Ala
<210> 79
   <211> 18
   <212> PRT
   <213> Artificial Sequence
<220>
   <223> CPP
<400> 79
   Lys Ala Leu Ala Lys Ala Leu Ala Lys Leu Trp Lys Ala Leu Ala Lys
   1               5                   10                  15
   Ala Ala
<210> 80
   <211> 18
   <212> PRT
   <213> Artificial Sequence
<220>
   <223> CPP
<400> 80
   Lys Leu Ala Leu Lys Leu Ala Leu Lys Trp Ala Lys Leu Ala Leu Lys
   1               5                   10                  15
   Ala Ala
<210> 81
   <211> 18
   <212> PRT
   <213> Artificial Sequence
<220>
   <223> CPP
<400> 81
   Lys Leu Leu Ala Lys Ala Ala Lys Lys Trp Leu Leu Leu Ala Leu Lys
   1 5 10 15
   Ala Ala
<210> 82
   <211> 18
   <212> PRT
   <213> Artificial Sequence
<220>
   <223> CPP
<400> 82
   Lys Leu Leu Ala Lys Ala Ala Leu Lys Trp Leu Leu Lys Ala Leu Lys
   1 5 10 15
   Ala Ala
<210> 83
   <211> 18
   <212> PRT
   <213> Artificial Sequence
<220>
   <223> CPP
<400> 83
   Lys Ala Leu Lys Lys Leu Leu Ala Lys Trp Leu Ala Ala Ala Lys Ala
   1 5 10 15
   Leu Leu
<210> 84
   <211> 18
   <212> PRT
   <213> Artificial Sequence
<220>
   <223> CPP
<400> 84
   Lys Leu Ala Ala Ala Leu Leu Lys Lys Trp Lys Lys Leu Ala Ala Ala
   1               5                   10                  15
   Leu Leu
<210> 85
   <211> 18
   <212> PRT
   <213> Artificial Sequence
<220>
   <223> CPP
<400> 85
   Lys Ala Leu Ala Ala Leu Leu Lys Lys Trp Ala Lys Leu Leu Ala Ala
   1               5                   10                  15
   Leu Lys
<210> 86
   <211> 18
   <212> PRT
   <213> Artificial Sequence
<220>
   <223> CPP
<400> 86
   Lys Ala Leu Ala Ala Leu Leu Lys Lys Leu Ala Lys Leu Leu Ala Ala
   1               5                   10                  15
   Leu Lys
<210> 87
   <211> 16
   <212> PRT
   <213> Artificial Sequence
<220>
   <223> CPP
<400> 87
   Lys Leu Ala Leu Lys Leu Ala Leu Lys Ala Leu Lys Ala Ala Leu Lys
   1               5                   10                  15
<210> 88
   <211> 14
   <212> PRT
   <213> Artificial Sequence
<220>
   <223> CPP
<400> 88
   Lys Leu Ala Leu Lys Ala Leu Lys Ala Ala Leu Lys Leu Ala
   1               5                   10
<210> 89
   <211> 14
   <212> PRT
   <213> Artificial Sequence
<220>
   <223> CPP
<400> 89
   Lys Leu Ala Leu Lys Leu Ala Leu Lys Ala Leu Lys Ala Ala
   1               5                   10
<210> 90
   <211> 18
   <212> PRT
   <213> Artificial Sequence
<220>
   <223> CPP
<400> 90
   Lys Leu Gly Leu Lys Leu Gly Leu Lys Gly Leu Lys Gly Gly Leu Lys
   1               5                   10                  15
   Leu Gly
<210> 91
   <211> 18
   <212> PRT
   <213> Artificial Sequence
<220>
   <223> CPP
<400> 91
   Lys Leu Ala Leu Lys Leu Ala Leu Lys Ala Leu Gln Ala Ala Leu Gln
   1               5                   10                  15
   Leu Ala
<210> 92
   <211> 18
   <212> PRT
   <213> Artificial Sequence
<220>
   <223> CPP
<400> 92
   Lys Leu Ala Leu Gln Leu Ala Leu Gln Ala Leu Gln Ala Ala Leu Gln
   1               5                   10                  15
   Leu Ala
<210> 93
   <211> 18
   <212> PRT
   <213> Artificial Sequence
<220>
   <223> CPP
<400> 93
   Gln Leu Ala Leu Gln Leu Ala Leu Gln Ala Leu Gln Ala Ala Leu Gln
   1               5                   10                  15
   Leu Ala
<210> 94
   <211> 18
   <212> PRT
   <213> Artificial Sequence
<220>
   <223> CPP
<400> 94
   Glu Leu Ala Leu Glu Leu Ala Leu Glu Ala Leu Glu Ala Ala Leu Glu
   1               5                   10                  15
   Leu Ala
<210> 95
   <211> 18
   <212> PRT
   <213> Artificial Sequence
<220>
   <223> CPP
<400> 95
   Leu Lys Thr Leu Ala Thr Ala Leu Thr Lys Leu Ala Lys Thr Leu Thr
   1               5                   10                  15
   Thr Leu
<210> 96
   <211> 18
   <212> PRT
   <213> Artificial Sequence
<220>
   <223> CPP
<400> 96
   Leu Leu Lys Thr Thr Ala Leu Leu Lys Thr Thr Ala Leu Leu Lys Thr
   1               5                   10                  15
   Thr Ala
<210> 97
   <211> 18
   <212> PRT
   <213> Artificial Sequence
<220>
   <223> CPP
<400> 97
   Leu Lys Thr Leu Thr Glu Thr Leu Lys Glu Leu Thr Lys Thr Leu Thr
   1               5                   10                  15
   Glu Leu
<210> 98
   <211> 18
   <212> PRT
   <213> Artificial Sequence
<220>
   <223> CPP
<400> 98
   Leu Leu Lys Thr Thr Glu Leu Leu Lys Thr Thr Glu Leu Leu Lys Thr
   1               5                   10                  15
   Thr Glu
<210> 99
   <211> 18
   <212> PRT
   <213> Artificial Sequence
<220>
   <223> CPP with all D-amino acids
<400> 99
   Lys Leu Ala Leu Lys Leu Ala Leu Lys Ala Leu Lys Ala Ala Leu Lys
   1               5                   10                  15
   Leu Ala
<210> 100
   <211> 18
   <212> PRT
   <213> Artificial Sequence
<220>
   <223> CPP
<400> 100
   Lys Ala Leu Lys Leu Lys Leu Ala Leu Ala Leu Leu Ala Lys Leu Lys
   1               5                   10                  15
   Leu Ala
<210> 101
   <211> 16
   <212> PRT
   <213> Artificial Sequence
<220>
   <223> CPP
<400> 101
   Lys Lys Trp Lys Met Arg Arg Asn Gln Phe Trp Ile Lys Ile Gln Arg
   1               5                   10                  15
<210> 102
   <211> 16
   <212> PRT
   <213> Artificial Sequence
<220>
   <223> CPP with all D-amino acids
<400> 102
   Arg Gln Ile Lys Ile Trp Phe Gln Asn Arg Arg Met Lys Trp Lys Lys
   1               5                   10                  15
<210> 103
   <211> 16
   <212> PRT
   <213> Artificial Sequence
<220>
   <223> CPP
<400> 103
   Arg Gln Ile Lys Ile Trp Phe Pro Asn Arg Arg Met Lys Trp Lys Lys
   1 5 10 15
<210> 104
   <211> 16
   <212> PRT
   <213> Artificial Sequence
<220>
   <223> CPP
<400> 104
   Arg Gln Pro Lys Ile Trp Phe Pro Asn Arg Arg Lys Pro Trp Lys Lys
   1 5 10 15
<210> 105
   <211> 15
   <212> PRT
   <213> Artificial Sequence
<220>
   <223> CPP
<400> 105
   Arg Gln Ile Lys Ile Trp Phe Gln Asn Arg Arg Met Lys Trp Lys
   1 5 10 15
<210> 106
   <211> 14
   <212> PRT
   <213> Artificial Sequence
<220>
   <223> CPP
<400> 106
   Arg Gln Ile Lys Ile Trp Phe Gln Asn Arg Arg Met Lys Trp
   1 5 10
<210> 107
   <211> 13
   <212> PRT
   <213> Artificial Sequence
<220>
   <223> CPP
<400> 107
   Arg Gln Ile Lys Ile Trp Phe Gln Asn Arg Arg Met Lys
   1 5 10
<210> 108
   <211> 12
   <212> PRT
   <213> Artificial Sequence
<220>
   <223> CPP
<400> 108
   Arg Gln Ile Lys Ile Trp Phe Gln Asn Arg Arg Met
   1 5 10
<210> 109
   <211> 11
   <212> PRT
   <213> Artificial Sequence
<220>
   <223> CPP
<400> 109
   Arg Gln Ile Lys Ile Trp Phe Gln Asn Arg Arg
   1 5 10
<210> 110
   <211> 10
   <212> PRT
   <213> Artificial Sequence
<220>
   <223> CPP
<400> 110
   Arg Gln Ile Lys Ile Trp Phe Gln Asn Arg
   1 5 10
<210> 111
   <211> 9
   <212> PRT
   <213> Artificial Sequence
<220>
   <223> CPP
<400> 111
   Arg Gln Ile Lys Ile Trp Phe Gln Asn
   1 5
<210> 112
   <211> 8
   <212> PRT
   <213> Artificial Sequence
<220>
   <223> CPP
<400> 112
   Arg Gln Ile Lys Ile Trp Phe Gln
   1 5
<210> 113
   <211> 6
   <212> PRT
   <213> Artificial Sequence
<220>
   <223> CPP
<400> 113
   Arg Gln Ile Lys Ile Trp
   1 5
<210> 114
   <211> 15
   <212> PRT
   <213> Artificial Sequence
<220>
   <223> CPP
<400> 114
   Gln Ile Lys Ile Trp Phe Gln Asn Arg Arg Met Lys Trp Lys Lys
   1 5 10 15
<210> 115
   <211> 14
   <212> PRT
   <213> Artificial Sequence
<220>
   <223> CPP
<400> 115
   Ile Lys Ile Trp Phe Gln Asn Arg Arg Met Lys Trp Lys Lys
   1 5 10
<210> 116
   <211> 13
   <212> PRT
   <213> Artificial Sequence
<220>
   <223> CPP
<400> 116
   Lys Ile Trp Phe Gln Asn Arg Arg Met Lys Trp Lys Lys
   1 5 10
<210> 117
   <211> 12
   <212> PRT
   <213> Artificial Sequence
<220>
   <223> CPP
<400> 117
   Ile Trp Phe Gln Asn Arg Arg Met Lys Trp Lys Lys
   1 5 10
<210> 118
   <211> 11
   <212> PRT
   <213> Artificial Sequence
<220>
   <223> CPP
<400> 118
   Trp Phe Gln Asn Arg Arg Met Lys Trp Lys Lys
   1 5 10
<210> 119
   <211> 10
   <212> PRT
   <213> Artificial Sequence
<220>
   <223> CPP
<400> 119
   Phe Gln Asn Arg Arg Met Lys Trp Lys Lys
   1 5 10
<210> 120
   <211> 9
   <212> PRT
   <213> Artificial Sequence
<220>
   <223> CPP
<400> 120
   Gln Asn Arg Arg Met Lys Trp Lys Lys
   1 5
<210> 121
   <211> 8
   <212> PRT
   <213> Artificial Sequence
<220>
   <223> CPP
<400> 121
   Asn Arg Arg Met Lys Trp Lys Lys
   1 5
<210> 122
   <211> 7
   <212> PRT
   <213> Artificial Sequence
<220>
   <223> CPP
<400> 122
   Arg Arg Met Lys Trp Lys Lys
   1 5
<210> 123
   <211> 6
   <212> PRT
   <213> Artificial Sequence
<220>
   <223> CPP
<400> 123
   Arg Met Lys Trp Lys Lys
   1 5
<210> 124
   <211> 16
   <212> PRT
   <213> Artificial Sequence
<220>
   <223> CPP
<400> 124
   Ala Gln Ile Lys Ile Trp Phe Gln Asn Arg Arg Met Lys Trp Lys Lys
   1 5 10 15
<210> 125
   <211> 16
   <212> PRT
   <213> Artificial Sequence
<220>
   <223> CPP
<400> 125
   Arg Ala Ile Lys Ile Trp Phe Gln Asn Arg Arg Met Lys Trp Lys Lys
   1 5 10 15
<210> 126
   <211> 16
   <212> PRT
   <213> Artificial Sequence
<220>
   <223> CPP
<400> 126
   Arg Gln Ala Lys Ile Trp Phe Gln Asn Arg Arg Met Lys Trp Lys Lys
   1               5                   10                  15
<210> 127
   <211> 16
   <212> PRT
   <213> Artificial Sequence
<220>
   <223> CPP
<400> 127
   Arg Gln Ile Ala Ile Trp Phe Gln Asn Arg Arg Met Lys Trp Lys Lys
   1               5                   10                  15
<210> 128
   <211> 16
   <212> PRT
   <213> Artificial Sequence
<220>
   <223> CPP
<400> 128
   Arg Gln Ile Lys Ala Trp Phe Gln Asn Arg Arg Met Lys Trp Lys Lys
   1               5                   10                  15
<210> 129
   <211> 16
   <212> PRT
   <213> Artificial Sequence
<220>
   <223> CPP
<400> 129
   Arg Gln Ile Lys Ile Ala Phe Gln Asn Arg Arg Met Lys Trp Lys Lys
   1               5                   10                  15
<210> 130
   <211> 16
   <212> PRT
   <213> Artificial Sequence
<220>
   <223> CPP
<400> 130
   Arg Gln Ile Lys Ile Trp Ala Gln Asn Arg Arg Met Lys Trp Lys Lys
   1 5 10 15
<210> 131
   <211> 16
   <212> PRT
   <213> Artificial Sequence
<220>
   <223> CPP
<400> 131
   Arg Gln Ile Lys Ile Trp Phe Ala Asn Arg Arg Met Lys Trp Lys Lys
   1 5 10 15
<210> 132
   <211> 16
   <212> PRT
   <213> Artificial Sequence
<220>
   <223> CPP
<400> 132
   Arg Gln Ile Lys Ile Trp Phe Gln Ala Arg Arg Met Lys Trp Lys Lys
   1 5 10 15
<210> 133
   <211> 16
   <212> PRT
   <213> Artificial Sequence
<220>
   <223> CPP
<400> 133
   Arg Gln Ile Lys Ile Trp Phe Gln Asn Ala Arg Met Lys Trp Lys Lys
   1 5 10 15
<210> 134
   <211> 16
   <212> PRT
   <213> Artificial Sequence
<220>
   <223> CPP
<400> 134
   Arg Gln Ile Lys Ile Trp Phe Gln Asn Arg Ala Met Lys Trp Lys Lys
   1 5 10 15
<210> 135
   <211> 16
   <212> PRT
   <213> Artificial Sequence
<220>
   <223> CPP
<400> 135
   Arg Gln Ile Lys Ile Trp Phe Gln Asn Arg Arg Ala Lys Trp Lys Lys
   1               5                   10                  15
<210> 136
   <211> 16
   <212> PRT
   <213> Artificial Sequence
<220>
   <223> CPP
<400> 136
   Arg Gln Ile Lys Ile Trp Phe Gln Asn Arg Arg Met Ala Trp Lys Lys
   1               5                   10                  15
<210> 137
   <211> 16
   <212> PRT
   <213> Artificial Sequence
<220>
   <223> CPP
<400> 137
   Arg Gln Ile Lys Ile Trp Phe Gln Asn Arg Arg Met Lys Ala Lys Lys
   1               5                   10                  15
<210> 138
   <211> 16
   <212> PRT
   <213> Artificial Sequence
<220>
   <223> CPP
<400> 138
   Arg Gln Ile Lys Ile Trp Phe Gln Asn Arg Arg Met Lys Trp Ala Lys
   1               5                   10                  15
<210> 139
   <211> 16
   <212> PRT
   <213> Artificial Sequence
<220>
   <223> CPP
<400> 139
   Arg Gln Ile Lys Ile Trp Phe Gln Asn Arg Arg Met Lys Trp Lys Ala
   1 5 10 15
<210> 140
   <211> 18
   <212> PRT
   <213> Artificial Sequence
<220>
   <223> CPP
<400> 140
   Cys Arg Gln Ile Lys Ile Trp Phe Pro Asn Arg Arg Met Lys Trp Lys
   1 5 10 15
   Lys Cys
<210> 141
   <211> 16
   <212> PRT
   <213> Artificial Sequence
<220>
   <223> CPP
<400> 141
   Arg Gln Ile Lys Ile Trp Phe Pro Asn Arg Arg Met Lys Trp Lys Lys
   1 5 10 15
<210> 142
   <211> 16
   <212> PRT
   <213> Artificial Sequence
<220>
   <223> CPP
<400> 142
   Arg Gln Ile Lys Ile Phe Phe Gln Asn Arg Arg Met Lys Phe Lys Lys
   1 5 10 15
<210> 143
   <211> 16
   <212> PRT
   <213> Artificial Sequence
<220>
   <223> CPP
<400> 143
   Arg Gln Ile Arg Ile Trp Phe Gln Asn Arg Arg Met Arg Trp Arg Arg
   1 5 10 15
<210> 144
   <211> 8
   <212> PRT
   <213> Artificial Sequence
<220>
   <223> CPP
<400> 144
   Arg Arg Arg Arg Arg Arg Arg Trp
   1               5
<210> 145
   <211> 13
   <212> PRT
   <213> Artificial Sequence
<220>
   <223> CPP
<400> 145
   Gly Arg Lys Lys Arg Arg Gln Arg Arg Arg Pro Trp Gln
   1               5                   10
<210> 146
   <211> 13
   <212> PRT
   <213> Artificial Sequence
<220>
   <223> CPP
<400> 146
   Gly Arg Lys Lys Arg Arg Gln Arg Arg Arg Pro Trp Gln
   1               5                   10
<210> 147
   <211> 16
   <212> PRT
   <213> Artificial Sequence
<220>
   <223> CPP
<400> 147
   Arg Gln Ile Arg Ile Trp Phe Gln Asn Arg Arg Met Arg Trp Arg Arg
   1               5                   10                  15
<210> 148
   <211> 16
   <212> PRT
   <213> Artificial Sequence
<220>
   <223> CPP
<400> 148
   Arg Arg Trp Arg Arg Trp Trp Arg Arg Trp Trp Arg Arg Trp Arg Arg
   1               5                   10                  15
<210> 149
   <211> 16
   <212> PRT
   <213> Artificial Sequence
<220>
   <223> CPP
<400> 149
   Arg Gln Ile Lys Ile Trp Phe Gln Asn Met Arg Arg Lys Trp Lys Lys
   1               5                   10                  15
<210> 150
   <211> 13
   <212> PRT
   <213> Artificial Sequence
<220>
   <223> CPP
<400> 150
   Lys Met Asp Cys Arg Trp Arg Trp Lys Cys Cys Lys Lys
   1               5                   10
<210> 151
   <211> 12
   <212> PRT
   <213> Artificial Sequence
<220>
   <223> CPP
<400> 151
   Met Asp Cys Arg Trp Arg Trp Lys Cys Cys Lys Lys
   1               5                   10
<210> 152
   <211> 11
   <212> PRT
   <213> Artificial Sequence
<220>
   <223> CPP
<400> 152
   Asp Cys Arg Trp Arg Trp Lys Cys Cys Lys Lys
   1               5                   10
<210> 153
   <211> 10
   <212> PRT
   <213> Artificial Sequence
<220>
   <223> CPP
<400> 153
   Cys Arg Trp Arg Trp Lys Cys Cys Lys Lys
   1               5                   10
<210> 154
   <211> 9
   <212> PRT
   <213> Artificial Sequence
<220>
   <223> CPP
<400> 154
   Arg Trp Arg Trp Lys Cys Cys Lys Lys
   1               5
<210> 155
   <211> 12
   <212> PRT
   <213> Artificial Sequence
<220>
   <223> CPP
<400> 155
   Lys Met Asp Cys Arg Trp Arg Trp Lys Cys Lys Lys
   1               5                   10
<210> 156
   <211> 11
   <212> PRT
   <213> Artificial Sequence
<220>
   <223> CPP
<400> 156
   Lys Met Asp Cys Arg Trp Arg Trp Lys Lys Lys
   1               5                   10
<210> 157
   <211> 10
   <212> PRT
   <213> Artificial Sequence
<220>
   <223> CPP
<400> 157
   Lys Met Asp Arg Trp Arg Trp Lys Lys Lys
   1               5                   10
<210> 158
   <211> 12
   <212> PRT
   <213> Artificial Sequence
<220>
   <223> CPP
<400> 158
   Lys Asp Cys Arg Trp Arg Trp Lys Cys Cys Lys Lys
   1               5                   10
<210> 159
   <211> 11
   <212> PRT
   <213> Artificial Sequence
<220>
   <223> CPP
<400> 159
   Lys Cys Arg Trp Arg Trp Lys Cys Cys Lys Lys
   1               5                   10
<210> 160
   <211> 10
   <212> PRT
   <213> Artificial Sequence
<220>
   <223> CPP
<400> 160
   Lys Arg Trp Arg Trp Lys Cys Cys Lys Lys
   1               5                   10
<210> 161
   <211> 12
   <212> PRT
   <213> Artificial Sequence
<220>
   <223> CPP
<220>
   <221> misc_feature
   <222> (9)..(9)
   <223> Xaa can be any naturally occurring amino acid
<400> 161
   Met Asp Cys Arg Trp Arg Trp Lys Xaa Cys Lys Lys
   1 5 10
<210> 162
   <211> 11
   <212> PRT
   <213> Artificial Sequence
<220>
   <223> CPP
<220>
   <221> misc_feature
   <222> (8)..(8)
   <223> Xaa can be any naturally occurring amino acid
<400> 162
   Asp Cys Arg Trp Arg Trp Lys Xaa Cys Lys Lys
   1 5 10
<210> 163
   <211> 11
   <212> PRT
   <213> Artificial Sequence
<220>
   <223> CPP
<220>
   <221> misc_feature
   <222> (9)..(9)
   <223> Xaa can be any naturally occurring amino acid
<400> 163
   Asp Cys Arg Trp Arg Trp Lys Cys Xaa Lys Lys
   1 5 10
<210> 164
   <211> 10
   <212> PRT
   <213> Artificial Sequence
<220>
   <223> CPP
<220>
   <221> misc_feature
   <222> (7)..(7)
   <223> Xaa can be any naturally occurring amino acid
<400> 164
   Cys Arg Trp Arg Trp Lys Xaa Cys Lys Lys
   1 5 10
<210> 165
   <211> 10
   <212> PRT
   <213> Artificial Sequence
<220>
   <223> CPP
<220>
   <221> misc_feature
   <222> (8)..(8)
   <223> Xaa can be any naturally occurring amino acid
<400> 165
   Cys Arg Trp Arg Trp Lys Cys Xaa Lys Lys
   1               5                   10
<210> 166
   <211> 9
   <212> PRT
   <213> Artificial Sequence
<220>
   <223> CPP
<220>
   <221> misc_feature
   <222> (6)..(6)
   <223> Xaa can be any naturally occurring amino acid
<400> 166
   Arg Trp Arg Trp Lys Xaa Cys Lys Lys
   1               5
<210> 167
   <211> 12
   <212> PRT
   <213> Artificial Sequence
<220>
   <223> CPP
<220>
   <221> misc_feature
   <222> (9)..(10)
   <223> Xaa can be any naturally occurring amino acid
<400> 167
   Met Asp Cys Arg Trp Arg Trp Lys Xaa Xaa Lys Lys
   1               5                   10
<210> 168
   <211> 11
   <212> PRT
   <213> Artificial Sequence
<220>
   <223> CPP
<220>
   <221> misc_feature
   <222> (8)..(9)
   <223> Xaa can be any naturally occurring amino acid
<400> 168
   Asp Cys Arg Trp Arg Trp Lys Xaa Xaa Lys Lys
   1               5                   10
<210> 169
   <211> 10
   <212> PRT
   <213> Artificial Sequence
<220>
   <223> CPP
<220>
   <221> misc_feature
   <222> (7)..(8)
   <223> Xaa can be any naturally occurring amino acid
<400> 169
   Cys Arg Trp Arg Trp Lys Xaa Xaa Lys Lys
   1               5                   10
<210> 170
   <211> 9
   <212> PRT
   <213> Artificial Sequence
<220>
   <223> CPP
<220>
   <221> misc_feature
   <222> (6)..(7)
   <223> Xaa can be any naturally occurring amino acid
<400> 170
   Arg Trp Arg Trp Lys Xaa Xaa Lys Lys
   1               5
<210> 171
   <211> 10
   <212> PRT
   <213> Artificial Sequence
<220>
   <223> CPP
<400> 171
   Cys Arg Trp Arg Trp Lys Cys Ser Lys Lys
   1 5 10
<210> 172
   <211> 10
   <212> PRT
   <213> Artificial Sequence
<220>
   <223> CPP
<400> 172
   Ser Arg Trp Arg Trp Lys Cys Cys Lys Lys
   1 5 10
<210> 173
   <211> 10
   <212> PRT
   <213> Artificial Sequence
<220>
   <223> CPP
<400> 173
   Ser Arg Trp Arg Trp Lys Cys Ser Lys Lys
   1 5 10
<210> 174
   <211> 10
   <212> PRT
   <213> Artificial Sequence
<220>
   <223> CPP
<400> 174
   Ser Arg Trp Arg Trp Lys Ser Cys Lys Lys
   1 5 10
<210> 175
   <211> 10
   <212> PRT
   <213> Artificial Sequence
<220>
   <223> CPP
<400> 175
   Cys Arg Trp Arg Trp Lys Ser Ser Lys Lys
   1 5 10
<210> 176
   <211> 10
   <212> PRT
   <213> Artificial Sequence
<220>
   <223> CPP
<400> 176
   Ser Arg Trp Arg Trp Lys Ser Ser Lys Lys
   1               5                   10
<210> 177
   <211> 10
   <212> PRT
   <213> Artificial Sequence
<220>
   <223> CPP
<400> 177
   Cys Arg Phe Arg Trp Lys Cys Cys Lys Lys
   1               5                   10
<210> 178
   <211> 10
   <212> PRT
   <213> Artificial Sequence
<220>
   <223> CPP
<400> 178
   Cys Arg Trp Arg Phe Lys Cys Cys Lys Lys
   1               5                   10
<210> 179
   <211> 10
   <212> PRT
   <213> Artificial Sequence
<220>
   <223> CPP
<400> 179
   Cys Arg Phe Arg Phe Lys Cys Cys Lys Lys
   1               5                   10
<210> 180
   <211> 10
   <212> PRT
   <213> Artificial Sequence
<220>
   <223> CPP
<400> 180
   Cys Arg Trp Arg Trp Lys Cys Cys Lys Lys
   1 5 10
<210> 181
   <211> 10
   <212> PRT
   <213> Artificial Sequence
<220>
   <223> CPP
<400> 181
   Lys Cys Cys Lys Trp Arg Trp Arg Cys Lys
   1 5 10
<210> 182
   <211> 10
   <212> PRT
   <213> Artificial Sequence
<220>
   <223> CPP
<400> 182
   Lys Cys Cys Lys Trp Arg Trp Arg Cys Lys
   1 5 10
<210> 183
   <211> 10
   <212> PRT
   <213> Artificial Sequence
<220>
   <223> CPP
<400> 183
   Cys Arg Trp Arg Trp Lys Cys Cys Lys Lys
   1 5 10
<210> 184
   <211> 10
   <212> PRT
   <213> Artificial Sequence
<220>
   <223> CPP
<400> 184
   Cys Arg Trp Arg Trp Lys Cys Cys Lys Lys
   1 5 10
<210> 185
   <211> 10
   <212> PRT
   <213> Artificial Sequence
<220>
   <223> CPP
<400> 185
   Cys Arg Trp Arg Trp Lys Cys Cys Lys Lys
   1 5 10
<210> 186
   <211> 10
   <212> PRT
   <213> Artificial Sequence
<220>
   <223> CPP
<400> 186
   Cys Arg Trp Arg Trp Lys Cys Cys Lys Lys
   1 5 10
<210> 187
   <211> 10
   <212> PRT
   <213> Artificial Sequence
<220>
   <223> CPP
<400> 187
   Cys Arg Trp Arg Trp Lys Cys Cys Lys Lys
   1 5 10
<210> 188
   <211> 11
   <212> PRT
   <213> Artificial Sequence
<220>
   <223> CPP
<400> 188
   Cys Arg Trp Arg Trp Lys Cys Gly Cys Lys Lys
   1 5 10
<210> 189
   <211> 14
   <212> PRT
   <213> Artificial Sequence
<220>
   <223> CPP
<400> 189
   Lys Cys Gly Cys Arg Trp Arg Trp Lys Cys Gly Cys Lys Lys
   1 5 10
<210> 190
   <211> 8
   <212> PRT
   <213> Artificial Sequence
<220>
   <223> CPP
<400> 190
   Cys Arg Trp Arg Trp Lys Cys Gly
   1 5
<210> 191
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<400> 191
   Lys Met Asp Xaa Arg Trp Arg Trp Lys Cys Cys Lys Lys
   1 5 10
<210> 192
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<220>
   <221> misc_feature
   <222> (10)..(10)
   <223> Xaa can be any naturally occurring amino acid
<400> 192
   Lys Met Asp Xaa Arg Trp Arg Trp Lys Xaa Cys Lys Lys
   1 5 10
<210> 193
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<220>
   <221> misc_feature
   <222> (10)..(11)
   <223> Xaa can be any naturally occurring amino acid
<400> 193
   Lys Met Asp Xaa Arg Trp Arg Trp Lys Xaa Xaa Lys Lys
   1               5                   10
<210> 194
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<220>
   <221> misc_feature
   <222> (11)..(11)
   <223> Xaa can be any naturally occurring amino acid
<400> 194
   Lys Met Asp Xaa Arg Trp Arg Trp Lys Cys Xaa Lys Lys
   1               5                   10
<210> 195
   <211> 12
   <212> PRT
   <213> Artificial Sequence
<220>
   <223> CPP
<220>
   <221> misc_feature
   <222> (10)..(10)
   <223> Xaa can be any naturally occurring amino acid
<400> 195
   Met Asp Cys Arg Trp Arg Trp Lys Cys Xaa Lys Lys
   1 5 10
<210> 196
   <211> 13
   <212> PRT
   <213> Artificial Sequence
<220>
   <223> CPP
<400> 196
   Lys Met Asp Cys Arg Trp Arg Trp Lys Cys Ser Lys Lys
   1 5 10
<210> 197
   <211> 13
   <212> PRT
   <213> Artificial Sequence
<220>
   <223> CPP
<400> 197
   Lys Met Asp Cys Arg Trp Arg Trp Lys Ser Cys Lys Lys
   1 5 10
<210> 198
   <211> 13
   <212> PRT
   <213> Artificial Sequence
<220>
   <223> CPP
<400> 198
   Lys Met Asp Ser Arg Trp Arg Trp Lys Cys Cys Lys Lys
   1 5 10
<210> 199
   <211> 13
   <212> PRT
   <213> Artificial Sequence
<220>
   <223> CPP
<400> 199
   Lys Met Asp Cys Arg Trp Arg Trp Lys Ser Ser Lys Lys
   1 5 10
<210> 200
   <211> 13
   <212> PRT
   <213> Artificial Sequence
<220>
   <223> CPP
<400> 200
   Lys Met Asp Ser Arg Trp Arg Trp Lys Ser Ser Lys Lys
   1               5                   10
<210> 201
   <211> 13
   <212> PRT
   <213> Artificial Sequence
<220>
   <223> CPP
<400> 201
   Lys Met Asp Ser Arg Trp Arg Trp Lys Ser Cys Lys Lys
   1               5                   10
<210> 202
   <211> 13
   <212> PRT
   <213> Artificial Sequence
<220>
   <223> CPP
<400> 202
   Lys Met Asp Ser Arg Trp Arg Trp Lys Cys Ser Lys Lys
   1               5                   10
<210> 203
   <211> 13
   <212> PRT
   <213> Artificial Sequence
<220>
   <223> CPP
<400> 203
   Lys Met Asp Cys Arg Trp Arg Pro Lys Cys Cys Lys Lys
   1               5                   10
<210> 204
   <211> 13
   <212> PRT
   <213> Artificial Sequence
<220>
   <223> CPP
<400> 204
   Lys Met Asp Cys Arg Pro Arg Pro Lys Cys Cys Lys Lys
   1               5                   10
<210> 205
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<400> 205
   Lys Met Asp Xaa Arg Pro Arg Pro Lys Cys Cys Lys Lys
   1               5                   10
<210> 206
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<220>
   <221> misc_feature
   <222> (10)..(10)
   <223> Xaa can be any naturally occurring amino acid
<400> 206
   Lys Met Asp Xaa Arg Pro Arg Pro Lys Xaa Cys Lys Lys
   1               5                   10
<210> 207
   <211> 13
   <212> PRT
   <213> Artificial Sequence
<220>
   <223> CPP
<220>
   <221> misc_feature
   <222> (4)..(4)
   <223> Xaa can be any naturally occurring amino acid
<220>
   <221> misc_feature
   <222> (11)..(11)
   <223> Xaa can be any naturally occurring amino acid
<400> 207
   Lys Met Asp Xaa Arg Pro Arg Pro Lys Cys Xaa Lys Lys
   1               5                   10
<210> 208
   <211> 13
   <212> PRT
   <213> Artificial Sequence
<220>
   <223> CPP
<220>
   <221> misc_feature
   <222> (10)..(10)
   <223> Xaa can be any naturally occurring amino acid
<400> 208
   Lys Met Asp Cys Arg Pro Arg Pro Lys Xaa Cys Lys Lys
   1               5                   10
<210> 209
   <211> 13
   <212> PRT
   <213> Artificial Sequence
<220>
   <223> CPP
<220>
   <221> misc_feature
   <222> (11)..(11)
   <223> Xaa can be any naturally occurring amino acid
<400> 209
   Lys Met Asp Cys Arg Pro Arg Pro Lys Cys Xaa Lys Lys
   1               5                   10
<210> 210
   <211> 9
   <212> PRT
   <213> Artificial Sequence
<220>
   <223> CPP
<400> 210
   Arg Lys Lys Arg Arg Gln Arg Arg Arg
   1               5
<210> 211
   <211> 9
   <212> PRT
   <213> Artificial Sequence
<220>
   <223> CPP
<400> 211
   Arg Arg Arg Gln Arg Arg Lys Lys Arg
   1 5
<210> 212
   <211> 16
   <212> PRT
   <213> Artificial Sequence
<220>
   <223> CPP
<400> 212
   Arg Lys Lys Arg Arg Arg Glu Ser Arg Lys Lys Arg Arg Arg Glu Ser
   1 5 10 15
<210> 213
   <211> 17
   <212> PRT
   <213> Artificial Sequence
<220>
   <223> CPP
<400> 213
   Gly Arg Pro Arg Glu Ser Gly Lys Lys Arg Lys Arg Lys Arg Leu Lys
   1 5 10 15
   Pro
<210> 214
   <211> 15
   <212> PRT
   <213> Artificial Sequence
<220>
   <223> CPP
<400> 214
   Gly Lys Arg Lys Lys Lys Gly Lys Leu Gly Lys Lys Arg Asp Pro
   1 5 10 15
<210> 215
   <211> 17
   <212> PRT
   <213> Artificial Sequence
<220>
   <223> CPP
<400> 215
   Gly Lys Arg Lys Lys Lys Gly Lys Leu Gly Lys Lys Arg Pro Arg Ser
   1 5 10 15
   Arg
<210> 216
   <211> 18
   <212> PRT
   <213> Artificial Sequence
<220>
   <223> CPP
<400> 216
   Arg Lys Lys Arg Arg Arg Glu Ser Arg Arg Ala Arg Arg Ser Pro Arg
   1 5 10 15
   His Leu
<210> 217
   <211> 19
   <212> PRT
   <213> Artificial Sequence
<220>
   <223> CPP
<400> 217
   Ser Arg Arg Ala Arg Arg Ser Pro Arg Glu Ser Gly Lys Lys Arg Lys
   1 5 10 15
   Arg Lys Arg
<210> 218
   <211> 19
   <212> PRT
   <213> Artificial Sequence
<220>
   <223> CPP
<400> 218
   Val Lys Arg Gly Leu Lys Leu Arg His Val Arg Pro Arg Val Thr Arg
   1 5 10 15
   Met Asp Val
<210> 219
   <211> 14
   <212> PRT
   <213> Artificial Sequence
<220>
   <223> CPP
<400> 219
   Ser Arg Arg Ala Arg Arg Ser Pro Arg His Leu Gly Ser Gly
   1 5 10
<210> 220
   <211> 16
   <212> PRT
   <213> Artificial Sequence
<220>
   <223> CPP
<400> 220
   Leu Arg Arg Glu Arg Gln Ser Arg Leu Arg Arg Glu Arg Gln Ser Arg
   1 5 10 15
<210> 221
   <211> 22
   <212> PRT
   <213> Artificial Sequence
<220>
   <223> CPP
<400> 221
   Gly Ala Tyr Asp Leu Arg Arg Arg Glu Arg Gln Ser Arg Leu Arg Arg
   1 5 10 15
   Arg Glu Arg Gln Ser Arg
   20
<210> 222
   <211> 5
   <212> PRT
   <213> Artificial Sequence
<220>
   <223> CPP
<400> 222
   Val Pro Met Leu Lys
   1 5
<210> 223
   <211> 5
   <212> PRT
   <213> Artificial Sequence
<220>
   <223> CPP
<400> 223
   Val Pro Thr Leu Lys
   1 5
<210> 224
   <211> 5
   <212> PRT
   <213> Artificial Sequence
<220>
   <223> CPP
<400> 224
   Val Pro Ala Leu Arg
   1 5
<210> 225
   <211> 5
   <212> PRT
   <213> Artificial Sequence
<220>
   <223> CPP
<400> 225
   Val Ser Ala Leu Lys
   1 5
<210> 226
   <211> 5
   <212> PRT
   <213> Artificial Sequence
<220>
   <223> CPP
<400> 226
   Pro Met Leu Lys Glu
   1 5
<210> 227
   <211> 5
   <212> PRT
   <213> Artificial Sequence
<220>
   <223> CPP
<400> 227
   Val Pro Ala Leu Lys
   1               5
<210> 228
   <211> 5
   <212> PRT
   <213> Artificial Sequence
<220>
   <223> CPP
<400> 228
   Val Ser Leu Lys Lys
   1               5
<210> 229
   <211> 5
   <212> PRT
   <213> Artificial Sequence
<220>
   <223> CPP
<400> 229
   Val Ser Gly Lys Lys
   1               5
<210> 230
   <211> 5
   <212> PRT
   <213> Artificial Sequence
<220>
   <223> CPP
<400> 230
   Lys Leu Pro Val Met
   1               5
<210> 231
   <211> 5
   <212> PRT
   <213> Artificial Sequence
<220>
   <223> CPP
<400> 231
   Ile Pro Met Ile Lys
   1 5
<210> 232
   <211> 5
   <212> PRT
   <213> Artificial Sequence
<220>
   <223> CPP
<400> 232
   Lys Leu Gly Val Met
   1 5
<210> 233
   <211> 5
   <212> PRT
   <213> Artificial Sequence
<220>
   <223> CPP
<400> 233
   Lys Leu Pro Val Thr
   1 5
<210> 234
   <211> 5
   <212> PRT
   <213> Artificial Sequence
<220>
   <223> CPP
<400> 234
   Val Pro Met Ile Lys
   1 5
<210> 235
   <211> 5
   <212> PRT
   <213> Artificial Sequence
<220>
   <223> CPP
<400> 235
   Ile Pro Ala Leu Lys
   1 5
<210> 236
   <211> 5
   <212> PRT
   <213> Artificial Sequence
<220>
   <223> CPP
<400> 236
   Ile Pro Met Leu Lys
   1 5
<210> 237
   <211> 5
   <212> PRT
   <213> Artificial Sequence
<220>
   <223> CPP
<400> 237
   Val Pro Thr Leu Gln
   1 5
<210> 238
   <211> 5
   <212> PRT
   <213> Artificial Sequence
<220>
   <223> CPP
<400> 238
   Gln Leu Pro Val Met
   1 5
<210> 239
   <211> 5
   <212> PRT
   <213> Artificial Sequence
<220>
   <223> CPP
<400> 239
   Glu Leu Pro Val Met
   1 5
<210> 240
   <211> 5
   <212> PRT
   <213> Artificial Sequence
<220>
   <223> CPP
<400> 240
   Val Pro Thr Leu Glu
   1               5
<210> 241
   <211> 5
   <212> PRT
   <213> Artificial Sequence
<220>
   <223> CPP
<400> 241
   Val Pro Thr Leu Lys
   1               5
<210> 242
   <211> 20
   <212> PRT
   <213> Artificial Sequence
<220>
   <223> CPP
<400> 242
   Ala Tyr Arg Ile Lys Pro Thr Phe Arg Arg Leu Lys Trp Lys Tyr Lys
   1               5                   10                  15
   Gly Lys Phe Trp
               20
<210> 243
   <211> 20
   <212> PRT
   <213> Artificial Sequence
<220>
   <223> CPP
<400> 243
   His Ala Arg Ile Lys Pro Thr Phe Arg Arg Leu Lys Trp Lys Tyr Lys
   1               5                   10                  15
   Gly Lys Phe Trp
               20
<210> 244
   <211> 20
   <212> PRT
   <213> Artificial Sequence
<220>
   <223> CPP
<400> 244
   His Tyr Arg Ile Lys Pro Thr Ala Arg Arg Leu Lys Trp Lys Tyr Lys
   1               5                   10                  15
   Gly Lys Phe Trp
               20
<210> 245
   <211> 20
   <212> PRT
   <213> Artificial Sequence
<220>
   <223> CPP
<400> 245
   His Tyr Arg Ile Lys Pro Thr Phe Arg Arg Leu Ala Trp Lys Tyr Lys
   1               5                   10                  15
   Gly Lys Phe Trp
               20
<210> 246
   <211> 20
   <212> PRT
   <213> Artificial Sequence
<220>
   <223> CPP
<400> 246
   His Tyr Arg Ile Lys Pro Thr Phe Arg Arg Leu Lys Trp Lys Tyr Lys
   1               5                   10                  15
   Gly Lys Phe Ala
               20
<210> 247
   <211> 21
   <212> PRT
   <213> Artificial Sequence
<220>
   <223> CPP
<400> 247
   Val Asn Ala Asp Ile Lys Ala Thr Thr Val Phe Gly Gly Lys Tyr Val
   1               5                   10                  15
   Ser Leu Thr Thr Pro
               20
<210> 248
   <211> 22
   <212> PRT
   <213> Artificial Sequence
<220>
   <223> CPP
<400> 248
   Gly Lys Tyr Val Ser Leu Thr Thr Pro Lys Asn Pro Thr Lys Arg Arg
   1               5                   10                  15
   Ile Thr Pro Lys Asp Val
               20
<210> 249
   <211> 22
   <212> PRT
   <213> Artificial Sequence
<220>
   <223> CPP
<400> 249
   Thr Lys Arg Arg Ile Thr Pro Lys Asp Val Ile Asp Val Arg Ser Val
   1               5                   10                  15
   Thr Thr Glu Ile Asn Thr
               20
<210> 250
   <211> 23
   <212> PRT
   <213> Artificial Sequence
<220>
   <223> CPP
<400> 250
   Arg Ser Val Thr Thr Glu Ile Asn Thr Leu Phe Gln Thr Leu Thr Ser
   1               5                   10                  15
   Ile Ala Glu Lys Val Asp Pro
               20
<210> 251
   <211> 26
   <212> PRT
   <213> Artificial Sequence
<220>
   <223> CPP
<400> 251
   Ala Glu Lys Val Asp Pro Val Lys Leu Asn Leu Thr Leu Ser Ala Ala
   1               5                   10                  15
   Ala Glu Ala Leu Thr Gly Leu Gly Asp Lys
               20                  25
<210> 252
   <211> 34
   <212> PRT
   <213> Artificial Sequence
<220>
   <223> CPP
<400> 252
   Gly Leu Gly Asp Lys Phe Gly Glu Ser Ile Val Asn Ala Asn Thr Val
   1               5                   10                  15
   Leu Asp Asp Leu Asn Ser Arg Met Pro Gln Ser Arg His Asp Ile Gln
               20                  25                  30
   Gln Leu
<210> 253
   <211> 27
   <212> PRT
   <213> Artificial Sequence
<220>
   <223> CPP
<400> 253
   Gly Asp Val Tyr Ala Asp Ala Ala Pro Asp Leu Phe Asp Phe Leu Asp
   1               5                   10                  15
   Ser Ser Val Thr Thr Ala Arg Thr Ile Asn Ala
               20                  25
<210> 254
   <211> 32
   <212> PRT
   <213> Artificial Sequence
<220>
   <223> CPP
<400> 254
   Ala Arg Thr Ile Asn Ala Gln Gln Ala Glu Leu Asp Ser Ala Leu Leu
   1               5                   10                  15
   Ala Ala Ala Gly Phe Gly Asn Thr Thr Ala Asp Val Phe Asp Arg Gly
               20                  25                  30
<210> 255
   <211> 30
   <212> PRT
   <213> Artificial Sequence
<220>
   <223> CPP
<400> 255
   Ala Asp Val Phe Asp Arg Gly Gly Pro Tyr Leu Gln Arg Gly Val Ala
   1               5                   10                  15
   Asp Leu Val Pro Thr Ala Thr Leu Leu Asp Thr Tyr Ser Pro
               20                  25                  30
<210> 256
   <211> 27
   <212> PRT
   <213> Artificial Sequence
<220>
   <223> CPP
<400> 256
   Leu Asp Thr Tyr Ser Pro Glu Leu Phe Cys Thr Ile Arg Asn Phe Tyr
   1               5                   10                  15
   Asp Ala Asp Arg Pro Asp Arg Gly Ala Ala Ala
               20                  25
<210> 257
   <211> 22
   <212> PRT
   <213> Artificial Sequence
<220>
   <223> CPP
<400> 257
   Thr Lys Arg Arg Ile Thr Pro Lys Asp Val Ile Asp Val Arg Ser Val
   1               5                   10                  15
   Thr Thr Glu Ile Asn Thr
               20
<210> 258
   <211> 22
   <212> PRT
   <213> Artificial Sequence
<220>
   <223> CPP
<400> 258
   Thr Lys Arg Arg Ile Thr Pro Asp Asp Val Ile Asp Val Arg Ser Val
   1               5                   10                  15
   Thr Thr Glu Ile Asn Thr
               20
<210> 259
   <211> 22
   <212> PRT
   <213> Artificial Sequence
<220>
   <223> CPP
<400> 259
   Thr Lys Arg Arg Ile Thr Pro Lys Lys Val Ile Asp Val Arg Ser Val
   1               5                   10                  15
   Thr Thr Glu Ile Asn Thr
               20
<210> 260
   <211> 22
   <212> PRT
   <213> Artificial Sequence
<220>
   <223> CPP
<400> 260
   Thr Lys Arg Arg Ile Thr Pro Lys Asp Val Ile Asp Val Arg Ser Val
   1               5                   10                  15
   Thr Thr Lys Ile Asn Thr
               20
<210> 261
   <211> 13
   <212> PRT
   <213> Artificial Sequence
<220>
   <223> CPP
<400> 261
   Thr Lys Arg Arg Ile Thr Pro Lys Asp Val Ile Asp Val
   1               5                   10
<210> 262
   <211> 22
   <212> PRT
   <213> Artificial Sequence
<220>
   <223> CPP
<400> 262
   Thr Lys Arg Arg Ile Thr Pro Lys Asp Val Ile Asp Val Glu Ser Val
   1               5                   10                  15
   Thr Thr Glu Ile Asn Thr
               20
<210> 263
   <211> 22
   <212> PRT
   <213> Artificial Sequence
<220>
   <223> CPP
<400> 263
   Thr Ala Arg Arg Ile Thr Pro Lys Asp Val Ile Asp Val Arg Ser Val
   1               5                   10                  15
   Thr Thr Glu Ile Asn Thr
               20
<210> 264
   <211> 23
   <212> PRT
   <213> Artificial Sequence
<220>
   <223> CPP
<400> 264
   Thr Lys Ala Ala Arg Ile Thr Pro Lys Asp Val Ile Asp Val Arg Ser
   1 5 10 15
   Val Thr Thr Glu Ile Asn Thr
   20
<210> 265
   <211> 28
   <212> PRT
   <213> Artificial Sequence
<220>
   <223> CPP
<400> 265
   His His His His His His Thr Lys Arg Arg Ile Thr Pro Lys Asp Val
   1 5 10 15
   Ile Asp Val Arg Ser Val Thr Thr Glu Ile Asn Thr
   20 25
<210> 266
   <211> 15
   <212> PRT
   <213> Artificial Sequence
<220>
   <223> CPP
<400> 266
   Lys Leu Trp Met Arg Trp Tyr Ser Pro Thr Thr Arg Arg Tyr Gly
   1 5 10 15
<210> 267
   <211> 15
   <212> PRT
   <213> Artificial Sequence
<220>
   <223> CPP
<400> 267
   Asp Ser Leu Lys Ser Tyr Trp Tyr Leu Gln Lys Phe Ser Trp Arg
   1 5 10 15
<210> 268
   <211> 15
   <212> PRT
   <213> Artificial Sequence
<220>
   <223> CPP
<400> 268
   Arg Thr Leu Val Asn Glu Tyr Lys Asn Thr Leu Lys Phe Ser Lys
   1               5                   10                  15
<210> 269
   <211> 15
   <212> PRT
   <213> Artificial Sequence
<220>
   <223> CPP
<400> 269
   Ile Pro Ser Arg Trp Lys Asp Gln Phe Trp Lys Arg Trp His Tyr
   1               5                   10                  15
<210> 270
   <211> 15
   <212> PRT
   <213> Artificial Sequence
<220>
   <223> CPP
<400> 270
   Gly Tyr Gly Asn Cys Arg His Phe Lys Gln Lys Pro Arg Arg Asp
   1               5                   10                  15
<210> 271
   <211> 15
   <212> PRT
   <213> Artificial Sequence
<220>
   <223> CPP
<400> 271
   Lys Asn Ala Trp Lys His Ser Ser Cys His His Arg His Gln Ile
   1               5                   10                  15
<210> 272
   <211> 15
   <212> PRT
   <213> Artificial Sequence
<220>
   <223> CPP
<400> 272
   Arg Val Arg Glu Trp Trp Tyr Thr Ile Thr Leu Lys Gln Glu Ser
   1               5                   10                  15
<210> 273
   <211> 15
   <212> PRT
   <213> Artificial Sequence
<220>
   <223> CPP
<400> 273
   Gln Gln His Leu Leu Ile Ala Ile Asn Gly Tyr Pro Arg Tyr Asn
   1 5 10 15
<210> 274
   <211> 15
   <212> PRT
   <213> Artificial Sequence
<220>
   <223> CPP
<400> 274
   Trp Lys Cys Arg Arg Gln Cys Phe Arg Val Leu His His Trp Asn
   1 5 10 15
<210> 275
   <211> 15
   <212> PRT
   <213> Artificial Sequence
<220>
   <223> CPP
<400> 275
   Arg Leu Trp Met Arg Trp Tyr Ser Pro Thr Thr Arg Arg Tyr Gly
   1 5 10 15
<210> 276
   <211> 15
   <212> PRT
   <213> Artificial Sequence
<220>
   <223> CPP
<400> 276
   Lys Leu Trp Met Arg Trp Tyr Ser Ala Thr Thr Arg Arg Tyr Gly
   1 5 10 15
<210> 277
   <211> 15
   <212> PRT
   <213> Artificial Sequence
<220>
   <223> CPP
<400> 277
   Lys Leu Trp Met Arg Trp Tyr Ser Pro Trp Thr Arg Arg Tyr Gly
   1 5 10 15
<210> 278
   <211> 15
   <212> PRT
   <213> Artificial Sequence
<220>
   <223> CPP
<400> 278
   Arg Leu Trp Met Arg Trp Tyr Ser Pro Trp Thr Arg Arg Tyr Gly
   1 5 10 15
<210> 279
   <211> 15
   <212> PRT
   <213> Artificial Sequence
<220>
   <223> CPP
<400> 279
   Arg Leu Trp Met Arg Trp Tyr Ser Pro Trp Thr Arg Arg Trp Gly
   1 5 10 15
<210> 280
   <211> 15
   <212> PRT
   <213> Artificial Sequence
<220>
   <223> CPP
<400> 280
   Ala Leu Trp Met Arg Trp Tyr Ser Pro Thr Thr Arg Arg Tyr Gly
   1 5 10 15
<210> 281
   <211> 15
   <212> PRT
   <213> Artificial Sequence
<220>
   <223> CPP
<400> 281
   Arg Ala Trp Met Arg Trp Tyr Ser Pro Thr Thr Arg Arg Tyr Gly
   1 5 10 15
<210> 282
   <211> 15
   <212> PRT
   <213> Artificial Sequence
<220>
   <223> CPP
<400> 282
   Arg Leu Ala Met Arg Trp Tyr Ser Pro Thr Thr Arg Arg Tyr Gly
   1               5                   10                  15
<210> 283
   <211> 15
   <212> PRT
   <213> Artificial Sequence
<220>
   <223> CPP
<400> 283
   Arg Leu Trp Ala Arg Trp Tyr Ser Pro Thr Thr Arg Arg Tyr Gly
   1               5                   10                  15
<210> 284
   <211> 15
   <212> PRT
   <213> Artificial Sequence
<220>
   <223> CPP
<400> 284
   Arg Leu Trp Met Ala Trp Tyr Ser Pro Thr Thr Arg Arg Tyr Gly
   1               5                   10                  15
<210> 285
   <211> 15
   <212> PRT
   <213> Artificial Sequence
<220>
   <223> CPP
<400> 285
   Arg Leu Trp Met Arg Ala Tyr Ser Pro Thr Thr Arg Arg Tyr Gly
   1               5                   10                  15
<210> 286
   <211> 15
   <212> PRT
   <213> Artificial Sequence
<220>
   <223> CPP
<400> 286
   Arg Leu Trp Met Arg Trp Ala Ser Pro Thr Thr Arg Arg Tyr Gly
   1 5 10 15
<210> 287
   <211> 15
   <212> PRT
   <213> Artificial Sequence
<220>
   <223> CPP
<400> 287
   Arg Leu Trp Met Arg Trp Tyr Ala Pro Thr Thr Arg Arg Tyr Gly
   1 5 10 15
<210> 288
   <211> 15
   <212> PRT
   <213> Artificial Sequence
<220>
   <223> CPP
<400> 288
   Arg Leu Trp Met Arg Trp Tyr Ser Pro Ala Thr Arg Arg Tyr Gly
   1 5 10 15
<210> 289
   <211> 15
   <212> PRT
   <213> Artificial Sequence
<220>
   <223> CPP
<400> 289
   Arg Leu Trp Met Arg Trp Tyr Ser Pro Thr Ala Arg Arg Tyr Gly
   1 5 10 15
<210> 290
   <211> 15
   <212> PRT
   <213> Artificial Sequence
<220>
   <223> CPP
<400> 290
   Arg Leu Trp Met Arg Trp Tyr Ser Pro Thr Thr Ala Arg Tyr Gly
   1 5 10 15
<210> 291
   <211> 15
   <212> PRT
   <213> Artificial Sequence
<220>
   <223> CPP
<400> 291
   Arg Leu Trp Met Arg Trp Tyr Ser Pro Thr Thr Arg Ala Tyr Gly
   1               5                   10                  15
<210> 292
   <211> 15
   <212> PRT
   <213> Artificial Sequence
<220>
   <223> CPP
<400> 292
   Arg Leu Trp Met Arg Trp Tyr Ser Pro Thr Thr Arg Arg Ala Gly
   1               5                   10                  15
<210> 293
   <211> 15
   <212> PRT
   <213> Artificial Sequence
<220>
   <223> CPP
<400> 293
   Arg Leu Trp Met Arg Trp Tyr Ser Pro Thr Thr Arg Arg Tyr Ala
   1               5                   10                  15
<210> 294
   <211> 15
   <212> PRT
   <213> Artificial Sequence
<220>
   <223> CPP
<400> 294
   Arg Leu Leu Met Arg Leu Tyr Ser Pro Thr Thr Arg Arg Tyr Gly
   1               5                   10                  15
<210> 295
   <211> 15
   <212> PRT
   <213> Artificial Sequence
<220>
   <223> CPP
<400> 295
   Arg Leu Phe Met Arg Phe Tyr Ser Pro Thr Thr Arg Arg Tyr Gly
   1 5 10 15
<210> 296
   <211> 15
   <212> PRT
   <213> Artificial Sequence
<220>
   <223> CPP
<400> 296
   Arg Leu Ile Met Arg Ile Tyr Ser Pro Thr Thr Arg Arg Tyr Gly
   1 5 10 15
<210> 297
   <211> 15
   <212> PRT
   <213> Artificial Sequence
<220>
   <223> CPP
<400> 297
   Arg Leu Val Met Arg Val Tyr Ser Pro Thr Thr Arg Arg Tyr Gly
   1 5 10 15
<210> 298
   <211> 15
   <212> PRT
   <213> Artificial Sequence
<220>
   <223> CPP
<400> 298
   Arg Leu Tyr Met Arg Tyr Tyr Ser Pro Thr Thr Arg Arg Tyr Gly
   1 5 10 15
<210> 299
   <211> 12
   <212> PRT
   <213> Artificial Sequence
<220>
   <223> CPP
<400> 299
   Tyr Gly Arg Lys Lys Lys Arg Arg Gln Arg Arg Arg
   1 5 10
<210> 300
   <211> 18
   <212> PRT
   <213> Artificial Sequence
<220>
   <223> CPP
<400> 300
   Ala Leu Ile Ile Leu Arg Arg Arg Ile Arg Lys Gln Ala His Ala His
   1               5                   10                  15
   Ser Lys
<210> 301
   <211> 18
   <212> PRT
   <213> Artificial Sequence
<220>
   <223> CPP
<400> 301
   Leu Ala Ile Ile Leu Arg Arg Arg Ile Arg Lys Gln Ala His Ala His
   1               5                   10                  15
   Ser Lys
<210> 302
   <211> 18
   <212> PRT
   <213> Artificial Sequence
<220>
   <223> CPP
<400> 302
   Leu Leu Ala Ile Leu Arg Arg Arg Ile Arg Lys Gln Ala His Ala His
   1               5                   10                  15
   Ser Lys
<210> 303
   <211> 18
   <212> PRT
   <213> Artificial Sequence
<220>
   <223> CPP
<400> 303
   Leu Leu Ile Ala Leu Arg Arg Arg Ile Arg Lys Gln Ala His Ala His
   1               5                   10                  15
   Ser Lys
<210> 304
   <211> 18
   <212> PRT
   <213> Artificial Sequence
<220>
   <223> CPP
<400> 304
   Leu Leu Ile Ile Ala Arg Arg Arg Ile Arg Lys Gln Ala His Ala His
   1               5                   10                  15
   Ser Lys
<210> 305
   <211> 18
   <212> PRT
   <213> Artificial Sequence
<220>
   <223> CPP
<400> 305
   Leu Leu Ile Ile Leu Ala Arg Arg Ile Arg Lys Gln Ala His Ala His
   1               5                   10                  15
   Ser Lys
<210> 306
   <211> 18
   <212> PRT
   <213> Artificial Sequence
<220>
   <223> CPP
<400> 306
   Leu Leu Ile Ile Leu Arg Ala Arg Ile Arg Lys Gln Ala His Ala His
   1               5                   10                  15
   Ser Lys
<210> 307
   <211> 18
   <212> PRT
   <213> Artificial Sequence
<220>
   <223> CPP
<400> 307
   Leu Leu Ile Ile Leu Arg Arg Ala Ile Arg Lys Gln Ala His Ala His
   1 5 10 15
   Ser Lys
<210> 308
   <211> 18
   <212> PRT
   <213> Artificial Sequence
<220>
   <223> CPP
<400> 308
   Leu Leu Ile Ile Leu Arg Arg Arg Ala Arg Lys Gln Ala His Ala His
   1 5 10 15
   Ser Lys
<210> 309
   <211> 19
   <212> PRT
   <213> Artificial Sequence
<220>
   <223> CPP
<400> 309
   Leu Leu Ile Ile Leu Arg Arg Arg Ile Ala Arg Lys Gln Ala His Ala
   1 5 10 15
   His Ser Lys
<210> 310
   <211> 18
   <212> PRT
   <213> Artificial Sequence
<220>
   <223> CPP
<400> 310
   Leu Leu Ile Ile Leu Arg Arg Arg Ile Arg Ala Gln Ala His Ala His
   1 5 10 15
   Ser Lys
<210> 311
   <211> 18
   <212> PRT
   <213> Artificial Sequence
<220>
   <223> CPP
<400> 311
   Leu Leu Ile Ile Leu Arg Arg Arg Ile Arg Lys Ala Ala His Ala His
   1               5                   10                  15
   Ser Lys
<210> 312
   <211> 18
   <212> PRT
   <213> Artificial Sequence
<220>
   <223> CPP
<400> 312
   Leu Leu Ile Ile Leu Arg Arg Arg Ile Arg Lys Gln Ala His Ala His
   1               5                   10                  15
   Ser Lys
<210> 313
   <211> 18
   <212> PRT
   <213> Artificial Sequence
<220>
   <223> CPP
<400> 313
   Leu Leu Ile Ile Leu Arg Arg Arg Ile Arg Lys Gln Ala Ala Ala His
   1               5                   10                  15
   Ser Lys
<210> 314
   <211> 18
   <212> PRT
   <213> Artificial Sequence
<220>
   <223> CPP
<400> 314
   Leu Leu Ile Ile Leu Arg Arg Arg Ile Arg Lys Gln Ala His Ala His
   1 5 10 15
   Ser Lys
<210> 315
   <211> 18
   <212> PRT
   <213> Artificial Sequence
<220>
   <223> CPP
<400> 315
   Leu Leu Ile Ile Leu Arg Arg Arg Ile Arg Lys Gln Ala His Ala Ala
   1 5 10 15
   Ser Lys
<210> 316
   <211> 18
   <212> PRT
   <213> Artificial Sequence
<220>
   <223> CPP
<400> 316
   Leu Leu Ile Ile Leu Arg Arg Arg Ile Arg Lys Gln Ala His Ala His
   1 5 10 15
   Ala Lys
<210> 317
   <211> 18
   <212> PRT
   <213> Artificial Sequence
<220>
   <223> CPP
<400> 317
   Leu Leu Ile Ile Leu Arg Arg Arg Ile Arg Lys Gln Ala His Ala His
   1 5 10 15
   Ser Ala
<210> 318
   <211> 18
   <212> PRT
   <213> Artificial Sequence
<220>
   <223> CPP
<400> 318
   Lys Ser His Ala His Ala Gln Lys Arg Ile Arg Arg Arg Leu Ile Ile
   1               5                   10                  15
   Leu Leu
<210> 319
   <211> 18
   <212> PRT
   <213> Artificial Sequence
<220>
   <223> CPP
<400> 319
   Leu Leu Ile Ile Leu Arg Arg Arg Ile Arg Lys Gln Ala His Ala His
   1               5                   10                  15
   Ser Lys
<210> 320
   <211> 7
   <212> PRT
   <213> Artificial Sequence
<220>
   <223> CPP
<400> 320
   Arg Arg Ile Arg Pro Arg Pro
   1               5
<210> 321
   <211> 15
   <212> PRT
   <213> Artificial Sequence
<220>
   <223> CPP
<400> 321
   Arg Arg Ile Arg Pro Arg Pro Pro Arg Leu Pro Arg Pro Arg Pro
   1               5                   10                  15
<210> 322
   <211> 24
   <212> PRT
   <213> Artificial Sequence
<220>
   <223> CPP
<400> 322
   Arg Arg Ile Arg Pro Arg Pro Pro Arg Leu Pro Arg Pro Arg Pro Arg
   1               5                   10                  15
   Pro Leu Pro Phe Pro Arg Pro Gly
               20
<210> 323
   <211> 17
   <212> PRT
   <213> Artificial Sequence
<220>
   <223> CPP
<400> 323
   Arg Arg Ile Arg Pro Arg Pro Pro Arg Leu Pro Arg Pro Arg Pro Arg
   1               5                   10                  15
   Pro
<210> 324
   <211> 20
   <212> PRT
   <213> Artificial Sequence
<220>
   <223> CPP
<400> 324
   Pro Arg Pro Pro Arg Leu Pro Arg Pro Arg Pro Arg Pro Leu Pro Phe
   1               5                   10                  15
   Pro Arg Pro Gly
               20
<210> 325
   <211> 18
   <212> PRT
   <213> Artificial Sequence
<220>
   <223> CPP
<400> 325
   Pro Pro Arg Leu Pro Arg Pro Arg Pro Arg Pro Leu Pro Phe Pro Arg
   1               5                   10                  15
   Pro Gly
<210> 326
   <211> 16
   <212> PRT
   <213> Artificial Sequence
<220>
   <223> CPP
<400> 326
   Arg Leu Pro Arg Pro Arg Pro Arg Pro Leu Pro Phe Pro Arg Pro Gly
   1               5                   10                  15
<210> 327
   <211> 14
   <212> PRT
   <213> Artificial Sequence
<220>
   <223> CPP
<400> 327
   Pro Arg Pro Arg Pro Arg Pro Leu Pro Phe Pro Arg Pro Gly
   1               5                   10
<210> 328
   <211> 12
   <212> PRT
   <213> Artificial Sequence
<220>
   <223> CPP
<400> 328
   Pro Arg Pro Arg Pro Leu Pro Phe Pro Arg Pro Gly
   1               5                   10
<210> 329
   <211> 10
   <212> PRT
   <213> Artificial Sequence
<220>
   <223> CPP
<400> 329
   Pro Arg Pro Leu Pro Phe Pro Arg Pro Gly
   1               5                   10
<210> 330
   <211> 22
   <212> PRT
   <213> Artificial Sequence
<220>
   <223> CPP
<400> 330
   Arg Val Thr Ser Trp Leu Gly Arg Gln Leu Arg Ile Ala Gly Lys Arg
   1               5                   10                  15
   Leu Glu Gly Arg Ser Lys
               20
<210> 331
   <211> 16
   <212> PRT
   <213> Artificial Sequence
<220>
   <223> CPP
<400> 331
   Gly Arg Gln Leu Arg Ile Ala Gly Lys Arg Leu Glu Gly Arg Ser Lys
   1               5                   10                  15
<210> 332
   <211> 23
   <212> PRT
   <213> Artificial Sequence
<220>
   <223> CPP
<400> 332
   Arg Arg Val Thr Ser Trp Leu Gly Arg Gln Leu Arg Ile Ala Gly Lys
   1               5                   10                  15
   Arg Leu Glu Gly Arg Ser Lys
               20
<210> 333
   <211> 22
   <212> PRT
   <213> Artificial Sequence
<220>
   <223> CPP
<400> 333
   Arg Val Arg Ser Trp Leu Gly Arg Gln Leu Arg Ile Ala Gly Lys Arg
   1               5                   10                  15
   Leu Glu Gly Arg Ser Lys
   20
<210> 334
   <211> 16
   <212> PRT
   <213> Artificial Sequence
<220>
   <223> CPP
<400> 334
   Gly Arg Gln Leu Arg Ile Ala Gly Lys Arg Leu Arg Gly Arg Ser Lys
   1 5 10 15
<210> 335
   <211> 16
   <212> PRT
   <213> Artificial Sequence
<220>
   <223> CPP
<400> 335
   Gly Arg Gln Leu Arg Ile Ala Gly Arg Arg Leu Arg Gly Arg Ser Arg
   1 5 10 15
<210> 336
   <211> 16
   <212> PRT
   <213> Artificial Sequence
<220>
   <223> CPP
<400> 336
   Gly Arg Gln Leu Arg Arg Ala Gly Arg Arg Leu Arg Gly Arg Ser Arg
   1 5 10 15
<210> 337
   <211> 16
   <212> PRT
   <213> Artificial Sequence
<220>
   <223> CPP
<400> 337
   Gly Arg Gln Leu Arg Ile Ala Gly Arg Arg Leu Arg Arg Arg Ser Arg
   1 5 10 15
<210> 338
   <211> 16
   <212> PRT
   <213> Artificial Sequence
<220>
   <223> CPP
<400> 338
   Gly Arg Gln Leu Arg Arg Ala Gly Arg Arg Leu Arg Arg Arg Ser Arg
   1               5                   10                  15
<210> 339
   <211> 15
   <212> PRT
   <213> Artificial Sequence
<220>
   <223> CPP
<400> 339
   Arg Gln Leu Arg Ile Ala Gly Arg Arg Leu Arg Gly Arg Ser Arg
   1               5                   10                  15
<210> 340
   <211> 16
   <212> PRT
   <213> Artificial Sequence
<220>
   <223> CPP
<400> 340
   Arg Ser Arg Gly Arg Leu Arg Arg Gly Ala Ile Arg Leu Gln Arg Gly
   1               5                   10                  15
<210> 341
   <211> 29
   <212> PRT
   <213> Artificial Sequence
<220>
   <223> CPP
<400> 341
   Lys Leu Ile Lys Gly Arg Thr Pro Ile Lys Phe Gly Lys Ala Asp Cys
   1               5                   10                  15
   Asp Arg Pro Pro Lys His Ser Gln Asn Gly Met Gly Lys
               20                  25
<210> 342
   <211> 27
   <212> PRT
   <213> Artificial Sequence
<220>
   <223> CPP
<400> 342
   Lys Leu Ile Lys Gly Arg Thr Pro Ile Lys Phe Gly Lys Ala Asp Cys
   1               5                   10                  15
   Asp Arg Pro Pro Lys His Ser Gln Asn Gly Met
               20                  25
<210> 343
   <211> 27
   <212> PRT
   <213> Artificial Sequence
<220>
   <223> CPP
<400> 343
   Lys Leu Ile Lys Gly Arg Thr Pro Ile Lys Phe Gly Lys Ala Asp Cys
   1               5                   10                  15
   Asp Arg Pro Pro Lys His Ser Gln Asn Gly Lys
               20                  25
<210> 344
   <211> 26
   <212> PRT
   <213> Artificial Sequence
<220>
   <223> CPP
<400> 344
   Lys Gly Arg Thr Pro Ile Lys Phe Gly Lys Ala Asp Cys Asp Arg Pro
   1               5                   10                  15
   Pro Lys His Ser Gln Asn Gly Met Gly Lys
               20                  25
<210> 345
   <211> 25
   <212> PRT
   <213> Artificial Sequence
<220>
   <223> CPP
<400> 345
   Lys Leu Ile Lys Gly Arg Thr Pro Ile Lys Phe Gly Lys Ala Asp Cys
   1               5                   10                  15
   Asp Arg Pro Pro Lys His Ser Gly Lys
                   20                  25
<210> 346
   <211> 25
   <212> PRT
   <213> Artificial Sequence
<220>
   <223> CPP
<400> 346
   Lys Leu Ile Lys Gly Arg Thr Pro Ile Lys Phe Gly Lys Ala Arg Cys
   1               5                   10                  15
   Arg Arg Pro Pro Lys His Ser Gly Lys
               20                  25
<210> 347
   <211> 13
   <212> PRT
   <213> Artificial Sequence
<220>
   <223> CPP
<400> 347
   Lys Leu Ile Lys Gly Arg Thr Pro Ile Lys Phe Gly Lys
   1               5                   10
<210> 348
   <211> 37
   <212> PRT
   <213> Artificial Sequence
<220>
   <223> CPP
<400> 348
   Lys Arg Ile Pro Asn Lys Lys Pro Gly Lys Lys Thr Thr Thr Lys Pro
   1               5                   10                  15
   Thr Lys Lys Pro Thr Ile Lys Thr Thr Lys Lys Asp Leu Lys Pro Gln
               20                  25                  30
   Thr Thr Lys Pro Lys
           35
<210> 349
   <211> 30
   <212> PRT
   <213> Artificial Sequence
<220>
   <223> CPP
<400> 349
   Lys Arg Ile Pro Asn Lys Lys Pro Gly Lys Lys Thr Thr Thr Lys Pro
   1               5                   10                  15
   Thr Lys Lys Pro Thr Ile Lys Thr Thr Lys Lys Asp Leu Lys
               20                  25                  30
<210> 350
   <211> 27
   <212> PRT
   <213> Artificial Sequence
<220>
   <223> CPP
<400> 350
   Lys Arg Ile Pro Asn Lys Lys Pro Gly Lys Lys Thr Thr Thr Lys Pro
   1               5                   10                  15
   Thr Lys Lys Pro Thr Ile Lys Thr Thr Lys Lys
               20                  25
<210> 351
   <211> 23
   <212> PRT
   <213> Artificial Sequence
<220>
   <223> CPP
<400> 351
   Lys Arg Ile Pro Asn Lys Lys Pro Gly Lys Lys Thr Thr Thr Lys Pro
   1 5 10 15
   Thr Lys Lys Pro Thr Ile Lys
   20
<210> 352
   <211> 19
   <212> PRT
   <213> Artificial Sequence
<220>
   <223> CPP
<400> 352
   Lys Arg Ile Pro Asn Lys Lys Pro Gly Lys Lys Thr Thr Thr Lys Pro
   1 5 10 15
   Thr Lys Lys
<210> 353
   <211> 12
   <212> PRT
   <213> Artificial Sequence
<220>
   <223> CPP
<400> 353
   Lys Arg Ile Pro Asn Lys Lys Pro Gly Lys Lys Thr
   1 5 10
<210> 354
   <211> 11
   <212> PRT
   <213> Artificial Sequence
<220>
   <223> CPP
<400> 354
   Lys Arg Ile Pro Asn Lys Lys Pro Gly Lys Lys
   1 5 10
<210> 355
   <211> 10
   <212> PRT
   <213> Artificial Sequence
<220>
   <223> CPP
<400> 355
   Lys Arg Ile Pro Asn Lys Lys Pro Lys Lys
   1 5 10
<210> 356
   <211> 22
   <212> PRT
   <213> Artificial Sequence
<220>
   <223> CPP
<400> 356
   Lys Lys Pro Gly Lys Lys Thr Thr Thr Lys Pro Thr Lys Lys Pro Thr
   1 5 10 15
   Ile Lys Thr Thr Lys Lys
   20
<210> 357
   <211> 14
   <212> PRT
   <213> Artificial Sequence
<220>
   <223> CPP
<400> 357
   Lys Lys Pro Gly Lys Lys Thr Thr Thr Lys Pro Thr Lys Lys
   1 5 10
<210> 358
   <211> 10
   <212> PRT
   <213> Artificial Sequence
<220>
   <223> CPP
<400> 358
   Lys Lys Pro Thr Ile Lys Thr Thr Lys Lys
   1 5 10
<210> 359
   <211> 10
   <212> PRT
   <213> Artificial Sequence
<220>
   <223> CPP
<400> 359
   Lys Lys Thr Thr Thr Lys Pro Thr Lys Lys
   1 5 10
<210> 360
   <211> 15
   <212> PRT
   <213> Artificial Sequence
<220>
   <223> CPP
<400> 360
   Lys Ser Ile Cys Lys Thr Ile Pro Ser Asn Lys Pro Lys Lys Lys
   1               5                   10                  15
<210> 361
   <211> 11
   <212> PRT
   <213> Artificial Sequence
<220>
   <223> CPP
<400> 361
   Lys Thr Ile Pro Ser Asn Lys Pro Lys Lys Lys
   1               5                   10
<210> 362
   <211> 12
   <212> PRT
   <213> Artificial Sequence
<220>
   <223> CPP
<400> 362
   Lys Pro Arg Ser Lys Asn Pro Pro Lys Lys Pro Lys
   1               5                   10
<210> 363
   <211> 17
   <212> PRT
   <213> Artificial Sequence
<220>
   <223> CPP
<400> 363
   Asp Arg Asp Asp Arg Asp Asp Arg Asp Asp Arg Asp Asp Arg Asp Asp
   1               5                   10                  15
   Arg
<210> 364
   <211> 14
   <212> PRT
   <213> Artificial Sequence
<220>
   <223> CPP
<400> 364
   Glu Arg Glu Arg Glu Arg Glu Arg Glu Arg Glu Arg Glu Arg
   1               5                   10
<210> 365
   <211> 14
   <212> PRT
   <213> Artificial Sequence
<220>
   <223> CPP
<400> 365
   Trp Arg Trp Arg Trp Arg Trp Arg Trp Arg Trp Arg Trp Arg
   1               5                   10
<210> 366
   <211> 10
   <212> PRT
   <213> Artificial Sequence
<220>
   <223> CPP
<400> 366
   Asp Arg Asp Arg Asp Arg Asp Arg Asp Arg
   1               5                   10
<210> 367
   <211> 27
   <212> PRT
   <213> Artificial Sequence
<220>
   <223> CPP
<400> 367
   Gly Ala Leu Phe Leu Gly Phe Leu Gly Ala Ala Gly Ser Thr Met Gly
   1               5                   10                  15
   Ala Trp Ser Gln Pro Lys Lys Lys Arg Lys Val
               20                  25
<210> 368
   <211> 17
   <212> PRT
   <213> Artificial Sequence
<220>
   <223> CPP
<400> 368
   Asp Arg Arg Arg Arg Gly Ser Arg Pro Ser Gly Ala Glu Arg Arg Arg
   1               5                   10                  15
   Arg
<210> 369
   <211> 11
   <212> PRT
   <213> Artificial Sequence
<220>
   <223> CPP
<400> 369
   Asn Arg Ala Arg Arg Asn Arg Arg Arg Val Arg
   1               5                   10
<210> 370
   <211> 10
   <212> PRT
   <213> Artificial Sequence
<220>
   <223> CPP
<400> 370
   Arg Thr Arg Arg Asn Arg Arg Arg Val Arg
   1               5                   10
<210> 371
   <211> 8
   <212> PRT
   <213> Artificial Sequence
<220>
   <223> CPP
<400> 371
   Arg Asn Arg Ser Arg His Arg Arg
   1               5
<210> 372
   <211> 22
   <212> PRT
   <213> Artificial Sequence
<220>
   <223> CPP
<400> 372
   Met Val Arg Arg Phe Leu Val Thr Leu Arg Ile Arg Arg Ala Cys Gly
   1               5                   10                  15
   Pro Pro Arg Val Arg Val
               20
<210> 373
   <211> 22
   <212> PRT
   <213> Artificial Sequence
<220>
   <223> CPP
<400> 373
   Phe Val Thr Arg Gly Cys Pro Arg Arg Leu Val Ala Arg Leu Ile Arg
   1               5                   10                  15
   Val Met Val Pro Arg Arg
               20
<210> 374
   <211> 13
   <212> PRT
   <213> Artificial Sequence
<220>
   <223> CPP
<400> 374
   Val Arg Arg Phe Leu Val Thr Leu Arg Ile Arg Arg Ala
   1               5                   10
<210> 375
   <211> 13
   <212> PRT
   <213> Artificial Sequence
<220>
   <223> CPP
<400> 375
   Arg Val Arg Ile Leu Ala Arg Phe Leu Arg Thr Arg Val
   1               5                   10
<210> 376
   <211> 13
   <212> PRT
   <213> Artificial Sequence
<220>
   <223> CPP
<400> 376
   Arg Val Arg Val Phe Val Val His Ile Pro Arg Leu Thr
   1               5                   10
<210> 377
   <211> 13
   <212> PRT
   <213> Artificial Sequence
<220>
   <223> CPP
<400> 377
   Val Ile Arg Val His Phe Arg Leu Pro Val Arg Thr Val
   1               5                   10
<210> 378
   <211> 37
   <212> PRT
   <213> Artificial Sequence
<220>
   <223> CPP
<400> 378
   Met Val Arg Arg Phe Leu Val Thr Leu Arg Ile Arg Arg Ala Cys Gly
   1               5                   10                  15
   Pro Pro Arg Val Arg Val Phe Val Val His Ile Pro Arg Leu Thr Gly
               20                  25                  30
   Glu Trp Ala Ala Pro
           35
<210> 379
   <211> 37
   <212> PRT
   <213> Artificial Sequence
<220>
   <223> CPP
<400> 379
   Phe Arg Val Pro Leu Arg Ile Arg Pro Cys Val Val Ala Pro Arg Leu
   1               5                   10                  15
   Val Met Val Arg His Thr Phe Gly Arg Ile Ala Arg Trp Val Ala Gly
               20                  25                  30
   Pro Leu Glu Thr Arg
           35
<210> 380
   <211> 21
   <212> PRT
   <213> Artificial Sequence
<220>
   <223> CPP
<400> 380
   Ala Gly Tyr Leu Leu Gly Lys Ile Asn Leu Lys Ala Leu Ala Ala Leu
   1               5                   10                  15
   Ala Lys Lys Ile Leu
               20
<210> 381
   <211> 25
   <212> PRT
   <213> Artificial Sequence
<220>
   <223> CPP
<400> 381
   Gly Thr Lys Met Ile Phe Val Gly Ile Lys Lys Lys Glu Glu Arg Ala
   1               5                   10                  15
   Asp Leu Ile Ala Tyr Leu Lys Lys Ala
               20                  25
<210> 382
   <211> 16
   <212> PRT
   <213> Artificial Sequence
<220>
   <223> CPP
<400> 382
   Lys Lys Lys Glu Glu Arg Ala Asp Leu Ile Ala Tyr Leu Lys Lys Ala
   1               5                   10                  15
<210> 383
   <211> 14
   <212> PRT
   <213> Artificial Sequence
<220>
   <223> CPP
<400> 383
   Lys Met Ile Phe Val Gly Ile Lys Lys Lys Glu Glu Arg Ala
   1               5                   10
<210> 384
   <211> 10
   <212> PRT
   <213> Artificial Sequence
<220>
   <223> CPP
<400> 384
   Lys Met Ile Phe Val Gly Ile Lys Lys Lys
   1               5                   10
<210> 385
   <211> 10
   <212> PRT
   <213> Artificial Sequence
<220>
   <223> CPP
<400> 385
   Glu Lys Gly Lys Lys Ile Phe Ile Met Lys
   1               5                   10
<210> 386
   <211> 9
   <212> PRT
   <213> Artificial Sequence
<220>
   <223> CPP
<400> 386
   Lys Gly Lys Lys Ile Phe Ile Met Lys
   1               5
<210> 387
   <211> 17
   <212> PRT
   <213> Artificial Sequence
<220>
   <223> CPP
<400> 387
   Arg Arg Arg Arg Asn Arg Thr Arg Arg Asn Arg Arg Arg Val Arg Gly
   1               5                   10                  15
   Cys
<210> 388
   <211> 15
   <212> PRT
   <213> Artificial Sequence
<220>
   <223> CPP
<400> 388
   Thr Arg Arg Gln Arg Thr Arg Arg Ala Arg Arg Asn Arg Gly Cys
   1               5                   10                  15
<210> 389
   <211> 21
   <212> PRT
   <213> Artificial Sequence
<220>
   <223> CPP
<400> 389
   Lys Met Thr Arg Ala Gln Arg Arg Ala Ala Ala Arg Arg Asn Arg Trp
   1               5                   10                  15
   Thr Ala Arg Gly Cys
               20
<210> 390
   <211> 21
   <212> PRT
   <213> Artificial Sequence
<220>
   <223> CPP
<400> 390
   Lys Leu Thr Arg Ala Gln Arg Arg Ala Ala Ala Arg Lys Asn Lys Arg
   1               5                   10                  15
   Asn Thr Arg Gly Cys
               20
<210> 391
   <211> 19
   <212> PRT
   <213> Artificial Sequence
<220>
   <223> CPP
<400> 391
   Asn Ala Lys Thr Arg Arg His Glu Arg Arg Arg Lys Leu Ala Ile Glu
   1               5                   10                  15
   Arg Gly Cys
<210> 392
   <211> 24
   <212> PRT
   <213> Artificial Sequence
<220>
   <223> CPP
<400> 392
   Met Asp Ala Gln Thr Arg Arg Arg Glu Arg Arg Ala Glu Lys Gln Ala
   1               5                   10                  15
   Gln Trp Lys Ala Ala Asn Gly Cys
               20
<210> 393
   <211> 20
   <212> PRT
   <213> Artificial Sequence
<220>
   <223> CPP
<400> 393
   Thr Ala Lys Thr Arg Tyr Lys Ala Arg Arg Ala Glu Leu Ile Ala Glu
   1               5                   10                  15
   Arg Arg Gly Cys
               20
<210> 394
   <211> 14
   <212> PRT
   <213> Artificial Sequence
<220>
   <223> CPP
<400> 394
   Ser Gln Met Thr Arg Gln Ala Arg Arg Leu Tyr Asx Gly Cys
   1 5 10
<210> 395
   <211> 28
   <212> PRT
   <213> Artificial Sequence
<220>
   <223> CPP
<400> 395
   Lys Arg Arg Ile Arg Arg Glu Arg Asn Lys Met Ala Ala Ala Lys Ser
   1 5 10 15
   Arg Asn Arg Arg Arg Glu Leu Thr Asp Thr Gly Cys
   20 25
<210> 396
   <211> 30
   <212> PRT
   <213> Artificial Sequence
<220>
   <223> CPP
<400> 396
   Arg Ile Lys Ala Glu Arg Lys Arg Met Arg Asn Arg Ile Ala Ala Ser
   1 5 10 15
   Lys Ser Arg Lys Arg Lys Leu Glu Arg Ile Ala Arg Gly Cys
   20 25 30
<210> 397
   <211> 24
   <212> PRT
   <213> Artificial Sequence
<220>
   <223> CPP
<400> 397
   Lys Arg Ala Arg Asn Thr Glu Ala Ala Arg Arg Ser Arg Ala Arg Lys
   1 5 10 15
   Leu Gln Arg Met Lys Gln Gly Cys
   20
<210> 398
   <211> 22
   <212> PRT
   <213> Artificial Sequence
<220>
   <223> CPP
<400> 398
   Lys Cys Phe Gln Trp Gln Arg Asn Met Arg Lys Val Arg Gly Pro Pro
   1               5                   10                  15
   Val Ser Cys Ile Lys Arg
               20
<210> 399
   <211> 19
   <212> PRT
   <213> Artificial Sequence
<220>
   <223> CPP
<400> 399
   Lys Cys Phe Gln Trp Gln Arg Asn Met Arg Lys Val Arg Gly Pro Pro
   1               5                   10                  15
   Val Ser Cys
<210> 400
   <211> 22
   <212> PRT
   <213> Artificial Sequence
<220>
   <223> CPP
<400> 400
   Lys Cys Phe Gln Trp Gln Arg Asn Met Arg Lys Val Arg Gly Pro Pro
   1               5                   10                  15
   Val Ser Ser Ile Lys Arg
               20
<210> 401
   <211> 13
   <212> PRT
   <213> Artificial Sequence
<220>
   <223> CPP
<400> 401
   Lys Cys Phe Gln Trp Gln Arg Asn Met Arg Lys Val Arg
   1               5                   10
<210> 402
   <211> 16
   <212> PRT
   <213> Artificial Sequence
<220>
   <223> CPP
<400> 402
   Phe Gln Trp Gln Arg Asn Met Arg Lys Val Arg Gly Pro Pro Val Ser
   1               5                   10                  15
<210> 403
   <211> 19
   <212> PRT
   <213> Artificial Sequence
<220>
   <223> CPP
<400> 403
   Gln Trp Gln Arg Asn Met Arg Lys Val Arg Gly Pro Pro Val Ser Cys
   1               5                   10                  15
   Ile Lys Arg
<210> 404
   <211> 10
   <212> PRT
   <213> Artificial Sequence
<220>
   <223> CPP
<400> 404
   Gln Trp Gln Arg Asn Met Arg Lys Val Arg
   1               5                   10
<210> 405
   <211> 22
   <212> PRT
   <213> Artificial Sequence
<220>
   <223> CPP
<400> 405
   Lys Cys Phe Met Trp Gln Glu Met Leu Asn Lys Ala Gly Val Pro Lys
   1               5                   10                  15
   Leu Arg Cys Ala Arg Lys
               20
<210> 406
   <211> 15
   <212> PRT
   <213> Artificial Sequence
<220>
   <223> CPP
<400> 406
   Lys Trp Phe Glu Thr Trp Phe Thr Glu Trp Pro Lys Lys Arg Lys
   1               5                   10                  15
<210> 407
   <211> 20
   <212> PRT
   <213> Artificial Sequence
<220>
   <223> CPP
<400> 407
   Gly Leu Trp Arg Ala Leu Trp Arg Leu Leu Arg Ser Leu Trp Arg Leu
   1               5                   10                  15
   Leu Trp Arg Ala
               20
<210> 408
   <211> 20
   <212> PRT
   <213> Artificial Sequence
<220>
   <223> CPP
<400> 408
   Gly Leu Trp Trp Arg Leu Trp Trp Arg Leu Arg Ser Trp Phe Arg Leu
   1               5                   10                  15
   Trp Phe Arg Ala
               20
<210> 409
   <211> 34
   <212> PRT
   <213> Artificial Sequence
<220>
   <223> CPP
<400> 409
   Asp Ala Ala Thr Ala Thr Arg Gly Arg Ser Ala Ala Ser Arg Pro Thr
   1               5                   10                  15
   Gln Arg Pro Arg Ala Pro Ala Arg Ser Ala Ser Arg Pro Arg Arg Pro
               20                  25                  30
   Val Glu
<210> 410
   <211> 27
   <212> PRT
   <213> Artificial Sequence
<220>
   <223> CPP
<400> 410
   Gly Ala Leu Phe Leu Gly Phe Leu Gly Ala Ala Gly Ser Thr Met Gly
   1               5                   10                  15
   Ala Trp Ser Gln Pro Lys Lys Lys Arg Lys Val
               20                  25
<210> 411
   <211> 27
   <212> PRT
   <213> Artificial Sequence
<220>
   <223> CPP
<400> 411
   Gly Ala Leu Phe Leu Gly Phe Leu Gly Ala Ala Gly Ser Thr Met Gly
   1               5                   10                  15
   Ala Trp Ser Gln Pro Lys Ser Lys Arg Lys Val
               20                  25
<210> 412
   <211> 34
   <212> PRT
   <213> Artificial Sequence
<220>
   <223> CPP
<400> 412
   Ala Lys Val Lys Asp Glu Pro Gln Arg Arg Ser Ala Arg Leu Ser Ala
   1               5                   10                  15
   Lys Pro Ala Pro Pro Lys Pro Glu Pro Lys Pro Lys Lys Ala Pro Ala
               20                  25                  30
   Lys Lys
<210> 413
   <211> 34
   <212> PRT
   <213> Artificial Sequence
<220>
   <223> CPP
<400> 413
   Ala Lys Val Lys Asp Glu Pro Gln Arg Arg Ser Ala Arg Leu Ser Ala
   1               5                   10                  15
   Lys Pro Ala Pro Pro Lys Pro Glu Pro Lys Pro Lys Lys Ala Pro Ala
               20                  25                  30
   Lys Lys
<210> 414
   <211> 12
   <212> PRT
   <213> Artificial Sequence
<220>
   <223> CPP
<400> 414
   Pro Ser Ser Ser Ser Ser Ser Arg Ile Gly Asp Pro
   1               5                   10
<210> 415
   <211> 18
   <212> PRT
   <213> Artificial Sequence
<220>
   <223> CPP
<400> 415
   Val Arg Leu Pro Pro Pro Val Arg Leu Pro Pro Pro Val Arg Leu Pro
   1               5                   10                  15
   Pro Pro
<210> 416
   <211> 18
   <212> PRT
   <213> Artificial Sequence
<220>
   <223> CPP
<400> 416
   Val Glu Leu Pro Pro Pro Val Glu Leu Pro Pro Pro Val Glu Leu Pro
   1               5                   10                  15
   Pro Pro
<210> 417
   <211> 28
   <212> PRT
   <213> Artificial Sequence
<220>
   <223> CPP
<400> 417
   Ala Leu Trp Met Thr Leu Leu Lys Lys Val Leu Lys Ala Ala Ala Lys
   1               5                   10                  15
   Ala Ala Leu Asn Ala Val Leu Val Gly Ala Asn Ala
               20                  25
<210> 418
   <211> 13
   <212> PRT
   <213> Artificial Sequence
<220>
   <223> CPP
<400> 418
   Ala Leu Trp Lys Thr Leu Leu Lys Lys Val Leu Lys Ala
   1               5                   10
<210> 419
   <211> 20
   <212> PRT
   <213> Artificial Sequence
<220>
   <223> CPP
<400> 419
   Ala Leu Trp Lys Thr Leu Leu Lys Lys Val Leu Lys Ala Pro Lys Lys
   1               5                   10                  15
   Lys Arg Lys Val
               20
<210> 420
   <211> 20
   <212> PRT
   <213> Artificial Sequence
<220>
   <223> CPP
<400> 420
   Pro Lys Lys Lys Arg Lys Val Ala Leu Trp Lys Thr Leu Leu Lys Lys
   1               5                   10                  15
   Val Leu Lys Ala
               20
<210> 421
   <211> 20
   <212> PRT
   <213> Artificial Sequence
<220>
   <223> CPP
<400> 421
   Val Lys Arg Lys Lys Lys Pro Ala Leu Trp Lys Thr Leu Leu Lys Lys
   1               5                   10                  15
   Val Leu Lys Ala
   20
<210> 422
   <211> 22
   <212> PRT
   <213> Artificial Sequence
<220>
   <223> CPP
<400> 422
   Arg Gln Ala Arg Arg Asn Arg Arg Arg Ala Leu Trp Lys Thr Leu Leu
   1 5 10 15
   Lys Lys Val Leu Lys Ala
   20
<210> 423
   <211> 10
   <212> PRT
   <213> Artificial Sequence
<220>
   <223> CPP
<400> 423
   Arg Gln Ala Arg Arg Asn Arg Arg Arg Cys
   1 5 10
<210> 424
   <211> 13
   <212> PRT
   <213> Artificial Sequence
<220>
   <223> CPP
<400> 424
   Glu Glu Glu Ala Ala Gly Arg Lys Arg Lys Lys Arg Thr
   1 5 10
<210> 425
   <211> 4
   <212> PRT
   <213> Artificial Sequence
<220>
   <223> CPP with amino acid sequence EEE
<400> 425
   Glu Glu Glu Glu
   1
<210> 426
   <211> 5
   <212> PRT
   <213> Artificial Sequence
<220>
   <223> CPP
<400> 426
   Glu Glu Glu Ala Ala
   1 5
<210> 427
   <211> 8
   <212> PRT
   <213> Artificial Sequence
<220>
   <223> CPP
<400> 427
   Glu Glu Glu Ala Ala Lys Lys Lys
   1               5
<210> 428
   <211> 13
   <212> PRT
   <213> Artificial Sequence
<220>
   <223> CPP
<400> 428
   Phe Phe Phe Ala Ala Gly Arg Lys Arg Lys Lys Arg Thr
   1               5                   10
<210> 429
   <211> 10
   <212> PRT
   <213> Artificial Sequence
<220>
   <223> CPP
<400> 429
   Ala Ala Gly Arg Lys Arg Lys Lys Arg Thr
   1               5                   10
<210> 430
   <211> 13
   <212> PRT
   <213> Artificial Sequence
<220>
   <223> CPP
<400> 430
   Tyr Tyr Tyr Ala Ala Gly Arg Lys Arg Lys Lys Arg Thr
   1               5                   10
<210> 431
   <211> 22
   <212> PRT
   <213> Artificial Sequence
<220>
   <223> CPP
<400> 431
   Met Val Thr Val Leu Phe Arg Arg Leu Arg Ile Arg Arg Ala Cys Gly
   1               5                   10                  15
   Pro Pro Arg Val Arg Val
               20
<210> 432
   <211> 21
   <212> PRT
   <213> Artificial Sequence
<220>
   <223> CPP
<400> 432
   Ala Gly Tyr Leu Leu Gly Lys Ile Asn Leu Lys Ala Leu Ala Ala Leu
   1               5                   10                  15
   Ala Lys Lys Ile Leu
               20
<210> 433
   <211> 11
   <212> PRT
   <213> Artificial Sequence
<220>
   <223> CPP
<400> 433
   Gly Lys Lys Lys Lys Arg Lys Arg Glu Lys Leu
   1               5                   10
<210> 434
   <211> 8
   <212> PRT
   <213> Artificial Sequence
<220>
   <223> CPP
<400> 434
   Glu Arg Lys Lys Arg Arg Arg Glu
   1               5
<210> 435
   <211> 7
   <212> PRT
   <213> Artificial Sequence
<220>
   <223> CPP
<400> 435
   Phe Lys Lys Phe Arg Lys Phe
   1 5
<210> 436
   <211> 21
   <212> PRT
   <213> Artificial Sequence
<220>
   <223> CPP
<400> 436
   Tyr Thr Gln Asp Phe Asn Lys Phe His Thr Phe Pro Gln Thr Ala Ile
   1 5 10 15
   Gly Val Gly Ala Pro
   20
<210> 437
   <211> 18
   <212> PRT
   <213> Artificial Sequence
<220>
   <223> CPP
<400> 437
   Asp Phe Asn Lys Phe His Thr Phe Pro Gln Thr Ala Ile Gly Val Gly
   1 5 10 15
   Ala Pro
<210> 438
   <211> 15
   <212> PRT
   <213> Artificial Sequence
<220>
   <223> CPP
<400> 438
   Lys Phe His Thr Phe Pro Gln Thr Ala Ile Gly Val Gly Ala Pro
   1 5 10 15
<210> 439
   <211> 12
   <212> PRT
   <213> Artificial Sequence
<220>
   <223> CPP
<400> 439
   Thr Phe Pro Gln Thr Ala Ile Gly Val Gly Ala Pro
   1 5 10
<210> 440
   <211> 13
   <212> PRT
   <213> Artificial Sequence
<220>
   <223> CPP
<400> 440
   Gly Tyr Gly Arg Lys Lys Arg Arg Gln Arg Arg Arg Gly
   1 5 10
<210> 441
   <211> 15
   <212> PRT
   <213> Artificial Sequence
<220>
   <223> CPP
<400> 441
   Phe Leu Gly Lys Lys Phe Lys Lys Tyr Phe Leu Gln Leu Leu Lys
   1 5 10 15
<210> 442
   <211> 23
   <212> PRT
   <213> Artificial Sequence
<220>
   <223> CPP
<400> 442
   Phe Leu Ile Phe Ile Arg Val Ile Cys Ile Val Ile Ala Lys Leu Lys
   1 5 10 15
   Ala Asn Leu Met Cys Lys Thr
   20
<210> 443
   <211> 17
   <212> PRT
   <213> Artificial Sequence
<220>
   <223> CPP
<400> 443
   Lys Lys Ala Ala Gln Ile Arg Ser Gln Val Met Thr His Leu Arg Val
   1 5 10 15
   Ile
<210> 444
   <211> 16
   <212> PRT
   <213> Artificial Sequence
<220>
   <223> CPP
<400> 444
   Tyr Ile Val Leu Arg Arg Arg Arg Lys Arg Val Asn Thr Lys Arg Ser
   1 5 10 15
<210> 445
   <211> 11
   <212> PRT
   <213> Artificial Sequence
<220>
   <223> CPP
<400> 445
   Arg Arg Lys Leu Ser Gln Gln Lys Glu Lys Lys
   1 5 10
<210> 446
   <211> 15
   <212> PRT
   <213> Artificial Sequence
<220>
   <223> CPP
<400> 446
   Val Gln Ala Ile Leu Arg Arg Asn Trp Asn Gln Tyr Lys Ile Gln
   1 5 10 15
<210> 447
   <211> 16
   <212> PRT
   <213> Artificial Sequence
<220>
   <223> CPP
<400> 447
   Lys Thr Val Leu Leu Arg Lys Leu Leu Lys Leu Leu Val Arg Lys Ile
   1               5                   10                  15
<210> 448
   <211> 16
   <212> PRT
   <213> Artificial Sequence
<220>
   <223> CPP
<400> 448
   Leu Leu Lys Lys Arg Lys Val Val Arg Leu Ile Lys Phe Leu Leu Lys
   1               5                   10                  15
<210> 449
   <211> 19
   <212> PRT
   <213> Artificial Sequence
<220>
   <223> CPP
<400> 449
   Lys Leu Pro Cys Arg Ser Asn Thr Phe Leu Asn Ile Phe Arg Arg Lys
   1               5                   10                  15
   Lys Pro Gly
<210> 450
   <211> 16
   <212> PRT
   <213> Artificial Sequence
<220>
   <223> CPP
<400> 450
   Lys Lys Ile Cys Thr Arg Lys Pro Arg Phe Met Ser Ala Trp Ala Gln
   1               5                   10                  15
<210> 451
   <211> 18
   <212> PRT
   <213> Artificial Sequence
<220>
   <223> CPP
<400> 451
   Arg Gly Gly Arg Leu Ser Tyr Ser Arg Arg Arg Phe Ser Thr Ser Thr
   1 5 10 15
   Gly Arg
<210> 452
   <211> 10
   <212> PRT
   <213> Artificial Sequence
<220>
   <223> CPP
<400> 452
   Arg Arg Leu Ser Tyr Ser Arg Arg Arg Phe
   1 5 10
<210> 453
   <211> 17
   <212> PRT
   <213> Artificial Sequence
<220>
   <223> CPP
<400> 453
   Arg Gly Gly Arg Leu Ala Tyr Leu Arg Arg Arg Trp Ala Val Leu Gly
   1 5 10 15
   Arg
<210> 454
   <211> 28
   <212> PRT
   <213> Artificial Sequence
<220>
   <223> CPP
<400> 454
   Met Ala Asn Leu Gly Cys Trp Met Leu Val Leu Phe Val Ala Thr Trp
   1 5 10 15
   Ser Asp Leu Gly Leu Cys Lys Lys Arg Pro Lys Pro
   20 25
<210> 455
   <211> 30
   <212> PRT
   <213> Artificial Sequence
<220>
   <223> CPP
<400> 455
   Met Val Lys Ser Lys Ile Gly Ser Trp Ile Leu Val Leu Phe Val Ala
   1               5                   10                  15
   Met Trp Ser Asp Val Gly Leu Cys Lys Lys Arg Pro Lys Pro
               20                  25                  30
<210> 456
   <211> 38
   <212> PRT
   <213> Artificial Sequence
<220>
   <223> CPP
<400> 456
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser Cys
           35
<210> 457
   <211> 23
   <212> PRT
   <213> Artificial Sequence
<220>
   <223> CPP
<400> 457
   Gly Ile Gly Lys Phe Leu His Ser Ala Lys Lys Trp Gly Lys Ala Phe
   1               5                   10                  15
   Val Gly Gln Ile Met Asn Cys
               20
<210> 458
   <211> 24
   <212> PRT
   <213> Artificial Sequence
<220>
   <223> CPP
<400> 458
   Thr Arg Ser Ser Arg Ala Gly Leu Gln Trp Pro Val Gly Arg Val His
   1               5                   10                  15
   Arg Leu Leu Arg Lys Gly Gly Cys
               20
<210> 459
   <211> 11
   <212> PRT
   <213> Artificial Sequence
<220>
   <223> CPP
<400> 459
   Tyr Gly Arg Lys Lys Arg Arg Gln Arg Arg Arg
   1               5                   10
<210> 460
   <211> 61
   <212> PRT
   <213> Artificial Sequence
<220>
   <223> CPP
<400> 460
   Glu Lys Arg Pro Arg Thr Ala Phe Ser Ser Glu Gln Leu Ala Arg Leu
   1               5                   10                  15
   Lys Arg Glu Phe Asn Glu Asn Arg Tyr Leu Thr Thr Glu Arg Arg Arg
               20                  25                  30
   Gln Gln Leu Ser Ser Glu Leu Gly Leu Asn Glu Ala Gln Ile Lys Ile
           35                  40                  45
   Trp Phe Gln Asn Lys Arg Ala Lys Ile Lys Lys Ser Thr
       50                  55                  60
<210> 461
   <211> 13
   <212> PRT
   <213> Artificial Sequence
<220>
   <223> CPP
<400> 461
   Gly Arg Arg Arg Arg Arg Arg Arg Arg Arg Pro Pro Gln
   1               5                   10
<210> 462
   <211> 27
   <212> PRT
   <213> Artificial Sequence
<220>
   <223> CPP
<400> 462
   Gly Ala Leu Phe Leu Gly Phe Leu Gly Ala Ala Gly Ser Thr Met Gly
   1               5                   10                  15
   Ala Trp Ser Gln Pro Lys Lys Lys Arg Lys Val
               20                  25
<210> 463
   <211> 27
   <212> PRT
   <213> Artificial Sequence
<220>
   <223> CPP
<400> 463
   Gly Ala Leu Phe Leu Ala Phe Leu Ala Ala Ala Leu Ser Leu Met Gly
   1               5                   10                  15
   Leu Trp Ser Gln Pro Lys Lys Lys Arg Arg Val
               20                  25
<210> 464
   <211> 10
   <212> PRT
   <213> Artificial Sequence
<220>
   <223> CPP
<400> 464
   Met Leu Leu Leu Thr Arg Arg Arg Ser Thr
   1               5                   10
<210> 465
   <211> 9
   <212> PRT
   <213> Artificial Sequence
<220>
   <223> CPP
<400> 465
   Cys Gly Asn Lys Arg Thr Arg Gly Cys
   1 5
<210> 466
   <211> 12
   <212> PRT
   <213> Artificial Sequence
<220>
   <223> CPP
<400> 466
   Thr Ser Pro Leu Asn Ile His Asn Gly Gln Lys Leu
   1 5 10
<210> 467
   <211> 16
   <212> PRT
   <213> Artificial Sequence
<220>
   <223> CPP
<400> 467
   Gly Leu Arg Lys Arg Leu Arg Lys Phe Arg Asn Lys Ile Lys Glu Lys
   1 5 10 15
<210> 468
   <211> 27
   <212> PRT
   <213> Artificial Sequence
<220>
   <223> CPP
<400> 468
   Gly Leu Leu Glu Ala Leu Ala Glu Leu Leu Glu Gly Leu Arg Lys Arg
   1 5 10 15
   Leu Arg Lys Phe Arg Asn Lys Ile Lys Glu Lys
   20 25
<210> 469
   <211> 13
   <212> PRT
   <213> Artificial Sequence
<220>
   <223> CPP
<400> 469
   Cys Val Gln Trp Ser Leu Leu Arg Gly Tyr Gln Pro Cys
   1 5 10
<210> 470
   <211> 16
   <212> PRT
   <213> Artificial Sequence
<220>
   <223> CPP
<400> 470
   Arg Gln Ile Lys Ile Phe Phe Gln Asn Arg Arg Met Lys Trp Lys Lys
   1               5                   10                  15
<210> 471
   <211> 21
   <212> PRT
   <213> Artificial Sequence
<220>
   <223> CPP
<400> 471
   Ala Ser Met Trp Glu Arg Val Lys Ser Ile Ile Lys Ser Ser Leu Ala
   1               5                   10                  15
   Ala Ala Ser Asn Ile
               20
<210> 472
   <211> 21
   <212> PRT
   <213> Artificial Sequence
<220>
   <223> CPP
<400> 472
   Ala Ser Met Trp Glu Arg Val Lys Ser Ile Ile Lys Ser Ser Leu Ala
   1               5                   10                  15
   Ala Ala Ser Asn Ile
               20
<210> 473
   <211> 26
   <212> PRT
   <213> Artificial Sequence
<220>
   <223> CPP
<400> 473
   Asp Pro Lys Gly Asp Pro Lys Gly Val Thr Val Thr Val Thr Val Thr
   1 5 10 15
   Val Thr Gly Lys Gly Asp Pro Lys Pro Asp
   20 25
<210> 474
   <211> 16
   <212> PRT
   <213> Artificial Sequence
<220>
   <223> CPP
<400> 474
   Cys Ser Ile Pro Pro Glu Val Lys Phe Asn Pro Phe Val Tyr Leu Ile
   1 5 10 15
<210> 475
   <211> 16
   <212> PRT
   <213> Artificial Sequence
<220>
   <223> CPP
<400> 475
   Cys Ser Ile Pro Pro Glu Val Lys Phe Asn Pro Phe Val Tyr Leu Ile
   1 5 10 15
<210> 476
   <211> 6
   <212> PRT
   <213> Artificial Sequence
<220>
   <223> CPP
<400> 476
   Pro Phe Val Tyr Leu Ile
   1 5
<210> 477
   <211> 8
   <212> PRT
   <213> Artificial Sequence
<220>
   <223> CPP
<400> 477
   Asn Lys Pro Ile Leu Val Phe Tyr
   1 5
<210> 478
   <211> 14
   <212> PRT
   <213> Artificial Sequence
<220>
   <223> CPP
<400> 478
   Tyr Lys Gln Cys His Lys Lys Gly Gly Lys Lys Gly Ser Gly
   1               5                   10
<210> 479
   <211> 15
   <212> PRT
   <213> Artificial Sequence
<220>
   <223> CPP
<220>
   <221> misc_feature
   <222> (10)..(10)
   <223> Xaa can be any naturally occurring amino acid
<400> 479
   Tyr Lys Gln Cys His Lys Lys Gly Gly Xaa Lys Lys Gly Ser Gly
   1               5                   10                  15
<210> 480
   <211> 14
   <212> PRT
   <213> Artificial Sequence
<220>
   <223> CPP
<400> 480
   Gly Ser Gly Lys Lys Gly Gly Lys Lys His Cys Gln Lys Tyr
   1               5                   10
<210> 481
   <211> 14
   <212> PRT
   <213> Artificial Sequence
<220>
   <223> CPP
<400> 481
   Gly Ser Gly Lys Lys Gly Gly Lys Lys Ile Cys Gln Lys Tyr
   1               5                   10
<210> 482
   <211> 16
   <212> PRT
   <213> Artificial Sequence
<220>
   <223> CPP
<400> 482
   Tyr Thr Ala Ile Ala Trp Val Lys Ala Phe Ile Arg Lys Leu Arg Lys
   1 5 10 15
<210> 483
   <211> 17
   <212> PRT
   <213> Artificial Sequence
<220>
   <223> CPP
<400> 483
   Ile Ala Trp Val Lys Ala Phe Ile Arg Lys Leu Arg Lys Gly Pro Leu
   1 5 10 15
   Gly
<210> 484
   <211> 23
   <212> PRT
   <213> Artificial Sequence
<220>
   <223> CPP
<400> 484
   Leu Ile Arg Leu Trp Ser His Leu Ile His Ile Trp Phe Gln Asn Arg
   1 5 10 15
   Arg Leu Lys Trp Lys Lys Lys
   20
<210> 485
   <211> 34
   <212> PRT
   <213> Artificial Sequence
<220>
   <223> CPP
<400> 485
   Lys Lys Lys Lys Lys Lys Gly Gly Phe Leu Gly Phe Trp Arg Gly Glu
   1 5 10 15
   Asn Gly Arg Lys Thr Arg Ser Ala Tyr Glu Arg Met Cys Ile Leu Lys
   20 25 30
   Gly Lys
<210> 486
   <211> 14
   <212> PRT
   <213> Artificial Sequence
<220>
   <223> CPP
<400> 486
   Arg Leu Ser Gly Met Asn Glu Val Leu Ser Phe Arg Trp Leu
   1 5 10
<210> 487
   <211> 13
   <212> PRT
   <213> Artificial Sequence
<220>
   <223> CPP
<400> 487
   Gly Pro Phe His Phe Tyr Gln Phe Leu Phe Pro Pro Val
   1 5 10
<210> 488
   <211> 13
   <212> PRT
   <213> Artificial Sequence
<220>
   <223> CPP
<400> 488
   Gly Ser Pro Trp Gly Leu Gln His His Pro Pro Arg Thr
   1 5 10
<210> 489
   <211> 41
   <212> PRT
   <213> Artificial Sequence
<220>
   <223> CPP
<400> 489
   Ala Ala Val Ala Leu Leu Pro Ala Val Leu Leu Ala Leu Leu Ala Pro
   1 5 10 15
   Glu Ile Leu Leu Pro Asn Asn Tyr Asn Ala Tyr Glu Ser Tyr Lys Tyr
   20 25 30
   Pro Gly Met Phe Ile Ala Leu Ser Lys
   35 40
<210> 490
   <211> 26
   <212> PRT
   <213> Artificial Sequence
<220>
   <223> CPP
<400> 490
   Ala Ala Val Ala Leu Leu Pro Ala Val Leu Leu Ala Leu Leu Ala Pro
   1 5 10 15
   Val Gln Arg Lys Arg Gln Lys Leu Met Pro
   20 25
<210> 491
   <211> 23
   <212> PRT
   <213> Artificial Sequence
<220>
   <223> CPP
<400> 491
   Met Gly Leu Gly Leu His Leu Leu Val Leu Ala Ala Ala Leu Gln Gly
   1 5 10 15
   Ala Lys Lys Lys Arg Lys Val
   20
<210> 492
   <211> 30
   <212> PRT
   <213> Artificial Sequence
<220>
   <223> CPP
<400> 492
   Trp Glu Ala Ala Leu Ala Glu Ala Leu Ala Glu Ala Leu Ala Glu His
   1 5 10 15
   Leu Ala Glu Ala Leu Ala Glu Ala Leu Glu Ala Leu Ala Ala
   20 25 30
<210> 493
   <211> 20
   <212> PRT
   <213> Artificial Sequence
<220>
   <223> CPP
<400> 493
   Gly Leu Phe Glu Ala Leu Leu Glu Leu Leu Glu Ser Leu Trp Glu Leu
   1 5 10 15
   Leu Leu Glu Ala
   20
<210> 494
   <211> 20
   <212> PRT
   <213> Artificial Sequence
<220>
   <223> CPP
<400> 494
   Gly Leu Phe Lys Ala Leu Leu Lys Leu Leu Lys Ser Leu Trp Lys Leu
   1 5 10 15
   Leu Leu Lys Ala
   20
<210> 495
   <211> 20
   <212> PRT
   <213> Artificial Sequence
<220>
   <223> CPP
<400> 495
   Gly Leu Phe Arg Ala Leu Leu Arg Leu Leu Arg Ser Leu Trp Arg Leu
   1 5 10 15
   Leu Leu Arg Ala
   20
<210> 496
   <211> 23
   <212> PRT
   <213> Artificial Sequence
<220>
   <223> CPP
<400> 496
   Cys Gly Ala Tyr Asp Leu Arg Arg Arg Glu Arg Gln Ser Arg Leu Arg
   1               5                   10                  15
   Arg Arg Glu Arg Gln Ser Arg
               20
<210> 497
   <211> 17
   <212> PRT
   <213> Artificial Sequence
<220>
   <223> CPP
<400> 497
   Arg Lys Lys Arg Arg Arg Glu Ser Arg Lys Lys Arg Arg Arg Glu Ser
   1               5                   10                  15
   Cys
<210> 498
   <211> 19
   <212> PRT
   <213> Artificial Sequence
<220>
   <223> CPP
<400> 498
   Cys Val Lys Arg Gly Leu Lys Leu Arg His Val Arg Pro Arg Val Thr
   1               5                   10                  15
   Arg Asp Val
<210> 499
   <211> 17
   <212> PRT
   <213> Artificial Sequence
<220>
   <223> CPP
<400> 499
   Cys Arg Gln Ile Lys Ile Trp Phe Gln Asn Arg Arg Met Lys Trp Lys
   1               5                   10                  15
   Lys
<210> 500
   <211> 15
   <212> PRT
   <213> Artificial Sequence
<220>
   <223> CPP
<400> 500
   Pro Pro Lys Lys Ser Ala Gln Cys Leu Arg Tyr Lys Lys Pro Glu
   1 5 10 15
<210> 501
   <211> 15
   <212> PRT
   <213> Artificial Sequence
<220>
   <223> CPP
<400> 501
   Asp Pro Val Asp Thr Pro Asn Pro Thr Arg Arg Lys Pro Gly Lys
   1 5 10 15
<210> 502
   <211> 13
   <212> PRT
   <213> Artificial Sequence
<220>
   <223> CPP
<400> 502
   Lys Arg Val Ser Arg Asn Lys Ser Glu Lys Lys Arg Arg
   1 5 10
<210> 503
   <211> 16
   <212> PRT
   <213> Artificial Sequence
<220>
   <223> CPP
<400> 503
   Gly Arg Arg His His Cys Arg Ser Lys Ala Lys Arg Ser Arg His His
   1 5 10 15
<210> 504
   <211> 16
   <212> PRT
   <213> Artificial Sequence
<220>
   <223> CPP
<400> 504
   Ser Ala Arg His His Cys Arg Ser Lys Ala Lys Arg Ser Arg His His
   1               5                   10                  15
<210> 505
   <211> 16
   <212> PRT
   <213> Artificial Sequence
<220>
   <223> CPP
<400> 505
   Ser Arg Ala His His Cys Arg Ser Lys Ala Lys Arg Ser Arg His His
   1               5                   10                  15
<210> 506
   <211> 16
   <212> PRT
   <213> Artificial Sequence
<220>
   <223> CPP
<400> 506
   Ser Arg Arg Ala His Cys Arg Ser Lys Ala Lys Arg Ser Arg His His
   1               5                   10                  15
<210> 507
   <211> 16
   <212> PRT
   <213> Artificial Sequence
<220>
   <223> CPP
<400> 507
   Ser Arg Arg His Ala Cys Arg Ser Lys Ala Lys Arg Ser Arg His His
   1               5                   10                  15
<210> 508
   <211> 16
   <212> PRT
   <213> Artificial Sequence
<220>
   <223> CPP
<400> 508
   Ser Arg Arg His His Ala Arg Ser Lys Ala Lys Arg Ser Arg His His
   1               5                   10                  15
<210> 509
   <211> 16
   <212> PRT
   <213> Artificial Sequence
<220>
   <223> CPP
<400> 509
   Ser Arg Arg His His Cys Arg Ala Lys Ala Lys Arg Ser Arg His His
   1               5                   10                  15
<210> 510
   <211> 16
   <212> PRT
   <213> Artificial Sequence
<220>
   <223> CPP
<400> 510
   Ser Arg Arg His His Cys Arg Ser Ala Ala Lys Arg Ser Arg His His
   1               5                   10                  15
<210> 511
   <211> 16
   <212> PRT
   <213> Artificial Sequence
<220>
   <223> CPP
<400> 511
   Ser Arg Arg His His Cys Arg Ser Lys Ala Ala Arg Ser Arg His His
   1               5                   10                  15
<210> 512
   <211> 16
   <212> PRT
   <213> Artificial Sequence
<220>
   <223> CPP
<400> 512
   Ser Arg Arg His His Cys Arg Ser Lys Ala Lys Ala Ser Arg His His
   1               5                   10                  15
<210> 513
   <211> 16
   <212> PRT
   <213> Artificial Sequence
<220>
   <223> CPP
<400> 513
   Ser Arg Arg His His Cys Arg Ser Lys Ala Lys Arg Ala Arg His His
   1               5                   10                  15
<210> 514
   <211> 16
   <212> PRT
   <213> Artificial Sequence
<220>
   <223> CPP
<400> 514
   Ser Arg Arg His His Cys Arg Ser Lys Ala Lys Arg Ser Ala His His
   1               5                   10                  15
<210> 515
   <211> 13
   <212> PRT
   <213> Artificial Sequence
<220>
   <223> CPP
<400> 515
   Arg Arg His His Cys Arg Ser Lys Ala Lys Arg Ser Arg
   1               5                   10
<210> 516
   <211> 12
   <212> PRT
   <213> Artificial Sequence
<220>
   <223> CPP
<400> 516
   Gly Arg Lys Gly Lys His Lys Arg Lys Lys Leu Pro
   1               5                   10
<210> 517
   <211> 10
   <212> PRT
   <213> Artificial Sequence
<220>
   <223> CPP
<400> 517
   Gly Lys Lys Lys Lys Lys Lys Lys Lys Lys
   1 5 10
<210> 518
   <211> 18
   <212> PRT
   <213> Artificial Sequence
<220>
   <223> CPP
<400> 518
   Gly Lys Arg Val Ala Lys Arg Lys Leu Ile Glu Gln Asn Arg Glu Arg
   1 5 10 15
   Arg Arg
<210> 519
   <211> 18
   <212> PRT
   <213> Artificial Sequence
<220>
   <223> CPP
<400> 519
   Gly Arg Lys Leu Lys Lys Lys Lys Asn Glu Lys Glu Asp Lys Arg Pro
   1 5 10 15
   Arg Thr
<210> 520
   <211> 17
   <212> PRT
   <213> Artificial Sequence
<220>
   <223> CPP
<400> 520
   Gly Lys Lys Thr Asn Leu Phe Ser Ala Leu Ile Lys Lys Lys Lys Thr
   1 5 10 15
   Ala
<210> 521
   <211> 18
   <212> PRT
   <213> Artificial Sequence
<220>
   <223> CPP
<400> 521
   Gly Arg Arg Glu Arg Asn Lys Met Ala Ala Ala Lys Cys Arg Asn Arg
   1               5                   10                  15
   Arg Arg
<210> 522
   <211> 18
   <212> PRT
   <213> Artificial Sequence
<220>
   <223> CPP
<400> 522
   Gly Lys Arg Ala Arg Asn Thr Glu Ala Ala Arg Arg Ser Arg Ala Arg
   1               5                   10                  15
   Lys Leu
<210> 523
   <211> 14
   <212> PRT
   <213> Artificial Sequence
<220>
   <223> CPP
<400> 523
   Gly Arg Arg Arg Arg Ala Thr Ala Lys Tyr Arg Thr Ala His
   1               5                   10
<210> 524
   <211> 15
   <212> PRT
   <213> Artificial Sequence
<220>
   <223> CPP
<400> 524
   Gly Lys Arg Arg Arg Arg Ala Thr Ala Lys Tyr Arg Ser Ala His
   1               5                   10                  15
<210> 525
   <211> 12
   <212> PRT
   <213> Artificial Sequence
<220>
   <223> CPP
<400> 525
   Gly Arg Arg Arg Arg Lys Arg Leu Ser His Arg Thr
   1 5 10
<210> 526
   <211> 10
   <212> PRT
   <213> Artificial Sequence
<220>
   <223> CPP
<400> 526
   Gly Arg Arg Arg Arg Arg Glu Arg Asn Lys
   1 5 10
<210> 527
   <211> 16
   <212> PRT
   <213> Artificial Sequence
<220>
   <223> CPP
<400> 527
   Gly Lys His Arg His Glu Arg Gly His His Arg Asp Arg Arg Glu Arg
   1 5 10 15
<210> 528
   <211> 17
   <212> PRT
   <213> Artificial Sequence
<220>
   <223> CPP
<400> 528
   Gly Lys Lys Lys Arg Lys Leu Ser Asn Arg Glu Ser Ala Lys Arg Ser
   1 5 10 15
   Arg
<210> 529
   <211> 10
   <212> PRT
   <213> Artificial Sequence
<220>
   <223> CPP
<400> 529
   Met Ile Ile Tyr Arg Asp Leu Ile Ser His
   1 5 10
<210> 530
   <211> 9
   <212> PRT
   <213> Artificial Sequence
<220>
   <223> CPP
<400> 530
   Met Ile Ile Tyr Arg Asp Leu Ile Ser
   1 5
<210> 531
   <211> 8
   <212> PRT
   <213> Artificial Sequence
<220>
   <223> CPP
<400> 531
   Met Ile Ile Tyr Arg Asp Leu Ile
   1 5
<210> 532
   <211> 9
   <212> PRT
   <213> Artificial Sequence
<220>
   <223> CPP
<400> 532
   Ile Ile Tyr Arg Asp Leu Ile Ser His
   1 5
<210> 533
   <211> 7
   <212> PRT
   <213> Artificial Sequence
<220>
   <223> CPP
<400> 533
   Met Ile Ile Tyr Arg Asp Leu
   1 5
<210> 534
   <211> 6
   <212> PRT
   <213> Artificial Sequence
<220>
   <223> CPP
<400> 534
   Met Ile Ile Tyr Arg Asp
   1 5
<210> 535
   <211> 8
   <212> PRT
   <213> Artificial Sequence
<220>
   <223> CPP
<400> 535
   Ile Tyr Arg Asp Leu Ile Ser His
   1 5
<210> 536
   <211> 9
   <212> PRT
   <213> Artificial Sequence
<220>
   <223> CPP
<400> 536
   Ala Ile Ile Tyr Arg Asp Leu Ile Ser
   1 5
<210> 537
   <211> 9
   <212> PRT
   <213> Artificial Sequence
<220>
   <223> CPP
<400> 537
   Met Ala Ile Tyr Arg Asp Leu Ile Ser
   1 5
<210> 538
   <211> 9
   <212> PRT
   <213> Artificial Sequence
<220>
   <223> CPP
<400> 538
   Met Ile Ala Tyr Arg Asp Leu Ile Ser
   1 5
<210> 539
   <211> 9
   <212> PRT
   <213> Artificial Sequence
<220>
   <223> CPP
<400> 539
   Met Ile Ile Ala Arg Asp Leu Ile Ser
   1 5
<210> 540
   <211> 9
   <212> PRT
   <213> Artificial Sequence
<220>
   <223> CPP
<400> 540
   Met Ile Ile Tyr Ala Asp Leu Ile Ser
   1 5
<210> 541
   <211> 9
   <212> PRT
   <213> Artificial Sequence
<220>
   <223> CPP
<400> 541
   Met Ile Ile Tyr Arg Ala Leu Ile Ser
   1 5
<210> 542
   <211> 9
   <212> PRT
   <213> Artificial Sequence
<220>
   <223> CPP
<400> 542
   Met Ile Ile Tyr Arg Asp Ala Ile Ser
   1 5
<210> 543
   <211> 9
   <212> PRT
   <213> Artificial Sequence
<220>
   <223> CPP
<400> 543
   Met Ile Ile Tyr Arg Asp Leu Ala Ser
   1               5
<210> 544
   <211> 9
   <212> PRT
   <213> Artificial Sequence
<220>
   <223> CPP
<400> 544
   Met Ile Ile Tyr Arg Asp Leu Ile Ala
   1               5
<210> 545
   <211> 11
   <212> PRT
   <213> Artificial Sequence
<220>
   <223> CPP
<400> 545
   Met Ile Ile Tyr Arg Asp Leu Ile Ser Lys Lys
   1               5                   10
<210> 546
   <211> 10
   <212> PRT
   <213> Artificial Sequence
<220>
   <223> CPP
<400> 546
   Met Ile Ile Tyr Arg Asp Lys Lys Ser His
   1               5                   10
<210> 547
   <211> 10
   <212> PRT
   <213> Artificial Sequence
<220>
   <223> CPP
<400> 547
   Met Ile Ile Phe Arg Asp Leu Ile Ser His
   1 5 10
<210> 548
   <211> 10
   <212> PRT
   <213> Artificial Sequence
<220>
   <223> CPP
<400> 548
   Met Ile Ile Ser Arg Asp Leu Ile Ser His
   1 5 10
<210> 549
   <211> 10
   <212> PRT
   <213> Artificial Sequence
<220>
   <223> CPP
<400> 549
   Gln Ile Ile Ser Arg Asp Leu Ile Ser His
   1 5 10
<210> 550
   <211> 10
   <212> PRT
   <213> Artificial Sequence
<220>
   <223> CPP
<400> 550
   Cys Ile Ile Ser Arg Asp Leu Ile Ser His
   1 5 10
<210> 551
   <211> 12
   <212> PRT
   <213> Artificial Sequence
<220>
   <223> CPP
<400> 551
   Met Ile Ile Tyr Arg Ala Leu Ile Ser His Lys Lys
   1 5 10
<210> 552
   <211> 12
   <212> PRT
   <213> Artificial Sequence
<220>
   <223> CPP
<400> 552
   Met Ile Ile Tyr Arg Ile Ala Ala Ser His Lys Lys
   1               5                   10
<210> 553
   <211> 10
   <212> PRT
   <213> Artificial Sequence
<220>
   <223> CPP
<400> 553
   Met Ile Ile Arg Arg Asp Leu Ile Ser Glu
   1               5                   10
<210> 554
   <211> 10
   <212> PRT
   <213> Artificial Sequence
<220>
   <223> CPP
<400> 554
   Met Ile Ile Tyr Arg Ala Glu Ile Ser His
   1               5                   10
<210> 555
   <211> 10
   <212> PRT
   <213> Artificial Sequence
<220>
   <223> CPP
<400> 555
   Met Ile Ile Tyr Ala Arg Arg Ala Glu Glu
   1               5                   10
<210> 556
   <211> 12
   <212> PRT
   <213> Artificial Sequence
<220>
   <223> CPP
<400> 556
   Met Ile Ile Phe Arg Ile Ala Ala Ser His Lys Lys
   1 5 10
<210> 557
   <211> 12
   <212> PRT
   <213> Artificial Sequence
<220>
   <223> CPP
<400> 557
   Met Ile Ile Phe Arg Ala Leu Ile Ser His Lys Lys
   1 5 10
<210> 558
   <211> 12
   <212> PRT
   <213> Artificial Sequence
<220>
   <223> CPP
<400> 558
   Met Ile Ile Phe Arg Ala Ala Ala Ser His Lys Lys
   1 5 10
<210> 559
   <211> 12
   <212> PRT
   <213> Artificial Sequence
<220>
   <223> CPP
<400> 559
   Phe Ile Ile Phe Arg Ile Ala Ala Ser His Lys Lys
   1 5 10
<210> 560
   <211> 12
   <212> PRT
   <213> Artificial Sequence
<220>
   <223> CPP
<400> 560
   Leu Ile Ile Phe Arg Ile Ala Ala Ser His Lys Lys
   1 5 10
<210> 561
   <211> 12
   <212> PRT
   <213> Artificial Sequence
<220>
   <223> CPP
<400> 561
   Trp Ile Ile Phe Arg Ile Ala Ala Ser His Lys Lys
   1               5                   10
<210> 562
   <211> 12
   <212> PRT
   <213> Artificial Sequence
<220>
   <223> CPP
<400> 562
   Trp Ile Ile Phe Arg Ala Ala Ala Ser His Lys Lys
   1               5                   10
<210> 563
   <211> 12
   <212> PRT
   <213> Artificial Sequence
<220>
   <223> CPP
<400> 563
   Trp Ile Ile Phe Arg Ala Leu Ile Ser His Lys Lys
   1               5                   10
<210> 564
   <211> 12
   <212> PRT
   <213> Artificial Sequence
<220>
   <223> CPP
<400> 564
   Met Ile Ile Phe Arg Ile Ala Ala Tyr His Lys Lys
   1               5                   10
<210> 565
   <211> 12
   <212> PRT
   <213> Artificial Sequence
<220>
   <223> CPP
<400> 565
   Trp Ile Ile Phe Arg Ile Ala Ala Tyr His Lys Lys
   1 5 10
<210> 566
   <211> 12
   <212> PRT
   <213> Artificial Sequence
<220>
   <223> CPP
<400> 566
   Met Ile Ile Phe Arg Ile Ala Ala Thr His Lys Lys
   1 5 10
<210> 567
   <211> 12
   <212> PRT
   <213> Artificial Sequence
<220>
   <223> CPP
<400> 567
   Trp Ile Ile Phe Arg Ile Ala Ala Thr His Lys Lys
   1 5 10
<210> 568
   <211> 12
   <212> PRT
   <213> Artificial Sequence
<220>
   <223> CPP
<400> 568
   Met Ile Ile Phe Lys Ile Ala Ala Ser His Lys Lys
   1 5 10
<210> 569
   <211> 12
   <212> PRT
   <213> Artificial Sequence
<220>
   <223> CPP
<400> 569
   Trp Ile Ile Phe Lys Ile Ala Ala Ser His Lys Lys
   1 5 10
<210> 570
   <211> 12
   <212> PRT
   <213> Artificial Sequence
<220>
   <223> CPP
<400> 570
   Met Ile Ile Phe Ala Ile Ala Ala Ser His Lys Lys
   1               5                   10
<210> 571
   <211> 12
   <212> PRT
   <213> Artificial Sequence
<220>
   <223> CPP
<400> 571
   Leu Ile Ile Phe Arg Ile Leu Ile Ser His Lys Lys
   1               5                   10
<210> 572
   <211> 12
   <212> PRT
   <213> Artificial Sequence
<220>
   <223> CPP
<400> 572
   Met Ile Ile Phe Arg Ile Leu Ile Ser His Lys Lys
   1               5                   10
<210> 573
   <211> 12
   <212> PRT
   <213> Artificial Sequence
<220>
   <223> CPP
<400> 573
   Leu Ile Ile Phe Arg Ile Leu Ile Ser His Arg Arg
   1               5                   10
<210> 574
   <211> 12
   <212> PRT
   <213> Artificial Sequence
<220>
   <223> CPP
<400> 574
   Leu Ile Ile Phe Arg Ile Leu Ile Ser His His His
   1 5 10
<210> 575
   <211> 11
   <212> PRT
   <213> Artificial Sequence
<220>
   <223> CPP
<400> 575
   Leu Ile Ile Phe Arg Ile Leu Ile Ser His Lys
   1 5 10
<210> 576
   <211> 11
   <212> PRT
   <213> Artificial Sequence
<220>
   <223> CPP
<400> 576
   Leu Ile Ile Phe Arg Ile Leu Ile Ser His Arg
   1 5 10
<210> 577
   <211> 10
   <212> PRT
   <213> Artificial Sequence
<220>
   <223> CPP
<400> 577
   Leu Ile Ile Phe Arg Ile Leu Ile Ser His
   1 5 10
<210> 578
   <211> 12
   <212> PRT
   <213> Artificial Sequence
<220>
   <223> CPP
<400> 578
   Leu Ile Ile Phe Ala Ile Ala Ala Ser His Lys Lys
   1 5 10
<210> 579
   <211> 12
   <212> PRT
   <213> Artificial Sequence
<220>
   <223> CPP
<400> 579
   Leu Ile Ile Phe Ala Ile Leu Ile Ser His Lys Lys
   1               5                   10
<210> 580
   <211> 20
   <212> PRT
   <213> Artificial Sequence
<220>
   <223> CPP
<400> 580
   Arg Ile Leu Gln Gln Leu Leu Phe Ile His Phe Arg Ile Gly Cys Arg
   1               5                   10                  15
   His Ser Arg Ile
               20
<210> 581
   <211> 17
   <212> PRT
   <213> Artificial Sequence
<220>
   <223> CPP
<400> 581
   Arg Ile Leu Gln Gln Leu Leu Phe Ile His Phe Arg Ile Gly Cys Arg
   1               5                   10                  15
   His
<210> 582
   <211> 15
   <212> PRT
   <213> Artificial Sequence
<220>
   <223> CPP
<400> 582
   Arg Ile Leu Gln Gln Leu Leu Phe Ile His Phe Arg Ile Gly Cys
   1               5                   10                  15
<210> 583
   <211> 10
   <212> PRT
   <213> Artificial Sequence
<220>
   <223> CPP
<400> 583
   Arg Ile Phe Ile His Phe Arg Ile Gly Cys
   1               5                   10
<210> 584
   <211> 8
   <212> PRT
   <213> Artificial Sequence
<220>
   <223> CPP
<400> 584
   Arg Ile Phe Ile Arg Ile Gly Cys
   1               5
<210> 585
   <211> 11
   <212> PRT
   <213> Artificial Sequence
<220>
   <223> CPP
<400> 585
   Arg Ile Leu Gln Gln Leu Leu Phe Ile His Phe
   1               5                   10
<210> 586
   <211> 6
   <212> PRT
   <213> Artificial Sequence
<220>
   <223> CPP
<400> 586
   Arg Ile Phe Ile Gly Cys
   1               5
<210> 587
   <211> 6
   <212> PRT
   <213> Artificial Sequence
<220>
   <223> CPP
<400> 587
   Phe Ile Arg Ile Gly Cys
   1 5
<210> 588
   <211> 22
   <212> PRT
   <213> Artificial Sequence
<220>
   <223> CPP
<400> 588
   Asp Thr Trp Ala Gly Val Glu Ala Ile Ile Arg Ile Leu Gln Gln Leu
   1 5 10 15
   Leu Phe Ile His Phe Arg
   20
<210> 589
   <211> 5
   <212> PRT
   <213> Artificial Sequence
<220>
   <223> CPP
<400> 589
   Ile Gly Cys Arg His
   1 5
<210> 590
   <211> 23
   <212> PRT
   <213> Artificial Sequence
<220>
   <223> CPP
<400> 590
   Gly Tyr Gly Arg Lys Lys Arg Arg Gly Arg Arg Arg Thr His Arg Leu
   1 5 10 15
   Pro Arg Arg Arg Arg Arg Arg
   20
<210> 591
   <211> 12
   <212> PRT
   <213> Artificial Sequence
<220>
   <223> CPP
<400> 591
   Lys Arg Ile Ile Gln Arg Ile Leu Ser Arg Asn Ser
   1               5                   10
<210> 592
   <211> 12
   <212> PRT
   <213> Artificial Sequence
<220>
   <223> CPP
<400> 592
   Lys Arg Ile His Pro Arg Leu Thr Arg Ser Ile Arg
   1               5                   10
<210> 593
   <211> 12
   <212> PRT
   <213> Artificial Sequence
<220>
   <223> CPP
<400> 593
   Pro Pro Arg Leu Arg Lys Arg Arg Gln Leu Asn Met
   1               5                   10
<210> 594
   <211> 12
   <212> PRT
   <213> Artificial Sequence
<220>
   <223> CPP
<400> 594
   Met His Lys Arg Pro Thr Thr Pro Ser Arg Lys Met
   1               5                   10
<210> 595
   <211> 12
   <212> PRT
   <213> Artificial Sequence
<220>
   <223> CPP
<400> 595
   Arg Gln Arg Ser Arg Arg Arg Pro Leu Asn Ile Arg
   1 5 10
<210> 596
   <211> 12
   <212> PRT
   <213> Artificial Sequence
<220>
   <223> CPP
<400> 596
   Arg Ile Arg Met Ile Gln Asn Leu Ile Lys Lys Thr
   1 5 10
<210> 597
   <211> 12
   <212> PRT
   <213> Artificial Sequence
<220>
   <223> CPP
<400> 597
   Ser Arg Arg Lys Arg Gln Arg Ser Asn Met Arg Ile
   1 5 10
<210> 598
   <211> 12
   <212> PRT
   <213> Artificial Sequence
<220>
   <223> CPP
<400> 598
   Gln Arg Ile Arg Lys Ser Lys Ile Ser Arg Thr Leu
   1 5 10
<210> 599
   <211> 12
   <212> PRT
   <213> Artificial Sequence
<220>
   <223> CPP
<400> 599
   Pro Ser Lys Arg Leu Leu His Asn Asn Leu Arg Arg
   1 5 10
<210> 600
   <211> 12
   <212> PRT
   <213> Artificial Sequence
<220>
   <223> CPP
<400> 600
   His Arg His Ile Arg Arg Gln Ser Leu Ile Met Leu
   1               5                   10
<210> 601
   <211> 12
   <212> PRT
   <213> Artificial Sequence
<220>
   <223> CPP
<400> 601
   Pro Gln Asn Arg Leu Gln Ile Arg Arg His Ser Lys
   1               5                   10
<210> 602
   <211> 12
   <212> PRT
   <213> Artificial Sequence
<220>
   <223> CPP
<400> 602
   Pro Pro His Asn Arg Ile Gln Arg Arg Leu Asn Met
   1               5                   10
<210> 603
   <211> 12
   <212> PRT
   <213> Artificial Sequence
<220>
   <223> CPP
<400> 603
   Ser Met Leu Lys Arg Asn His Ser Thr Ser Asn Arg
   1               5                   10
<210> 604
   <211> 12
   <212> PRT
   <213> Artificial Sequence
<220>
   <223> CPP
<400> 604
   Gly Ser Arg His Pro Ser Leu Ile Ile Pro Arg Gln
   1 5 10
<210> 605
   <211> 12
   <212> PRT
   <213> Artificial Sequence
<220>
   <223> CPP
<400> 605
   Ser Pro Met Gln Lys Thr Met Asn Leu Pro Pro Met
   1 5 10
<210> 606
   <211> 12
   <212> PRT
   <213> Artificial Sequence
<220>
   <223> CPP
<400> 606
   Asn Lys Arg Ile Leu Ile Arg Ile Met Thr Arg Pro
   1 5 10
<210> 607
   <211> 12
   <212> PRT
   <213> Artificial Sequence
<220>
   <223> CPP
<400> 607
   His Gly Trp Glx Ile His Gly Leu Leu His Arg Ala
   1 5 10
<210> 608
   <211> 11
   <212> PRT
   <213> Artificial Sequence
<220>
   <223> CPP
<400> 608
   Ala Val Pro Ala Lys Lys Arg Glx Lys Ser Val
   1               5                   10
<210> 609
   <211> 11
   <212> PRT
   <213> Artificial Sequence
<220>
   <223> CPP
<400> 609
   Pro Asn Thr Arg Val Arg Pro Asp Val Ser Phe
   1               5                   10
<210> 610
   <211> 12
   <212> PRT
   <213> Artificial Sequence
<220>
   <223> CPP
<400> 610
   Leu Thr Arg Asn Tyr Glu Ala Trp Val Pro Thr Pro
   1               5                   10
<210> 611
   <211> 13
   <212> PRT
   <213> Artificial Sequence
<220>
   <223> CPP
<400> 611
   Ser Ala Glu Thr Val Glu Ser Cys Leu Ala Lys Ser His
   1               5                   10
<210> 612
   <211> 12
   <212> PRT
   <213> Artificial Sequence
<220>
   <223> CPP
<400> 612
   Tyr Ser His Ile Ala Thr Leu Pro Phe Thr Pro Thr
   1               5                   10
<210> 613
   <211> 12
   <212> PRT
   <213> Artificial Sequence
<220>
   <223> CPP
<400> 613
   Ser Tyr Ile Gln Arg Thr Pro Ser Thr Thr Leu Pro
   1 5 10
<210> 614
   <211> 12
   <212> PRT
   <213> Artificial Sequence
<220>
   <223> CPP
<400> 614
   Ala Val Pro Ala Glu Asn Ala Leu Asn Asn Pro Phe
   1 5 10
<210> 615
   <211> 12
   <212> PRT
   <213> Artificial Sequence
<220>
   <223> CPP
<400> 615
   Ser Phe His Gln Phe Ala Arg Ala Thr Leu Ala Ser
   1 5 10
<210> 616
   <211> 12
   <212> PRT
   <213> Artificial Sequence
<220>
   <223> CPP
<400> 616
   Gln Ser Pro Thr Asp Phe Thr Phe Pro Asn Pro Leu
   1 5 10
<210> 617
   <211> 12
   <212> PRT
   <213> Artificial Sequence
<220>
   <223> CPP
<400> 617
   His Phe Ala Ala Trp Gly Gly Trp Ser Leu Val His
   1 5 10
<210> 618
   <211> 12
   <212> PRT
   <213> Artificial Sequence
<220>
   <223> CPP
<400> 618
   His Ile Gln Leu Ser Pro Phe Ser Gln Ser Trp Arg
   1 5 10
<210> 619
   <211> 12
   <212> PRT
   <213> Artificial Sequence
<220>
   <223> CPP
<400> 619
   Leu Thr Met Pro Ser Asp Leu Gln Pro Val Leu Trp
   1 5 10
<210> 620
   <211> 12
   <212> PRT
   <213> Artificial Sequence
<220>
   <223> CPP
<400> 620
   Phe Gln Pro Tyr Asp His Pro Ala Glu Val Ser Tyr
   1 5 10
<210> 621
   <211> 12
   <212> PRT
   <213> Artificial Sequence
<220>
   <223> CPP
<400> 621
   Phe Asp Pro Phe Phe Trp Lys Tyr Ser Pro Arg Asp
   1 5 10
<210> 622
   <211> 12
   <212> PRT
   <213> Artificial Sequence
<220>
   <223> CPP
<400> 622
   Phe Ala Pro Trp Asp Thr Ala Ser Phe Met Leu Gly
   1 5 10
<210> 623
   <211> 12
   <212> PRT
   <213> Artificial Sequence
<220>
   <223> CPP
<400> 623
   Phe Thr Tyr Lys Asn Phe Phe Trp Leu Pro Glu Leu
   1 5 10
<210> 624
   <211> 12
   <212> PRT
   <213> Artificial Sequence
<220>
   <223> CPP
<400> 624
   Ser Ala Thr Gly Ala Pro Trp Lys Met Trp Val Arg
   1 5 10
<210> 625
   <211> 12
   <212> PRT
   <213> Artificial Sequence
<220>
   <223> CPP
<400> 625
   Ser Leu Gly Trp Met Leu Pro Phe Ser Pro Pro Phe
   1 5 10
<210> 626
   <211> 12
   <212> PRT
   <213> Artificial Sequence
<220>
   <223> CPP
<400> 626
   Ser His Ala Phe Thr Trp Pro Thr Tyr Leu Gln Leu
   1 5 10
<210> 627
   <211> 12
   <212> PRT
   <213> Artificial Sequence
<220>
   <223> CPP
<400> 627
   Ser His Asn Trp Leu Pro Leu Trp Pro Leu Arg Pro
   1 5 10
<210> 628
   <211> 12
   <212> PRT
   <213> Artificial Sequence
<220>
   <223> CPP
<400> 628
   Ser Trp Leu Pro Tyr Pro Trp His Val Pro Ser Ser
   1 5 10
<210> 629
   <211> 12
   <212> PRT
   <213> Artificial Sequence
<220>
   <223> CPP
<400> 629
   Ser Trp Trp Thr Pro Trp His Val His Ser Glu Ser
   1 5 10
<210> 630
   <211> 12
   <212> PRT
   <213> Artificial Sequence
<220>
   <223> CPP
<400> 630
   Ser Trp Ala Gln His Leu Ser Leu Pro Pro Val Leu
   1 5 10
<210> 631
   <211> 12
   <212> PRT
   <213> Artificial Sequence
<220>
   <223> CPP
<400> 631
   Ser Ser Ser Ile Phe Pro Pro Trp Leu Ser Phe Phe
   1 5 10
<210> 632
   <211> 12
   <212> PRT
   <213> Artificial Sequence
<220>
   <223> CPP
<400> 632
   Leu Asn Val Pro Pro Ser Trp Phe Leu Ser Gln Arg
   1 5 10
<210> 633
   <211> 12
   <212> PRT
   <213> Artificial Sequence
<220>
   <223> CPP
<400> 633
   Leu Asp Ile Thr Pro Phe Leu Ser Leu Thr Leu Pro
   1 5 10
<210> 634
   <211> 12
   <212> PRT
   <213> Artificial Sequence
<220>
   <223> CPP
<400> 634
   Leu Pro His Pro Val Leu His Met Gly Pro Leu Arg
   1 5 10
<210> 635
   <211> 12
   <212> PRT
   <213> Artificial Sequence
<220>
   <223> CPP
<400> 635
   Val Ser Lys Gln Pro Tyr Tyr Met Trp Asn Gly Asn
   1               5                   10
<210> 636
   <211> 12
   <212> PRT
   <213> Artificial Sequence
<220>
   <223> CPP
<400> 636
   Asn Tyr Thr Thr Tyr Lys Ser His Phe Gln Asp Arg
   1               5                   10
<210> 637
   <211> 12
   <212> PRT
   <213> Artificial Sequence
<220>
   <223> CPP
<400> 637
   Ala Ile Pro Asn Asn Gln Leu Gly Phe Pro Phe Lys
   1               5                   10
<210> 638
   <211> 12
   <212> PRT
   <213> Artificial Sequence
<220>
   <223> CPP
<400> 638
   Asn Ile Glu Asn Ser Thr Leu Ala Thr Pro Leu Ser
   1               5                   10
<210> 639
   <211> 12
   <212> PRT
   <213> Artificial Sequence
<220>
   <223> CPP
<400> 639
   Tyr Pro Tyr Asp Ala Asn His Thr Arg Ser Pro Thr
   1 5 10
<210> 640
   <211> 12
   <212> PRT
   <213> Artificial Sequence
<220>
   <223> CPP
<400> 640
   Asp Pro Ala Thr Asn Pro Gly Pro His Phe Pro Arg
   1 5 10
<210> 641
   <211> 12
   <212> PRT
   <213> Artificial Sequence
<220>
   <223> CPP
<400> 641
   Thr Leu Pro Ser Pro Leu Ala Leu Leu Thr Val His
   1 5 10
<210> 642
   <211> 12
   <212> PRT
   <213> Artificial Sequence
<220>
   <223> CPP
<400> 642
   His Pro Gly Ser Pro Phe Pro Pro Glu His Arg Pro
   1 5 10
<210> 643
   <211> 12
   <212> PRT
   <213> Artificial Sequence
<220>
   <223> CPP
<400> 643
   Thr Ser His Thr Asp Ala Pro Pro Ala Arg Ser Pro
   1 5 10
<210> 644
   <211> 12
   <212> PRT
   <213> Artificial Sequence
<220>
   <223> CPP
<400> 644
   Met Thr Pro Ser Ser Leu Ser Thr Leu Pro Trp Pro
   1 5 10
<210> 645
   <211> 12
   <212> PRT
   <213> Artificial Sequence
<220>
   <223> CPP
<400> 645
   Val Leu Gly Gln Ser Gly Tyr Leu Met Pro Met Arg
   1 5 10
<210> 646
   <211> 12
   <212> PRT
   <213> Artificial Sequence
<220>
   <223> CPP
<400> 646
   Gln Pro Ile Ile Ile Thr Ser Pro Tyr Leu Pro Ser
   1 5 10
<210> 647
   <211> 12
   <212> PRT
   <213> Artificial Sequence
<220>
   <223> CPP
<400> 647
   Thr Pro Lys Thr Met Thr Gln Thr Tyr Asp Phe Ser
   1 5 10
<210> 648
   <211> 12
   <212> PRT
   <213> Artificial Sequence
<220>
   <223> CPP
<400> 648
   Asn Ser Gly Thr Met Gln Ser Ala Ser Arg Ala Thr
   1               5                   10
<210> 649
   <211> 12
   <212> PRT
   <213> Artificial Sequence
<220>
   <223> CPP
<400> 649
   Gln Ala Ala Ser Arg Val Glu Asn Tyr Met His Arg
       1               5                   10
<210> 650
   <211> 12
   <212> PRT
   <213> Artificial Sequence
<220>
   <223> CPP
<400> 650
   His Gln His Lys Pro Pro Pro Leu Thr Asn Asn Trp
   1               5                   10
<210> 651
   <211> 12
   <212> PRT
   <213> Artificial Sequence
<220>
   <223> CPP
<400> 651
   Ser Asn Pro Trp Asp Ser Leu Leu Ser Val Ser Thr
   1               5                   10
<210> 652
   <211> 12
   <212> PRT
   <213> Artificial Sequence
<220>
   <223> CPP
<400> 652
   Lys Thr Ile Glu Ala His Pro Pro Tyr Tyr Ala Ser
   1 5 10
<210> 653
   <211> 12
   <212> PRT
   <213> Artificial Sequence
<220>
   <223> CPP
<400> 653
   Glu Pro Asp Asn Trp Ser Leu Asp Phe Pro Arg Arg
   1 5 10
<210> 654
   <211> 12
   <212> PRT
   <213> Artificial Sequence
<220>
   <223> CPP
<400> 654
   His Gln His Lys Pro Pro Pro Leu Thr Asn Asn Trp
   1 5 10
<210> 655
   <211> 20
   <212> PRT
   <213> Artificial Sequence
<220>
   <223> CPP
<400> 655
   Gly Leu Trp Arg Ala Leu Trp Arg Leu Leu Arg Ser Leu Trp Arg Leu
   1 5 10 15
   Leu Trp Lys Ala
   20
<210> 656
   <211> 21
   <212> PRT
   <213> Artificial Sequence
<220>
   <223> CPP
<400> 656
   Gly Leu Trp Arg Ala Leu Trp Arg Ala Leu Trp Arg Ser Leu Trp Lys
   1               5                   10                  15
   Leu Lys Arg Lys Val
               20
<210> 657
   <211> 20
   <212> PRT
   <213> Artificial Sequence
<220>
   <223> CPP
<400> 657
   Gly Leu Trp Arg Ala Leu Trp Arg Ala Leu Arg Ser Leu Trp Lys Leu
   1               5                   10                  15
   Lys Arg Lys Val
               20
<210> 658
   <211> 20
   <212> PRT
   <213> Artificial Sequence
<220>
   <223> CPP
<400> 658
   Gly Leu Trp Arg Ala Leu Trp Arg Gly Leu Arg Ser Leu Trp Lys Leu
   1               5                   10                  15
   Lys Arg Lys Val
               20
<210> 659
   <211> 20
   <212> PRT
   <213> Artificial Sequence
<220>
   <223> CPP
<400> 659
   Gly Leu Trp Arg Ala Leu Trp Arg Gly Leu Arg Ser Leu Trp Lys Lys
   1               5                   10                  15
   Lys Arg Lys Val
               20
<210> 660
   <211> 21
   <212> PRT
   <213> Artificial Sequence
<220>
   <223> CPP
<400> 660
   Gly Leu Trp Arg Ala Leu Trp Arg Ala Leu Trp Arg Ser Leu Trp Lys
   1               5                   10                  15
   Leu Lys Trp Lys Val
               20
<210> 661
   <211> 21
   <212> PRT
   <213> Artificial Sequence
<220>
   <223> CPP
<400> 661
   Gly Leu Trp Arg Ala Leu Trp Arg Ala Leu Trp Arg Ser Leu Trp Lys
   1               5                   10                  15
   Ser Lys Arg Lys Val
               20
<210> 662
   <211> 21
   <212> PRT
   <213> Artificial Sequence
<220>
   <223> CPP
<400> 662
   Gly Leu Trp Arg Ala Leu Trp Arg Ala Leu Trp Arg Ser Leu Trp Lys
   1               5                   10                  15
   Lys Lys Arg Lys Val
               20
<210> 663
   <211> 21
   <212> PRT
   <213> Artificial Sequence
<220>
   <223> CPP
<400> 663
   Gly Leu Trp Arg Ala Leu Trp Arg Ala Leu Trp Arg Ser Leu Trp Lys
   1               5                   10                  15
   Leu Lys Arg Lys Val
               20
<210> 664
   <211> 27
   <212> PRT
   <213> Artificial Sequence
<220>
   <223> CPP
<400> 664
   Gly Leu Trp Arg Ala Leu Trp Arg Leu Leu Arg Ser Leu Trp Arg Leu
   1               5                   10                  15
   Leu Trp Ser Gln Pro Lys Lys Lys Arg Lys Val
               20                  25
<210> 665
   <211> 11
   <212> PRT
   <213> Artificial Sequence
<220>
   <223> CPP
<400> 665
   Tyr Ala Arg Ala Ala Arg Arg Ala Ala Arg Arg
   1               5                   10
<210> 666
   <211> 11
   <212> PRT
   <213> Artificial Sequence
<220>
   <223> CPP
<400> 666
   Pro Ala Arg Ala Ala Arg Arg Ala Ala Arg Arg
   1 5 10
<210> 667
   <211> 11
   <212> PRT
   <213> Artificial Sequence
<220>
   <223> CPP
<400> 667
   Tyr Pro Arg Ala Ala Arg Arg Ala Ala Arg Arg
   1 5 10
<210> 668
   <211> 11
   <212> PRT
   <213> Artificial Sequence
<220>
   <223> CPP
<400> 668
   Tyr Arg Arg Ala Ala Arg Arg Ala Ala Arg Ala
   1 5 10
<210> 669
   <211> 11
   <212> PRT
   <213> Artificial Sequence
<220>
   <223> CPP
<400> 669
   Tyr Gly Arg Ala Ala Arg Arg Ala Ala Arg Arg
   1 5 10
<210> 670
   <211> 11
   <212> PRT
   <213> Artificial Sequence
<220>
   <223> CPP
<400> 670
   Tyr Ala Arg Glu Ala Arg Arg Ala Ala Arg Arg
   1 5 10
<210> 671
   <211> 11
   <212> PRT
   <213> Artificial Sequence
<220>
   <223> CPP
<400> 671
   Tyr Glu Arg Glu Ala Arg Arg Ala Ala Arg Arg
   1               5                   10
<210> 672
   <211> 11
   <212> PRT
   <213> Artificial Sequence
<220>
   <223> CPP
<400> 672
   Tyr Lys Arg Ala Ala Arg Arg Ala Ala Arg Arg
   1               5                   10
<210> 673
   <211> 11
   <212> PRT
   <213> Artificial Sequence
<220>
   <223> CPP
<400> 673
   Tyr Ala Arg Lys Ala Arg Arg Ala Ala Arg Arg
   1               5                   10
<210> 674
   <211> 11
   <212> PRT
   <213> Artificial Sequence
<220>
   <223> CPP
<400> 674
   Tyr Lys Arg Lys Ala Arg Arg Ala Ala Arg Arg
   1               5                   10
<210> 675
   <211> 11
   <212> PRT
   <213> Artificial Sequence
<220>
   <223> CPP
<400> 675
   Tyr Gly Arg Arg Ala Arg Arg Ala Ala Arg Arg
   1 5 10
<210> 676
   <211> 11
   <212> PRT
   <213> Artificial Sequence
<220>
   <223> CPP
<400> 676
   Tyr Gly Arg Arg Ala Arg Arg Arg Ala Arg Arg
   1 5 10
<210> 677
   <211> 11
   <212> PRT
   <213> Artificial Sequence
<220>
   <223> CPP
<400> 677
   Tyr Gly Arg Arg Arg Arg Arg Arg Arg Arg Arg
   1 5 10
<210> 678
   <211> 11
   <212> PRT
   <213> Artificial Sequence
<220>
   <223> CPP
<400> 678
   Tyr Arg Arg Arg Arg Arg Arg Arg Arg Arg Arg
   1 5 10
<210> 679
   <211> 16
   <212> PRT
   <213> Artificial Sequence
<220>
   <223> CPP
<400> 679
   Gly Lys Ile Asn Leu Lys Ala Leu Ala Ala Leu Ala Lys Lys Ile Leu
   1 5 10 15
<210> 680
   <211> 39
   <212> PRT
   <213> Artificial Sequence
<220>
   <223> CPP
<400> 680
   Gly Arg Lys Lys Arg Arg Gln Arg Arg Arg Pro Pro Gln Gly Arg Lys
   1               5                   10                  15
   Lys Arg Arg Gln Arg Arg Arg Pro Pro Gln Gly Arg Lys Lys Arg Arg
               20                  25                  30
   Gln Arg Arg Arg Pro Pro Gln
           35
<210> 681
   <211> 22
   <212> PRT
   <213> Artificial Sequence
<220>
   <223> CPP
<400> 681
   Gly Glu Gln Ile Ala Gln Leu Ile Ala Gly Tyr Ile Asp Ile Ile Leu
   1               5                   10                  15
   Lys Lys Lys Lys Ser Lys
               20
<210> 682
   <211> 28
   <212> PRT
   <213> Artificial Sequence
<220>
   <223> CPP
<400> 682
   Ala Ala Val Ala Leu Leu Pro Ala Val Leu Leu Ala Leu Leu Ala Pro
   1               5                   10                  15
   Arg Lys Lys Arg Arg Gln Arg Arg Arg Pro Pro Gln
               20                  25
<210> 683
   <211> 29
   <212> PRT
   <213> Artificial Sequence
<220>
   <223> CPP
<400> 683
   Ala Ala Val Ala Leu Leu Pro Ala Val Leu Leu Ala Leu Leu Ala Pro
   1               5                   10                  15
   Arg Lys Lys Arg Arg Gln Arg Arg Arg Pro Pro Gln Cys
               20                  25
<210> 684
   <211> 29
   <212> PRT
   <213> Artificial Sequence
<220>
   <223> CPP
<400> 684
   Arg Lys Lys Arg Arg Gln Arg Arg Arg Pro Pro Gln Cys Ala Ala Val
   1               5                   10                  15
   Ala Leu Leu Pro Ala Val Leu Leu Ala Leu Leu Ala Pro
               20                  25
<210> 685
   <211> 29
   <212> PRT
   <213> Artificial Sequence
<220>
   <223> CPP
<400> 685
   Arg Arg Arg Gln Arg Arg Lys Arg Gly Gly Asp Ile Met Gly Glu Trp
   1               5                   10                  15
   Gly Asn Glu Ile Phe Gly Ala Ile Ala Gly Phe Leu Gly
               20                  25
<210> 686
   <211> 29
   <212> PRT
   <213> Artificial Sequence
<220>
   <223> CPP
<400> 686
   Arg Arg Arg Gln Arg Arg Lys Arg Gly Gly Asp Ile Met Gly Glu Trp
   1               5                   10                  15
   Gly Asn Glu Ile Phe Gly Ala Ile Ala Gly Phe Leu Gly
               20                  25
<210> 687
   <211> 25
   <212> PRT
   <213> Artificial Sequence
<220>
   <223> CPP
<400> 687
   Tyr Gly Arg Lys Lys Arg Arg Gln Arg Arg Arg Gly Cys Tyr Gly Arg
   1               5                   10                  15
   Lys Lys Arg Arg Gln Arg Arg Arg Gly
               20                  25
<210> 688
   <211> 22
   <212> PRT
   <213> Artificial Sequence
<220>
   <223> CPP
<400> 688
   Ala Ala Val Ala Leu Leu Pro Ala Val Leu Leu Ala Leu Leu Ala Pro
   1               5                   10                  15
   Arg Arg Arg Arg Arg Arg
               20
<210> 689
   <211> 16
   <212> PRT
   <213> Artificial Sequence
<220>
   <223> CPP
<400> 689
   Arg Leu Trp Arg Ala Leu Pro Arg Val Leu Arg Arg Leu Leu Arg Pro
   1               5                   10                  15
<210> 690
   <211> 28
   <212> PRT
   <213> Artificial Sequence
<220>
   <223> CPP
<400> 690
   Ala Ala Val Ala Leu Leu Pro Ala Val Leu Leu Ala Leu Leu Ala Pro
   1 5 10 15
   Ser Gly Ala Ser Gly Leu Asp Lys Arg Asp Tyr Val
   20 25
<210> 691
   <211> 33
   <212> PRT
   <213> Artificial Sequence
<220>
   <223> CPP
<400> 691
   Leu Leu Glu Thr Leu Leu Lys Pro Phe Gln Cys Arg Ile Cys Met Arg
   1 5 10 15
   Asn Phe Ser Thr Arg Gln Ala Arg Arg Asn His Arg Arg Arg His Arg
   20 25 30
   Arg
<210> 692
   <211> 27
   <212> PRT
   <213> Artificial Sequence
<220>
   <223> CPP
<400> 692
   Ala Ala Val Ala Cys Arg Ile Cys Met Arg Asn Phe Ser Thr Arg Gln
   1 5 10 15
   Ala Arg Arg Asn His Arg Arg Arg His Arg Arg
   20 25
<210> 693
   <211> 35
   <212> PRT
   <213> Artificial Sequence
<220>
   <223> CPP
<400> 693
   Arg Gln Ile Lys Ile Trp Phe Gln Asn Arg Arg Met Lys Trp Lys Lys
   1               5                   10                  15
   Asp Ile Met Gly Glu Trp Gly Asn Glu Ile Phe Gly Ala Ile Ala Gly
               20                  25                  30
   Phe Leu Gly
           35
<210> 694
   <211> 37
   <212> PRT
   <213> Artificial Sequence
<220>
   <223> CPP
<400> 694
   Ser Gly Arg Gly Lys Gln Gly Gly Lys Ala Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly
           35
<210> 695
   <211> 38
   <212> PRT
   <213> Artificial Sequence
<220>
   <223> CPP
<400> 695
   Ser Gly Arg Gly Lys Gln Gly Gly Lys Ala Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly Cys
           35
<210> 696
   <211> 25
   <212> PRT
   <213> Artificial Sequence
<220>
   <223> CPP
<400> 696
   Lys Lys Asp Gly Lys Lys Arg Lys Arg Ser Arg Lys Glu Ser Tyr Ser
   1               5                   10                  15
   Val Tyr Val Tyr Lys Val Leu Lys Gln
               20                  25
<210> 697
   <211> 36
   <212> PRT
   <213> Artificial Sequence
<220>
   <223> CPP
<400> 697
   Lys Gly Ser Lys Lys Ala Val Thr Lys Ala Gln Lys Lys Asp Gly Lys
   1               5                   10                  15
   Lys Arg Lys Arg Ser Arg Lys Glu Ser Tyr Ser Val Tyr Val Tyr Lys
               20                  25                  30
   Val Leu Lys Gln
           35
<210> 698
   <211> 28
   <212> PRT
   <213> Artificial Sequence
<220>
   <223> CPP
<400> 698
   Lys Glu Thr Trp Trp Glu Thr Trp Trp Thr Glu Trp Ser Gln Pro Gly
   1               5                   10                  15
   Arg Lys Lys Arg Arg Gln Arg Arg Arg Pro Pro Gln
               20                  25
<210> 699
   <211> 15
   <212> PRT
   <213> Artificial Sequence
<220>
   <223> CPP
<400> 699
   Arg Val Ile Arg Trp Phe Gln Asn Lys Arg Cys Lys Asp Lys Lys
   1               5                   10                  15
<210> 700
   <211> 18
   <212> PRT
   <213> Artificial Sequence
<220>
   <223> CPP
<400> 700
   Leu Gly Leu Leu Leu Arg His Leu Arg His His Ser Asn Leu Leu Ala
   1               5                   10                  15
   Asn Ile
<210> 701
   <211> 16
   <212> PRT
   <213> Artificial Sequence
<220>
   <223> CPP
<400> 701
   Lys Leu Trp Ser Ala Trp Pro Ser Leu Trp Ser Ser Leu Trp Lys Pro
   1               5                   10                  15
<210> 702
   <211> 22
   <212> PRT
   <213> Artificial Sequence
<220>
   <223> CPP
<400> 702
   Gly Leu Gly Ser Leu Leu Lys Lys Ala Gly Lys Lys Leu Lys Gln Pro
   1               5                   10                  15
   Lys Ser Lys Arg Lys Val
               20
<210> 703
   <211> 12
   <212> PRT
   <213> Artificial Sequence
<220>
   <223> CPP
<400> 703
   Phe Lys Gln Gln Gln Gln Gln Gln Gln Gln Gln Gln
   1               5                   10
<210> 704
   <211> 4
   <212> PRT
   <213> Artificial Sequence
<220>
   <223> CPP
<400> 704
   Tyr Arg Phe Lys
   1
<210> 705
   <211> 13
   <212> PRT
   <213> Artificial Sequence
<220>
   <223> CPP
<400> 705
   Tyr Arg Phe Lys Tyr Arg Phe Lys Tyr Arg Leu Phe Lys
   1               5                   10
<210> 706
   <211> 10
   <212> PRT
   <213> Artificial Sequence
<220>
   <223> CPP
<400> 706
   Trp Arg Phe Lys Lys Ser Lys Arg Lys Val
   1               5                   10
<210> 707
   <211> 20
   <212> PRT
   <213> Artificial Sequence
<220>
   <223> CPP
<400> 707
   Trp Arg Phe Lys Ala Ala Val Ala Leu Leu Pro Ala Val Leu Leu Ala
   1               5                   10                  15
   Leu Leu Ala Pro
               20
<210> 708
   <211> 8
   <212> PRT
   <213> Artificial Sequence
<220>
   <223> CPP
<400> 708
   Trp Arg Phe Lys Trp Arg Phe Lys
   1               5
<210> 709
   <211> 12
   <212> PRT
   <213> Artificial Sequence
<220>
   <223> CPP
<400> 709
   Trp Arg Phe Lys Trp Arg Phe Lys Trp Arg Phe Lys
   1               5                   10
<210> 710
   <211> 36
   <212> PRT
   <213> Artificial Sequence
<220>
   <223> CPP
<400> 710
   Lys Gly Ser Lys Lys Ala Val Thr Lys Ala Gln Lys Lys Asp Gly Lys
   1               5                   10                  15
   Lys Arg Lys Arg Ser Arg Lys Glu Ser Tyr Ser Val Tyr Val Tyr Lys
               20                  25                  30
   Val Leu Lys Gln
           35
<210> 711
   <211> 36
   <212> PRT
   <213> Artificial Sequence
<220>
   <223> CPP
<400> 711
   Arg Gly Ser Arg Arg Ala Val Thr Arg Ala Gln Arg Arg Asp Gly Arg
   1               5                   10                  15
   Arg Arg Arg Arg Ser Arg Arg Glu Ser Tyr Ser Val Tyr Val Tyr Arg
               20                  25                  30
   Val Leu Arg Gln
           35
<210> 712
   <211> 15
   <212> PRT
   <213> Artificial Sequence
<220>
   <223> CPP
<400> 712
   Arg Val Ile Arg Trp Phe Gln Asn Lys Arg Ser Lys Asp Lys Lys
   1               5                   10                  15
<210> 713
   <211> 28
   <212> PRT
   <213> Artificial Sequence
<220>
   <223> CPP
<400> 713
   Ala Ala Val Ala Leu Leu Pro Ala Val Leu Leu Ala Leu Leu Ala Pro
   1               5                   10                  15
   Arg Lys Lys Arg Arg Gln Arg Arg Arg Pro Pro Gln
               20                  25
<210> 714
   <211> 5
   <212> PRT
   <213> Artificial Sequence
<220>
   <223> CPP
<400> 714
   Cys Trp Lys Lys Lys
   1               5
<210> 715
   <211> 10
   <212> PRT
   <213> Artificial Sequence
<220>
   <223> CPP
<400> 715
   Cys Trp Lys Lys Lys Lys Lys Lys Lys Lys
   1               5                   10
<210> 716
   <211> 15
   <212> PRT
   <213> Artificial Sequence
<220>
   <223> CPP
<400> 716
   Cys Trp Lys Lys Lys Lys Lys Lys Lys Lys Lys Lys Lys Lys Lys
   1               5                   10                  15
<210> 717
   <211> 20
   <212> PRT
   <213> Artificial Sequence
<220>
   <223> CPP
<400> 717
   Cys Trp Lys Lys Lys Lys Lys Lys Lys Lys Lys Lys Lys Lys Lys Lys
   1               5                   10                  15
   Lys Lys Lys Lys
               20
<210> 718
   <211> 19
   <212> PRT
   <213> Artificial Sequence
<220>
   <223> CPP
<400> 718
   Lys Lys Lys Lys Lys Lys Lys Lys Lys Lys Lys Lys Lys Lys Lys Lys
   1               5                   10                  15
   Lys Lys Lys
<210> 719
   <211> 19
   <212> PRT
   <213> Artificial Sequence
<220>
   <223> CPP
<400> 719
   Lys Lys Trp Lys Met Arg Arg Gly Ala Gly Arg Arg Arg Arg Arg Arg
   1               5                   10                  15
   Arg Arg Arg
<210> 720
   <211> 12
   <212> PRT
   <213> Artificial Sequence
<220>
   <223> CPP
<400> 720
   Ala Pro Trp His Leu Ser Ser Gln Tyr Ser Arg Thr
   1               5                   10
<210> 721
   <211> 26
   <212> PRT
   <213> Artificial Sequence
<220>
   <223> CPP
<400> 721
   Ala Ala Val Ala Leu Leu Pro Ala Val Leu Leu Ala Leu Leu Ala Lys
   1               5                   10                  15
   Lys Asn Asn Leu Lys Asp Cys Gly Leu Phe
               20                  25
<210> 722
   <211> 26
   <212> PRT
   <213> Artificial Sequence
<220>
   <223> CPP
<400> 722
   Ala Ala Val Ala Leu Leu Pro Ala Val Leu Leu Ala Leu Leu Ala Lys
   1 5 10 15
   Lys Asn Asn Leu Lys Glu Cys Gly Leu Tyr
   20 25
<210> 723
   <211> 26
   <212> PRT
   <213> Artificial Sequence
<220>
   <223> CPP
<400> 723
   Ala His Ala Leu Cys Leu Thr Glu Arg Gln Ile Lys Ile Trp Phe Gln
   1 5 10 15
   Asn Arg Arg Met Lys Trp Lys Lys Glu Asn
   20 25
<210> 724
   <211> 26
   <212> PRT
   <213> Artificial Sequence
<220>
   <223> CPP
<400> 724
   Ala His Ala Leu Cys Pro Pro Glu Arg Gln Ile Lys Ile Trp Phe Gln
   1 5 10 15
   Asn Arg Arg Met Lys Trp Lys Lys Glu Asn
   20 25
<210> 725
   <211> 26
   <212> PRT
   <213> Artificial Sequence
<220>
   <223> CPP
<400> 725
   Ala Tyr Ala Leu Cys Leu Thr Glu Arg Gln Ile Lys Ile Trp Phe Ala
   1 5 10 15
   Asn Arg Arg Met Lys Trp Lys Lys Glu Asn
   20 25
<210> 726
   <211> 34
   <212> PRT
   <213> Artificial Sequence
<220>
   <223> CPP
<400> 726
   Gly Gly Val Cys Pro Lys Ile Leu Lys Lys Cys Arg Arg Asp Ser Asp
   1 5 10 15
   Cys Pro Gly Ala Cys Ile Cys Arg Gly Asn Gly Tyr Cys Gly Ser Gly
   20 25 30
   Ser Asp
<210> 727
   <211> 34
   <212> PRT
   <213> Artificial Sequence
<220>
   <223> CPP
<400> 727
   Gly Gly Val Cys Pro Lys Ile Leu Ala Ala Cys Arg Arg Asp Ser Asp
   1 5 10 15
   Cys Pro Gly Ala Cys Ile Cys Arg Gly Asn Gly Tyr Cys Gly Ser Gly
   20 25 30
   Ser Asp
<210> 728
   <211> 34
   <212> PRT
   <213> Artificial Sequence
<220>
   <223> CPP
<400> 728
   Gly Gly Val Cys Pro Ala Ile Leu Lys Lys Cys Arg Arg Asp Ser Asp
   1 5 10 15
   Cys Pro Gly Ala Cys Ile Cys Arg Gly Asn Gly Tyr Cys Gly Ser Gly
   20 25 30
   Ser Asp
<210> 729
   <211> 34
   <212> PRT
   <213> Artificial Sequence
<220>
   <223> CPP
<400> 729
   Gly Gly Val Cys Pro Lys Ile Leu Ala Lys Cys Arg Arg Asp Ser Asp
   1 5 10 15
   Cys Pro Gly Ala Cys Ile Cys Arg Gly Asn Gly Tyr Cys Gly Ser Gly
   20 25 30
   Ser Asp
<210> 730
   <211> 34
   <212> PRT
   <213> Artificial Sequence
<220>
   <223> CPP
<400> 730
   Gly Gly Val Cys Pro Lys Ile Leu Lys Ala Cys Arg Arg Asp Ser Asp
   1 5 10 15
   Cys Pro Gly Ala Cys Ile Cys Arg Gly Asn Gly Tyr Cys Gly Ser Gly
   20 25 30
   Ser Asp
<210> 731
   <211> 29
   <212> PRT
   <213> Artificial Sequence
<220>
   <223> CPP
<400> 731
   Gly Leu Pro Val Cys Gly Glu Thr Cys Val Gly Gly Thr Cys Asn Thr
   1 5 10 15
   Pro Gly Cys Lys Cys Ser Trp Pro Val Cys Thr Arg Asn
   20 25
<210> 732
   <211> 29
   <212> PRT
   <213> Artificial Sequence
<220>
   <223> CPP
<400> 732
   Gly Leu Pro Val Cys Gly Glu Thr Cys Val Gly Gly Thr Cys Asn Thr
   1 5 10 15
   Pro Gly Cys Thr Cys Ser Trp Pro Lys Cys Thr Arg Asn
   20 25
<210> 733
   <211> 14
   <212> PRT
   <213> Artificial Sequence
<220>
   <223> CPP
<400> 733
   Gly Arg Cys Thr Lys Ser Ile Pro Pro Ile Cys Phe Pro Asp
   1 5 10
<210> 734
   <211> 33
   <212> PRT
   <213> Artificial Sequence
<220>
   <223> CPP
<400> 734
   Arg Gln Ile Lys Ile Trp Phe Gln Asn Arg Arg Met Lys Trp Lys Lys
   1 5 10 15
   Thr Tyr Ala Asp Phe Ile Ala Ser Gly Arg Thr Gly Arg Arg Asn Ala
   20 25 30
   Ile
<210> 735
   <211> 30
   <212> PRT
   <213> Artificial Sequence
<220>
   <223> CPP
<400> 735
   Gly Arg Lys Lys Arg Arg Gln Arg Arg Arg Pro Pro Gln Thr Tyr Ala
   1               5                   10                  15
   Asp Phe Ile Ala Ser Gly Arg Thr Gly Arg Arg Asn Ala Ile
               20                  25                  30
<210> 736
   <211> 21
   <212> PRT
   <213> Artificial Sequence
<220>
   <223> CPP
<400> 736
   Ala Gly Tyr Leu Leu Gly Lys Ile Asn Leu Lys Ala Leu Ala Ala Leu
   1               5                   10                  15
   Ala Lys Lys Ile Leu
               20
<210> 737
   <211> 38
   <212> PRT
   <213> Artificial Sequence
<220>
   <223> CPP
<400> 737
   Ala Gly Tyr Leu Leu Gly Lys Ile Asn Leu Lys Ala Leu Ala Ala Leu
   1               5                   10                  15
   Ala Lys Lys Ile Leu Thr Tyr Ala Asp Phe Ile Ala Ser Gly Arg Thr
               20                  25                  30
   Gly Arg Arg Asn Ala Ile
           35
<210> 738
   <211> 11
   <212> PRT
   <213> Artificial Sequence
<220>
   <223> CPP
<400> 738
   Arg Arg Arg Arg Arg Arg Arg Arg Arg Arg Arg
   1 5 10
<210> 739
   <211> 28
   <212> PRT
   <213> Artificial Sequence
<220>
   <223> CPP
<400> 739
   Arg Arg Arg Arg Arg Arg Arg Arg Arg Arg Arg Thr Tyr Ala Asp Phe
   1 5 10 15
   Ile Ala Ser Gly Arg Thr Gly Arg Arg Asn Ala Ile
   20 25
<210> 740
   <211> 10
   <212> PRT
   <213> Artificial Sequence
<220>
   <223> CPP
<400> 740
   Arg Arg Arg Arg Arg Arg Arg Arg Arg Lys
   1 5 10
<210> 741
   <211> 9
   <212> PRT
   <213> Artificial Sequence
<220>
   <223> CPP
<400> 741
   Arg Arg Arg Arg Arg Arg Arg Arg Arg
   1 5
<210> 742
   <211> 9
   <212> PRT
   <213> Artificial Sequence
<220>
   <223> CPP
<400> 742
   Arg Arg Arg Arg Arg Arg Arg Arg Arg
   1 5
<210> 743
   <211> 22
   <212> PRT
   <213> Artificial Sequence
<220>
   <223> CPP
<400> 743
   Lys Cys Phe Gln Trp Gln Arg Asn Met Arg Lys Val Arg Gly Pro Pro
   1 5 10 15
   Val Ser Cys Ile Lys Arg
   20
<210> 744
   <211> 22
   <212> PRT
   <213> Artificial Sequence
<220>
   <223> CPP
<400> 744
   Lys Cys Phe Gln Trp Gln Arg Asn Met Arg Lys Val Arg Gly Pro Pro
   1 5 10 15
   Val Ser Cys Ile Lys Arg
   20
<210> 745
   <211> 20
   <212> PRT
   <213> Artificial Sequence
<220>
   <223> CPP
<400> 745
   Lys Leu Ala Leu Lys Leu Ala Leu Lys Ala Leu Lys Ala Ala Leu Lys
   1 5 10 15
   Leu Ala Gly Cys
   20
<210> 746
   <211> 20
   <212> PRT
   <213> Artificial Sequence
<220>
   <223> CPP MAP (Aib), wherein each Xaa = Aib, U (alpha-amino isobutyric acid)
<220>
   <221> misc_feature
   <222> (3) .. (3)
   <223> Xaa can be any naturally occurring amino acid
<220>
   <221> misc_feature
   <222> (7) .. (7)
   <223> Xaa can be any naturally occurring amino acid
<220>
   <221> misc_feature
   <222> (10) .. (10)
   <223> Xaa can be any naturally occurring amino acid
<220>
   <221> misc_feature
   <222> (14) .. (14)
   <223> Xaa can be any naturally occurring amino acid
<220>
   <221> misc_feature
   <222> (18) .. (18)
   <223> Xaa can be any naturally occurring amino acid
<400> 746
   Lys Leu Xaa Leu Lys Leu Xaa Leu Lys Xaa Leu Lys Ala Xaa Leu Lys
   1               5                   10                  15
   Leu Xaa Gly Cys
               20
<210> 747
   <211> 35
   <212> PRT
   <213> Artificial Sequence
<220>
   <223> CPP
<400> 747
   Gly Gly Gly Ala Arg Lys Lys Ala Ala Lys Ala Ala Arg Lys Lys Ala
   1               5                   10                  15
   Ala Lys Ala Ala Arg Lys Lys Ala Ala Lys Ala Ala Arg Lys Lys Ala
               20                  25                  30
   Ala Lys Ala
           35
<210> 748
   <211> 14
   <212> PRT
   <213> Artificial Sequence
<220>
   <223> CPP
<400> 748
   Gly Arg Lys Lys Arg Arg Gln Arg Arg Arg Pro Pro Gln Cys
   1               5                   10
<210> 749
   <211> 19
   <212> PRT
   <213> Artificial Sequence
<220>
   <223> CPP
<400> 749
   Thr Arg Gln Ala Arg Arg Asn Arg Arg Arg Arg Trp Arg Glu Arg Gln
   1               5                   10                  15
   Arg Gly Cys
<210> 750
   <211> 17
   <212> PRT
   <213> Artificial Sequence
<220>
   <223> CPP
<400> 750
   Arg Arg Arg Arg Asn Arg Thr Arg Arg Asn Arg Arg Arg Val Arg Gly
   1               5                   10                  15
   Cys
<210> 751
   <211> 21
   <212> PRT
   <213> Artificial Sequence
<220>
   <223> CPP
<400> 751
   Lys Met Thr Arg Ala Gln Arg Arg Ala Ala Ala Arg Arg Asn Arg Trp
   1               5                   10                  15
   Thr Ala Arg Gly Cys
               20
<210> 752
   <211> 15
   <212> PRT
   <213> Artificial Sequence
<220>
   <223> CPP
<400> 752
   Thr Arg Arg Gln Arg Thr Arg Arg Ala Arg Arg Asn Arg Gly Cys
   1               5                   10                  15
<210> 753
   <211> 30
   <212> PRT
   <213> Artificial Sequence
<220>
   <223> CPP
<400> 753
   Arg Ile Lys Ala Glu Arg Lys Arg Met Arg Asn Arg Ile Ala Ala Ser
   1               5                   10                  15
   Lys Ser Arg Lys Arg Lys Leu Glu Arg Ile Ala Arg Gly Cys
               20                  25                  30
<210> 754
   <211> 28
   <212> PRT
   <213> Artificial Sequence
<220>
   <223> CPP
<400> 754
   Lys Arg Arg Ile Arg Arg Glu Arg Asn Lys Met Ala Ala Ala Lys Ser
   1 5 10 15
   Arg Asn Arg Arg Arg Glu Leu Thr Asp Thr Gly Cys
   20 25
<210> 755
   <211> 23
   <212> PRT
   <213> Artificial Sequence
<220>
   <223> CPP
<400> 755
   Trp Leu Arg Arg Ile Lys Ala Trp Leu Arg Arg Ile Lys Ala Leu Asn
   1 5 10 15
   Arg Gln Leu Gly Val Ala Ala
   20
<210> 756
   <211> 10
   <212> PRT
   <213> Artificial Sequence
<220>
   <223> CPP
<400> 756
   Cys Arg Lys Lys Arg Arg Gln Arg Arg Arg
   1 5 10
<210> 757
   <211> 10
   <212> PRT
   <213> Artificial Sequence
<220>
   <223> CPP
<400> 757
   Cys Arg Arg Arg Arg Arg Arg Arg Arg Arg
   1 5 10
<210> 758
   <211> 10
   <212> PRT
   <213> Artificial Sequence
<220>
   <223> CPP
<400> 758
   Cys Lys Lys Lys Lys Lys Lys Lys Lys Lys
   1               5                   10
<210> 759
   <211> 12
   <212> PRT
   <213> Artificial Sequence
<220>
   <223> CPP
<400> 759
   Gly Arg Lys Lys Arg Arg Gln Arg Arg Arg Pro Pro
   1               5                   10
<210> 760
   <211> 8
   <212> PRT
   <213> Artificial Sequence
<220>
   <223> CPP
<400> 760